# EUROPEAN PATENT SPECIFICATION

(11) **EP 2 975 062 B1**
(45) Date of publication and mention of the grant of the patent: **25.07.2018**
(21) Application number: 14775837.9
(22) Date of filing: 15.05.2014
(51) Int. Cl.: C07K 19/00, A61K 38/16, A61P 7/04, A61K 38/00, C07K 14/765, C07K 14/52

(54) **PREPARATION AND USE OF DIMERIZED FUSION PROTEIN**
HERSTELLUNG UND VERWENDUNG VON DIMERISIERTEM FUSIONSPROTEIN
PRÉPARATION ET UTILISATION D'UNE PROTÉINE DE FUSION DIMÉRISÉE

(43) Date of publication of application: 20.01.2016
(73) Proprietor: Lanzhou University, Lanzhou, Gansu 730000 (CN)
(72) Inventor: LI, Hongyu, Lanzhou Gansu 730000 (CN); AN, Lizhe, Lanzhou Gansu 730000 (CN); ZHI, Dejuan, Lanzhou Gansu 730000 (CN); LI, Yang, Lanzhou Gansu 730000 (CN); FENG, Na, Lanzhou Gansu 730000 (CN); GUO, Dingding, Lanzhou Gansu 730000 (CN); WANG, Yong, Lanzhou Gansu 730000 (CN); SHI, Yanbin, Lanzhou Gansu 730000 (CN); RU, Yi, Lanzhou Gansu 730000 (CN); WANG, Meizhu, Lanzhou Gansu 730000 (CN); TANG, Wei, Lanzhou Gansu 730000 (CN); GAO, Jiangli, Lanzhou Gansu 730000 (CN); HOU, Rongli, Lanzhou Gansu 730000 (CN); XU, Jing, Lanzhou Gansu 730000 (CN); XIAN, Jun, Lanzhou Gansu 730000 (CN); XU, Yinli, Lanzhou Gansu 730000 (CN); ZHANG, Liyun, Lanzhou Gansu 730000 (CN); WANG, Haiqing, Lanzhou Gansu 730000 (CN); DONG, Xinfang, Lanzhou Gansu 730000 (CN); MA, Xingming, Lanzhou Gansu 730000 (CN); LI, Jiazhong, Lanzhou Gansu 730000 (CN); ZHU, Hongmei, Lanzhou Gansu 730000 (CN); LI, Jianyin, Lanzhou Gansu 730000 (CN); ZHAO, Chunyan, Lanzhou Gansu 730000 (CN)
(74) Representative: J A Kemp
(86) International application number: PCT/CN2014/077617
(87) International publication number: WO 2014/154187

(56) References cited:
- WO-A2-03/031589
- CN-A- 1 254 718
- CN-A- 1 810 832
- CN-A- 1 966 520
- CN-A- 101 400 703
- CN-A- 102 408 485

### SEQUENCE LISTING

<110> LANZHOU university
<120> Preparation and Use of Dimerized Fusion Protein
<130> N406794EP
<140> EP 14775837.9
   <141> 2014-05-15
<150> CN201310084212.8
   <151> 2013-03-15
<160> 48
<170> PatentIn version 3.5
<210> 1
   <211> 42
   <212> DNA
   <213> gene sequence of peptide(H)
<400> 1
   gagcccaaga gcagcgacaa aactcacaca tgcccaccgt gc 42
<210> 2
   <211> 14
   <212> PRT
   <213> amino acid sequence of peptide(H)
<400> 2
   Glu Pro Lys Ser Ser Asp Lys Thr His Thr Cys Pro Pro Cys
   1               5                   10
<210> 3
   <211> 114
   <212> DNA
   <213> gene sequence of peptide (fip) selected from fip
<400> 3
   gtgagcaggg acgagctgat ggaggccatc cagaagcagg aggagatcaa cttcaggctg       60
   caggactaca tcgacaggat catcgtggcc atcatggaga ccaaccccag catc            114
<210> 4
   <211> 38
   <212> PRT
   <213> amino acid sequence of peptide (fip) selected from fip
<400> 4
   Val Ser Arg Asp Glu Leu Met Glu Ala Ile Gln Lys Gln Glu Glu Ile
   1               5                   10                  15
   Asn Phe Arg Leu Gln Asp Tyr Ile Asp Arg Ile Ile Val Ala Ile Met
               20                  25                  30
   Glu Thr Asn Pro Ser Ile
           35
<210> 5
   <211> 42
   <212> DNA
   <213> gene sequence of thrombopoietin mimetic peptide TMP
<400> 5
   atcgagggac ccaccctgag gcagtggctg gccgccagag cc 42
<210> 6
   <211> 14
   <212> PRT
   <213> amino acid sequence of thrombopoietin mimetic peptide TMP
<400> 6
   Ile Glu Gly Pro Thr Leu Arg Gln Trp Leu Ala Ala Arg Ala
   1               5                   10
<210> 7
   <211> 1755
   <212> DNA
   <213> gene sequence of Human Serum Albumin(HSA)
<400> 7
   gatgcacaca agagtgaggt tgctcatcgg tttaaagatt tgggagaaga aaatttcaaa      60
   gccttggtgt tgattgcctt tgctcagtat cttcagcagt gtccatttga agatcatgta     120
   aaattagtga atgaagtaac tgaatttgca aaaacatgtg ttgctgatga gtcagctgaa     180
   aattgtgaca aatcacttca taccctttttggagacaaat tatgcacagt tgcaactctt     240
   cgtgaaacct atggtgaaat ggctgactgt tgtgcaaaac aagaacctga gagaaatgaa     300
   tgcttcttgc aacacaaaga tgacaaccca aacctccccc gattggtgag accagaggtt     360
   gatgtgatgt gcactgcttt tcatgacaat gaagagacat ttttgaaaaa atacttatat     420
   gaaattgcca gaagacatcc ttacttttat gccccggaac tccttttctt tgctaaaagg     480
   tataaagctg cttttacaga atgttgccaa gctgctgata aagctgcctg cctgttgcca     540
   aagctcgatg aacttcggga tgaagggaag gcttcgtctg ccaaacagag actcaagtgt     600
   gccagtctcc aaaaatttgg agaaagagct ttcaaagcat gggcagtagc tcgcctgagc     660
   cagagatttc ccaaagctga gtttgcagaa gtttccaagt tagtgacaga tcttaccaaa     720
   gtccacacgg aatgctgcca tggagatctg cttgaatgtg ctgatgacag ggcggacctt     780
   gccaagtata tctgtgaaaa tcaagattcg atctccagta aactgaagga atgctgtgaa     840
   aaacctctgt tggaaaaatc ccactgcatt gccgaagtgg aaaatgatga gatgcctgct     900
   gacttgcctt cattagctgc tgattttgtt gaaagtaagg atgtttgcaa aaactatgct     960
   gaggcaaagg atgtcttcct gggcatgttt ttgtatgaat atgcaagaag gcatcctgat      1020
   tactctgtcg tgctgctgct gagacttgcc aagacatatg aaaccactct agagaagtgc      1080
   tgtgccgctg cagatcctca tgaatgctat gccaaagtgt tcgatgaatt taaacctctt      1140
   gtggaagagc ctcagaattt aatcaaacaa aattgtgagc tttttgagca gcttggagag      1200
   tacaaattcc agaatgcgct attagttcgt tacaccaaga aagtacccca agtgtcaact      1260
   ccaactcttg tagaggtctc aagaaaccta ggaaaagtgg gcagcaaatg ttgtaaacat      1320
   cctgaagcaa aaagaatgcc ctgtgcagaa gactatctat ccgtggtcct gaaccagtta      1380
   tgtgtgttgc atgagaaaac gccagtaagt gacagagtca ccaaatgctg cacagaatcc      1440
   ttggtgaaca ggcgaccatg cttttcagct ctggaagtcg atgaaacata cgttcccaaa      1500
   gagtttaatg ctgaaacgtt caccttccat gcagatatat gcacactttc tgagaaggag      1560
   agacaaatca agaaacaaac tgcacttgtt gagcttgtga aacacaagcc caaggcaaca      1620
   aaagagcaac tgaaagctgt tatggatgat ttcgcagctt ttgtagagaa gtgctgcaag      1680
   gctgacgata aggagacctg ctttgccgag gagggtaaaa aacttgttgc tgcaagtcaa      1740
   gctgccttag gctta                                                        1755
<210> 8
   <211> 585
   <212> PRT
   <213> amino acid sequence of Human Serum Albumin(HSA)
<400> 8
<210> 9
   <211> 1131
   <212> DNA
   <213> fip(Postl)-HSA(Prel) the front end of Human Serum Albumin(HSA) and gene sequence selected from the rear end of fip
<400> 9
   gctgcaggac tacatcgaca ggatcatcgt ggccatcatg gagaccaacc ccagcatcga 60
   tgcacacaag agtgaggttg ctcatcggtt taaagatttg ggagaagaaa atttcaaagc 120
   cttggtgttg attgcctttg ctcagtatct tcagcagtgt ccatttgaag atcatgtaaa 180
   attagtgaat gaagtaactg aatttgcaaa aacatgtgtt gctgatgagt cagctgaaaa 240
   ttgtgacaaa tcacttcata ccctttttgg agacaaatta tgcacagttg caactcttcg 300
   tgaaacctat ggtgaaatgg ctgactgttg tgcaaaacaa gaacctgaga gaaatgaatg 360
   cttcttgcaa cacaaagatg acaacccaaa cctcccccga ttggtgagac cagaggttga 420
   tgtgatgtgc actgcttttc atgacaatga agagacattt ttgaaaaaat acttatatga 480
   aattgccaga agacatcctt acttttatgc cccggaactc cttttctttg ctaaaaggta 540
   taaagctgct tttacagaat gttgccaagc tgctgataaa gctgcctgcc tgttgccaaa 600
   gctcgatgaa cttcgggatg aagggaaggc ttcgtctgcc aaacagagac tcaagtgtgc 660
   cagtctccaa aaatttggag aaagagcttt caaagcatgg gcagtagctc gcctgagcca 720
   gagatttccc aaagctgagt ttgcagaagt ttccaagtta gtgacagatc ttaccaaagt 780
   ccacacggaa tgctgccatg gagatctgct tgaatgtgct gatgacaggg cggaccttgc 840
   caagtatatc tgtgaaaatc aagattcgat ctccagtaaa ctgaaggaat gctgtgaaaa 900
   acctctgttg gaaaaatccc actgcattgc cgaagtggaa aatgatgaga tgcctgctga 960
   cttgccttca ttagctgctg attttgttga aagtaaggat gtttgcaaaa actatgctga 1020
   ggcaaaggat gtcttcctgg gcatgttttt gtatgaatat gcaagaaggc atcctgatta 1080
   ctctgtcgtg ctgctgctga gacttgccaa gacatatgaa accactctag a 1131
<210> 10
   <211> 376
   <212> PRT
   <213> fip(Postl)-HSA(Prel) the front end of Human Serum Albumin(HSA) and amino acid sequence selected from the rear end of fip
<400> 10
   Leu Gln Asp Tyr Ile Asp Arg Ile Ile Val Ala Ile Met Glu Thr Asn
   1               5                   10                  15
   Pro Ser Ile Asp Ala His Lys Ser Glu Val Ala His Arg Phe Lys Asp
               20                  25                  30
   Leu Gly Glu Glu Asn Phe Lys Ala Leu Val Leu Ile Ala Phe Ala Gln
           35                  40                  45
   Tyr Leu Gln Gln Cys Pro Phe Glu Asp His Val Lys Leu Val Asn Glu
       50                  55                  60
   Val Thr Glu Phe Ala Lys Thr Cys Val Ala Asp Glu Ser Ala Glu Asn
   65                  70                  75                  80
   Cys Asp Lys Ser Leu His Thr Leu Phe Gly Asp Lys Leu Cys Thr Val
                   85                  90                  95
   Ala Thr Leu Arg Glu Thr Tyr Gly Glu Met Ala Asp Cys Cys Ala Lys
               100                 105                 110
   Gln Glu Pro Glu Arg Asn Glu Cys Phe Leu Gln His Lys Asp Asp Asn
           115                 120                 125
   Pro Asn Leu Pro Arg Leu Val Arg Pro Glu Val Asp Val Met Cys Thr
       130                 135                 140
   Ala Phe His Asp Asn Glu Glu Thr Phe Leu Lys Lys Tyr Leu Tyr Glu
   145                 150                 155                 160
   Ile Ala Arg Arg His Pro Tyr Phe Tyr Ala Pro Glu Leu Leu Phe Phe
                   165                 170                 175
   Ala Lys Arg Tyr Lys Ala Ala Phe Thr Glu Cys Cys Gln Ala Ala Asp
               180                 185                 190
   Lys Ala Ala Cys Leu Leu Pro Lys Leu Asp Glu Leu Arg Asp Glu Gly
           195                 200                 205
   Lys Ala Ser Ser Ala Lys Gln Arg Leu Lys Cys Ala Ser Leu Gln Lys
       210                 215                 220
   Phe Gly Glu Arg Ala Phe Lys Ala Trp Ala Val Ala Arg Leu Ser Gln
   225                 230                 235                 240
   Arg Phe Pro Lys Ala Glu Phe Ala Glu Val Ser Lys Leu Val Thr Asp
                   245                 250                 255
   Leu Thr Lys Val His Thr Glu Cys Cys His Gly Asp Leu Leu Glu Cys
               260                 265                 270
   Ala Asp Asp Arg Ala Asp Leu Ala Lys Tyr Ile Cys Glu Asn Gln Asp
           275                 280                 285
   Ser Ile Ser Ser Lys Leu Lys Glu Cys Cys Glu Lys Pro Leu Leu Glu
       290                 295                 300
   Lys Ser His Cys Ile Ala Glu Val Glu Asn Asp Glu Met Pro Ala Asp
   305                 310                 315                 320
   Leu Pro Ser Leu Ala Ala Asp Phe Val Glu Ser Lys Asp Val Cys Lys
                   325                 330                 335
   Asn Tyr Ala Glu Ala Lys Asp Val Phe Leu Gly Met Phe Leu Tyr Glu
               340                 345                 350
   Tyr Ala Arg Arg His Pro Asp Tyr Ser Val Val Leu Leu Leu Arg Leu
           355                 360                 365
   Ala Lys Thr Tyr Glu Thr Thr Leu
       370                 375
<210> 11
   <211> 709
   <212> DNA
   <213> HSA(Postl)-His gene sequence of the rear end of Human Serum Albumin
<400> 11
   tctagagaag tgctgtgccg ctgcagatcc tcatgaatgc tatgccaaag tgttcgatga    60
   atttaaacct cttgtggaag agcctcagaa tttaatcaaa caaaattgtg agctttttga   120
   gcagcttgga gagtacaaat tccagaatgc gctattagtt cgttacacca agaaagtacc   180
   ccaagtgtca actccaactc ttgtagaggt ctcaagaaac ctaggaaaag tgggcagcaa   240
   atgttgtaaa catcctgaag caaaaagaat gccctgtgca gaagactatc tatccgtggt   300
   cctgaaccag ttatgtgtgt tgcatgagaa aacgccagta agtgacagag tcaccaaatg   360
   ctgcacagaa tccttggtga acaggcgacc atgcttttca gctctggaag tcgatgaaac   420
   atacgttccc aaagagttta atgctgaaac gttcaccttc catgcagata tatgcacact   480
   ttctgagaag gagagacaaa tcaagaaaca aactgcactt gttgagcttg tgaaacacaa   540
   gcccaaggca acaaaagagc aactgaaagc tgttatggat gatttcgcag cttttgtaga   600
   gaagtgctgc aaggctgacg ataaggagac ctgctttgcc gaggagggta aaaaacttgt   660
   tgctgcaagt caagctgcct taggcttaca ccaccaccac caccactga               709
<210> 12
   <211> 235
   <212> PRT
   <213> HSA(Postl)-His amino acid sequence of the rear end of Human Serum Albumin
<400> 12
   Leu Glu Lys Cys Cys Ala Ala Ala Asp Pro His Glu Cys Tyr Ala Lys
   1               5                   10                  15
   Val Phe Asp Glu Phe Lys Pro Leu Val Glu Glu Pro Gln Asn Leu Ile
               20                  25                  30
   Lys Gln Asn Cys Glu Leu Phe Glu Gln Leu Gly Glu Tyr Lys Phe Gln
           35                  40                  45
   Asn Ala Leu Leu Val Arg Tyr Thr Lys Lys Val Pro Gln Val Ser Thr
       50                  55                  60
   Pro Thr Leu Val Glu Val Ser Arg Asn Leu Gly Lys Val Gly Ser Lys
   65                  70                  75                  80
   Cys Cys Lys His Pro Glu Ala Lys Arg Met Pro Cys Ala Glu Asp Tyr
                   85                  90                  95
   Leu Ser Val Val Leu Asn Gln Leu Cys Val Leu His Glu Lys Thr Pro
               100                 105                 110
   Val Ser Asp Arg Val Thr Lys Cys Cys Thr Glu Ser Leu Val Asn Arg
           115                 120                 125
   Arg Pro Cys Phe Ser Ala Leu Glu Val Asp Glu Thr Tyr Val Pro Lys
       130                 135                 140
   Glu Phe Asn Ala Glu Thr Phe Thr Phe His Ala Asp Ile Cys Thr Leu
   145                 150                 155                 160
   Ser Glu Lys Glu Arg Gln Ile Lys Lys Gln Thr Ala Leu Val Glu Leu
                   165                 170                 175
   Val Lys His Lys Pro Lys Ala Thr Lys Glu Gln Leu Lys Ala Val Met
               180                 185                 190
   Asp Asp Phe Ala Ala Phe Val Glu Lys Cys Cys Lys Ala Asp Asp Lys
           195                 200                 205
   Glu Thr Cys Phe Ala Glu Glu Gly Lys Lys Leu Val Ala Ala Ser Gln
       210                 215                 220
   Ala Ala Leu Gly Leu His His His His His His
   225                 230                 235
<210> 13
   <211> 165
   <212> DNA
   <213> gene sequence of ss-TMP-L1-TMP
<400> 13
   atggagacag acacactcct gctatgggta ctgctgctct gggttccagg ttccactggt        60
   gacggtatcg aaggccccac actgcggcag tggctggctg ctagggccgg tggcggctcc       120
   ggaatcgagg gacccaccct gaggcagtgg ctggccgcca gagcc                       165
<210> 14
   <211> 55
   <212> PRT
   <213> amino acid sequence of ss-TMP-L1-TMP
<400> 14
   Met Glu Thr Asp Thr Leu Leu Leu Trp Val Leu Leu Leu Trp Val Pro
   1               5                   10                  15
   Gly Ser Thr Gly Asp Gly Ile Glu Gly Pro Thr Leu Arg Gln Trp Leu
               20                  25                  30
   Ala Ala Arg Ala Gly Gly Gly Ser Gly Ile Glu Gly Pro Thr Leu Arg
           35                  40                  45
   Gln Trp Leu Ala Ala Arg Ala
       50                  55
<210> 15
   <211> 291
   <212> DNA
   <213> gene sequence of ss-TMP-L1-TMP-L5-H-fip(Pre1)
<400> 15
   atggagacag acacactcct gctatgggta ctgctgctct gggttccagg ttccactggt        60
   gacggtatcg aaggccccac actgcggcag tggctggctg ctagggccgg tggcggctcc       120
   ggaatcgagg gacccaccct gaggcagtgg ctggccgcca gagccggcgg cggcggaagc       180
   agatctgagc ccaagagcag cgacaaaact cacacatgcc caccgtgcgt gagcagggac       240
   gagctgatgg aggccatcca gaagcaggag gagatcaact tcaggctgca g                291
<210> 16
   <211> 97
   <212> PRT
   <213> amino acid sequence of ss-TMP-L1-TMP-L5-H-fip(Pre1)
<400> 16
   Met Glu Thr Asp Thr Leu Leu Leu Trp Val Leu Leu Leu Trp Val Pro
   1               5                   10                  15
   Gly Ser Thr Gly Asp Gly Ile Glu Gly Pro Thr Leu Arg Gln Trp Leu
               20                  25                  30
   Ala Ala Arg Ala Gly Gly Gly Ser Gly Ile Glu Gly Pro Thr Leu Arg
           35                  40                  45
   Gln Trp Leu Ala Ala Arg Ala Gly Gly Gly Gly Ser Arg Ser Glu Pro
       50                  55                  60
   Lys Ser Ser Asp Lys Thr His Thr Cys Pro Pro Cys Val Ser Arg Asp
   65                  70                  75                  80
   Glu Leu Met Glu Ala Ile Gln Lys Gln Glu Glu Ile Asn Phe Arg Leu
                   85                  90                  95
   Gln
<210> 17
   <211> 2118
   <212> DNA
   <213> gene sequence of ss-TMP-L1-TMP-L5-H-fip-HSA-His
<400> 17
   atggagacag acacactcct gctatgggta ctgctgctct gggttccagg ttccactggt     60
   gacggtatcg aaggccccac actgcggcag tggctggctg ctagggccgg tggcggctcc    120
   ggaatcgagg gacccaccct gaggcagtgg ctggccgcca gagccggcgg cggcggaagc    180
   agatctgagc ccaagagcag cgacaaaact cacacatgcc caccgtgcgt gagcagggac    240
   gagctgatgg aggccatcca gaagcaggag gagatcaact tcaggctgca ggactacatc    300
   gacaggatca tcgtggccat catggagacc aaccccagca tcgatgcaca caagagtgag    360
   gttgctcatc ggtttaaaga tttgggagaa gaaaatttca aagccttggt gttgattgcc    420
   tttgctcagt atcttcagca gtgtccattt gaagatcatg taaaattagt gaatgaagta    480
   actgaatttg caaaaacatg tgttgctgat gagtcagctg aaaattgtga caaatcactt    540
   catacccttt ttggagacaa attatgcaca gttgcaactc ttcgtgaaac ctatggtgaa    600
   atggctgact gttgtgcaaa acaagaacct gagagaaatg aatgcttctt gcaacacaaa    660
   gatgacaacc caaacctccc ccgattggtg agaccagagg ttgatgtgat gtgcactgct    720
   tttcatgaca atgaagagac atttttgaaa aaatacttat atgaaattgc cagaagacat    780
   ccttactttt atgccccgga actccttttc tttgctaaaa ggtataaagc tgcttttaca    840
   gaatgttgcc aagctgctga taaagctgcc tgcctgttgc caaagctcga tgaacttcgg    900
   gatgaaggga aggcttcgtc tgccaaacag agactcaagt gtgccagtct ccaaaaattt    960
   ggagaaagag ctttcaaagc atgggcagta gctcgcctga gccagagatt tcccaaagct   1020
   gagtttgcag aagtttccaa gttagtgaca gatcttacca aagtccacac ggaatgctgc   1080
   catggagatc tgcttgaatg tgctgatgac agggcggacc ttgccaagta tatctgtgaa   1140
   aatcaagatt cgatctccag taaactgaag gaatgctgtg aaaaacctct gttggaaaaa   1200
   tcccactgca ttgccgaagt ggaaaatgat gagatgcctg ctgacttgcc ttcattagct 1260
   gctgattttg ttgaaagtaa ggatgtttgc aaaaactatg ctgaggcaaa ggatgtcttc 1320
   ctgggcatgt ttttgtatga atatgcaaga aggcatcctg attactctgt cgtgctgctg 1380
   ctgagacttg ccaagacata tgaaaccact ctagagaagt gctgtgccgc tgcagatcct 1440
   catgaatgct atgccaaagt gttcgatgaa tttaaacctc ttgtggaaga gcctcagaat 1500
   ttaatcaaac aaaattgtga gctttttgag cagcttggag agtacaaatt ccagaatgcg 1560
   ctattagttc gttacaccaa gaaagtaccc caagtgtcaa ctccaactct tgtagaggtc 1620
   tcaagaaacc taggaaaagt gggcagcaaa tgttgtaaac atcctgaagc aaaaagaatg 1680
   ccctgtgcag aagactatct atccgtggtc ctgaaccagt tatgtgtgtt gcatgagaaa 1740
   acgccagtaa gtgacagagt caccaaatgc tgcacagaat ccttggtgaa caggcgacca 1800
   tgcttttcag ctctggaagt cgatgaaaca tacgttccca aagagtttaa tgctgaaacg 1860
   ttcaccttcc atgcagatat atgcacactt tctgagaagg agagacaaat caagaaacaa 1920
   actgcacttg ttgagcttgt gaaacacaag cccaaggcaa caaaagagca actgaaagct 1980
   gttatggatg atttcgcagc ttttgtagag aagtgctgca aggctgacga taaggagacc 2040
   tgctttgccg aggagggtaa aaaacttgtt gctgcaagtc aagctgcctt aggcttacac 2100
   caccaccacc accactga 2118
<210> 18
   <211> 705
   <212> PRT
   <213> amino acid sequence of ss-TMP-L1-TMP-L5-H-fip-HSA-His
<400> 18
   Met Glu Thr Asp Thr Leu Leu Leu Trp Val Leu Leu Leu Trp Val Pro
   1               5                   10                  15
   Gly Ser Thr Gly Asp Gly Ile Glu Gly Pro Thr Leu Arg Gln Trp Leu
               20                  25                  30
   Ala Ala Arg Ala Gly Gly Gly Ser Gly Ile Glu Gly Pro Thr Leu Arg
           35                  40                  45
   Gln Trp Leu Ala Ala Arg Ala Gly Gly Gly Gly Ser Arg Ser Glu Pro
       50                  55                  60
   Lys Ser Ser Asp Lys Thr His Thr Cys Pro Pro Cys Val Ser Arg Asp
   65                  70                  75                  80
   Glu Leu Met Glu Ala Ile Gln Lys Gln Glu Glu Ile Asn Phe Arg Leu
                   85                  90                  95
   Gln Asp Tyr Ile Asp Arg Ile Ile Val Ala Ile Met Glu Thr Asn Pro
               100                 105                 110
   Ser Ile Asp Ala His Lys Ser Glu Val Ala His Arg Phe Lys Asp Leu
           115                 120                 125
   Gly Glu Glu Asn Phe Lys Ala Leu Val Leu Ile Ala Phe Ala Gln Tyr
       130                 135                 140
   Leu Gln Gln Cys Pro Phe Glu Asp His Val Lys Leu Val Asn Glu Val
   145                 150                 155                 160
   Thr Glu Phe Ala Lys Thr Cys Val Ala Asp Glu Ser Ala Glu Asn Cys
                   165                 170                 175
   Asp Lys Ser Leu His Thr Leu Phe Gly Asp Lys Leu Cys Thr Val Ala
               180                 185                 190
   Thr Leu Arg Glu Thr Tyr Gly Glu Met Ala Asp Cys Cys Ala Lys Gln
           195                 200                 205
   Glu Pro Glu Arg Asn Glu Cys Phe Leu Gln His Lys Asp Asp Asn Pro
       210                 215                 220
   Asn Leu Pro Arg Leu Val Arg Pro Glu Val Asp Val Met Cys Thr Ala
   225                 230                 235                 240
   Phe His Asp Asn Glu Glu Thr Phe Leu Lys Lys Tyr Leu Tyr Glu Ile
                   245                 250                 255
   Ala Arg Arg His Pro Tyr Phe Tyr Ala Pro Glu Leu Leu Phe Phe Ala
               260                 265                 270
   Lys Arg Tyr Lys Ala Ala Phe Thr Glu Cys Cys Gln Ala Ala Asp Lys
           275                 280                 285
   Ala Ala Cys Leu Leu Pro Lys Leu Asp Glu Leu Arg Asp Glu Gly Lys
       290                 295                 300
   Ala Ser Ser Ala Lys Gln Arg Leu Lys Cys Ala Ser Leu Gln Lys Phe
   305                 310                 315                 320
   Gly Glu Arg Ala Phe Lys Ala Trp Ala Val Ala Arg Leu Ser Gln Arg
                   325                 330                 335
   Phe Pro Lys Ala Glu Phe Ala Glu Val Ser Lys Leu Val Thr Asp Leu
               340                 345                 350
   Thr Lys Val His Thr Glu Cys Cys His Gly Asp Leu Leu Glu Cys Ala
   355 360 365
   Asp Asp Arg Ala Asp Leu Ala Lys Tyr Ile Cys Glu Asn Gln Asp Ser
   370 375 380
   Ile Ser Ser Lys Leu Lys Glu Cys Cys Glu Lys Pro Leu Leu Glu Lys
   385 390 395 400
   Ser His Cys Ile Ala Glu Val Glu Asn Asp Glu Met Pro Ala Asp Leu
   405 410 415
   Pro Ser Leu Ala Ala Asp Phe Val Glu Ser Lys Asp Val Cys Lys Asn
   420 425 430
   Tyr Ala Glu Ala Lys Asp Val Phe Leu Gly Met Phe Leu Tyr Glu Tyr
   435 440 445
   Ala Arg Arg His Pro Asp Tyr Ser Val Val Leu Leu Leu Arg Leu Ala
   450 455 460
   Lys Thr Tyr Glu Thr Thr Leu Glu Lys Cys Cys Ala Ala Ala Asp Pro
   465 470 475 480
   His Glu Cys Tyr Ala Lys Val Phe Asp Glu Phe Lys Pro Leu Val Glu
   485 490 495
   Glu Pro Gln Asn Leu Ile Lys Gln Asn Cys Glu Leu Phe Glu Gln Leu
   500 505 510
   Gly Glu Tyr Lys Phe Gln Asn Ala Leu Leu Val Arg Tyr Thr Lys Lys
   515 520 525
   Val Pro Gln Val Ser Thr Pro Thr Leu Val Glu Val Ser Arg Asn Leu
   530 535 540
   Gly Lys Val Gly Ser Lys Cys Cys Lys His Pro Glu Ala Lys Arg Met
   545 550 555 560
   Pro Cys Ala Glu Asp Tyr Leu Ser Val Val Leu Asn Gln Leu Cys Val
   565 570 575
   Leu His Glu Lys Thr Pro Val Ser Asp Arg Val Thr Lys Cys Cys Thr
   580 585 590
   Glu Ser Leu Val Asn Arg Arg Pro Cys Phe Ser Ala Leu Glu Val Asp
           595                 600                 605
   Glu Thr Tyr Val Pro Lys Glu Phe Asn Ala Glu Thr Phe Thr Phe His
       610                 615                 620
   Ala Asp Ile Cys Thr Leu Ser Glu Lys Glu Arg Gln Ile Lys Lys Gln
   625                 630                 635                 640
   Thr Ala Leu Val Glu Leu Val Lys His Lys Pro Lys Ala Thr Lys Glu
                   645                 650                 655
   Gln Leu Lys Ala Val Met Asp Asp Phe Ala Ala Phe Val Glu Lys Cys
               660                 665                 670
   Cys Lys Ala Asp Asp Lys Glu Thr Cys Phe Ala Glu Glu Gly Lys Lys
           675                 680                 685
   Leu Val Ala Ala Ser Gln Ala Ala Leu Gly Leu His His His His His
       690                 695                 700
   His
   705
<210> 19
   <211> 2109
   <212> DNA
   <213> gene sequence of ss-TMP-L2-TMP-L5-H-fip-HSA-His
<400> 19
   atggagacag acacactcct gctatgggta ctgctgctct gggttccagg ttccactggt      60
   gacggtatcg aaggccccac actgcggcag tggctggctg ctagggcctc cggaatcgag     120
   ggacccaccc tgaggcagtg gctggccgcc agagccggcg gcggcggaag cagatctgag     180
   cccaagagca gcgacaaaac tcacacatgc ccaccgtgcg tgagcaggga cgagctgatg     240
   gaggccatcc agaagcagga ggagatcaac ttcaggctgc aggactacat cgacaggatc     300
   atcgtggcca tcatggagac caaccccagc atcgatgcac acaagagtga ggttgctcat     360
   cggtttaaag atttgggaga agaaaatttc aaagccttgg tgttgattgc ctttgctcag     420
   tatcttcagc agtgtccatt tgaagatcat gtaaaattag tgaatgaagt aactgaattt     480
   gcaaaaacat gtgttgctga tgagtcagct gaaaattgtg acaaatcact tcataccctt     540
   tttggagaca aattatgcac agttgcaact cttcgtgaaa cctatggtga aatggctgac     600
   tgttgtgcaa aacaagaacc tgagagaaat gaatgcttct tgcaacacaa agatgacaac     660
   ccaaacctcc cccgattggt gagaccagag gttgatgtga tgtgcactgc ttttcatgac     720
   aatgaagaga catttttgaa aaaatactta tatgaaattg ccagaagaca tccttacttt     780
   tatgccccgg aactcctttt ctttgctaaa aggtataaag ctgcttttac agaatgttgc     840
   caagctgctg ataaagctgc ctgcctgttg ccaaagctcg atgaacttcg ggatgaaggg     900
   aaggcttcgt ctgccaaaca gagactcaag tgtgccagtc tccaaaaatt tggagaaaga     960
   gctttcaaag catgggcagt agctcgcctg agccagagat ttcccaaagc tgagtttgca    1020
   gaagtttcca agttagtgac agatcttacc aaagtccaca cggaatgctg ccatggagat    1080
   ctgcttgaat gtgctgatga cagggcggac cttgccaagt atatctgtga aaatcaagat    1140
   tcgatctcca gtaaactgaa ggaatgctgt gaaaaacctc tgttggaaaa atcccactgc    1200
   attgccgaag tggaaaatga tgagatgcct gctgacttgc cttcattagc tgctgatttt    1260
   gttgaaagta aggatgtttg caaaaactat gctgaggcaa aggatgtctt cctgggcatg    1320
   tttttgtatg aatatgcaag aaggcatcct gattactctg tcgtgctgct gctgagactt    1380
   gccaagacat atgaaaccac tctagagaag tgctgtgccg ctgcagatcc tcatgaatgc    1440
   tatgccaaag tgttcgatga atttaaacct cttgtggaag agcctcagaa tttaatcaaa    1500
   caaaattgtg agctttttga gcagcttgga gagtacaaat tccagaatgc gctattagtt    1560
   cgttacacca agaaagtacc ccaagtgtca actccaactc ttgtagaggt ctcaagaaac    1620
   ctaggaaaag tgggcagcaa atgttgtaaa catcctgaag caaaaagaat gccctgtgca    1680
   gaagactatc tatccgtggt cctgaaccag ttatgtgtgt tgcatgagaa aacgccagta    1740
   agtgacagag tcaccaaatg ctgcacagaa tccttggtga acaggcgacc atgcttttca    1800
   gctctggaag tcgatgaaac atacgttccc aaagagttta atgctgaaac gttcaccttc    1860
   catgcagata tatgcacact ttctgagaag gagagacaaa tcaagaaaca aactgcactt    1920
   gttgagcttg tgaaacacaa gcccaaggca acaaaagagc aactgaaagc tgttatggat    1980
   gatttcgcag cttttgtaga gaagtgctgc aaggctgacg ataaggagac ctgctttgcc    2040
   gaggagggta aaaaacttgt tgctgcaagt caagctgcct taggcttaca ccaccaccac    2100
   caccactga                                                            2109
<210> 20
   <211> 702
   <212> PRT
   <213> amino acid sequence of ss-TMP-L2-TMP-L5-H-fip-HSA-His
<400> 20
   Met Glu Thr Asp Thr Leu Leu Leu Trp Val Leu Leu Leu Trp Val Pro
   1               5                   10                  15
   Gly Ser Thr Gly Asp Gly Ile Glu Gly Pro Thr Leu Arg Gln Trp Leu
               20                  25                  30
   Ala Ala Arg Ala Ser Gly Ile Glu Gly Pro Thr Leu Arg Gln Trp Leu
           35                  40                  45
   Ala Ala Arg Ala Gly Gly Gly Gly Ser Arg Ser Glu Pro Lys Ser Ser
       50                  55                  60
   Asp Lys Thr His Thr Cys Pro Pro Cys Val Ser Arg Asp Glu Leu Met
   65                  70                  75                  80
   Glu Ala Ile Gln Lys Gln Glu Glu Ile Asn Phe Arg Leu Gln Asp Tyr
                   85                  90                  95
   Ile Asp Arg Ile Ile Val Ala Ile Met Glu Thr Asn Pro Ser Ile Asp
               100                 105                 110
   Ala His Lys Ser Glu Val Ala His Arg Phe Lys Asp Leu Gly Glu Glu
           115                 120                 125
   Asn Phe Lys Ala Leu Val Leu Ile Ala Phe Ala Gln Tyr Leu Gln Gln
       130                 135                 140
   Cys Pro Phe Glu Asp His Val Lys Leu Val Asn Glu Val Thr Glu Phe
   145                 150                 155                 160
   Ala Lys Thr Cys Val Ala Asp Glu Ser Ala Glu Asn Cys Asp Lys Ser
                   165                 170                 175
   Leu His Thr Leu Phe Gly Asp Lys Leu Cys Thr Val Ala Thr Leu Arg
               180                 185                 190
   Glu Thr Tyr Gly Glu Met Ala Asp Cys Cys Ala Lys Gln Glu Pro Glu
           195                 200                 205
   Arg Asn Glu Cys Phe Leu Gln His Lys Asp Asp Asn Pro Asn Leu Pro
       210                 215                 220
   Arg Leu Val Arg Pro Glu Val Asp Val Met Cys Thr Ala Phe His Asp
   225                 230                 235                 240
   Asn Glu Glu Thr Phe Leu Lys Lys Tyr Leu Tyr Glu Ile Ala Arg Arg
                   245                 250                 255
   His Pro Tyr Phe Tyr Ala Pro Glu Leu Leu Phe Phe Ala Lys Arg Tyr
               260                 265                 270
   Lys Ala Ala Phe Thr Glu Cys Cys Gln Ala Ala Asp Lys Ala Ala Cys
           275                 280                 285
   Leu Leu Pro Lys Leu Asp Glu Leu Arg Asp Glu Gly Lys Ala Ser Ser
       290                 295                 300
   Ala Lys Gln Arg Leu Lys Cys Ala Ser Leu Gln Lys Phe Gly Glu Arg
   305                 310                 315                 320
   Ala Phe Lys Ala Trp Ala Val Ala Arg Leu Ser Gln Arg Phe Pro Lys
                   325                 330                 335
   Ala Glu Phe Ala Glu Val Ser Lys Leu Val Thr Asp Leu Thr Lys Val
               340                 345                 350
   His Thr Glu Cys Cys His Gly Asp Leu Leu Glu Cys Ala Asp Asp Arg
           355                 360                 365
   Ala Asp Leu Ala Lys Tyr Ile Cys Glu Asn Gln Asp Ser Ile Ser Ser
       370                 375                 380
   Lys Leu Lys Glu Cys Cys Glu Lys Pro Leu Leu Glu Lys Ser His Cys
   385                 390                 395                 400
   Ile Ala Glu Val Glu Asn Asp Glu Met Pro Ala Asp Leu Pro Ser Leu
                   405                 410                 415
   Ala Ala Asp Phe Val Glu Ser Lys Asp Val Cys Lys Asn Tyr Ala Glu
               420                 425                 430
   Ala Lys Asp Val Phe Leu Gly Met Phe Leu Tyr Glu Tyr Ala Arg Arg
           435                 440                 445
   His Pro Asp Tyr Ser Val Val Leu Leu Leu Arg Leu Ala Lys Thr Tyr
       450                 455                 460
   Glu Thr Thr Leu Glu Lys Cys Cys Ala Ala Ala Asp Pro His Glu Cys
   465                 470                 475                 480
   Tyr Ala Lys Val Phe Asp Glu Phe Lys Pro Leu Val Glu Glu Pro Gln
                   485                 490                 495
   Asn Leu Ile Lys Gln Asn Cys Glu Leu Phe Glu Gln Leu Gly Glu Tyr
               500                 505                 510
   Lys Phe Gln Asn Ala Leu Leu Val Arg Tyr Thr Lys Lys Val Pro Gln
           515                 520                 525
   Val Ser Thr Pro Thr Leu Val Glu Val Ser Arg Asn Leu Gly Lys Val
       530                 535                 540
   Gly Ser Lys Cys Cys Lys His Pro Glu Ala Lys Arg Met Pro Cys Ala
   545                 550                 555                 560
   Glu Asp Tyr Leu Ser Val Val Leu Asn Gln Leu Cys Val Leu His Glu
                   565                 570                 575
   Lys Thr Pro Val Ser Asp Arg Val Thr Lys Cys Cys Thr Glu Ser Leu
               580                 585                 590
   Val Asn Arg Arg Pro Cys Phe Ser Ala Leu Glu Val Asp Glu Thr Tyr
           595                 600                 605
   Val Pro Lys Glu Phe Asn Ala Glu Thr Phe Thr Phe His Ala Asp Ile
       610                 615                 620
   Cys Thr Leu Ser Glu Lys Glu Arg Gln Ile Lys Lys Gln Thr Ala Leu
   625                 630                 635                 640
   Val Glu Leu Val Lys His Lys Pro Lys Ala Thr Lys Glu Gln Leu Lys
                   645                 650                 655
   Ala Val Met Asp Asp Phe Ala Ala Phe Val Glu Lys Cys Cys Lys Ala
               660                 665                 670
   Asp Asp Lys Glu Thr Cys Phe Ala Glu Glu Gly Lys Lys Leu Val Ala
           675                 680                 685
   Ala Ser Gln Ala Ala Leu Gly Leu His His His His His His
       690                 695                 700
<210> 21
   <211> 2118
   <212> DNA
   <213> gene sequence of ss-TMP-L3-TMP-L5-H-fip-HSA-His
<400> 21
   atggagacag acacactcct gctatgggta ctgctgctct gggttccagg ttccactggt        60
   gacggtatcg aaggccccac actgcggcag tggctggctg ctagggccgg tggcccctcc       120
   ggaatcgagg gacccaccct gaggcagtgg ctggccgcca gagccggcgg cggcggaagc       180
   agatctgagc ccaagagcag cgacaaaact cacacatgcc caccgtgcgt gagcagggac       240
   gagctgatgg aggccatcca gaagcaggag gagatcaact tcaggctgca ggactacatc       300
   gacaggatca tcgtggccat catggagacc aaccccagca tcgatgcaca caagagtgag       360
   gttgctcatc ggtttaaaga tttgggagaa gaaaatttca aagccttggt gttgattgcc       420
   tttgctcagt atcttcagca gtgtccattt gaagatcatg taaaattagt gaatgaagta       480
   actgaatttg caaaaacatg tgttgctgat gagtcagctg aaaattgtga caaatcactt      540
   catacccttt ttggagacaa attatgcaca gttgcaactc ttcgtgaaac ctatggtgaa      600
   atggctgact gttgtgcaaa acaagaacct gagagaaatg aatgcttctt gcaacacaaa      660
   gatgacaacc caaacctccc ccgattggtg agaccagagg ttgatgtgat gtgcactgct      720
   tttcatgaca atgaagagac atttttgaaa aaatacttat atgaaattgc cagaagacat      780
   ccttactttt atgccccgga actccttttc tttgctaaaa ggtataaagc tgcttttaca      840
   gaatgttgcc aagctgctga taaagctgcc tgcctgttgc caaagctcga tgaacttcgg      900
   gatgaaggga aggcttcgtc tgccaaacag agactcaagt gtgccagtct ccaaaaattt      960
   ggagaaagag ctttcaaagc atgggcagta gctcgcctga gccagagatt tcccaaagct     1020
   gagtttgcag aagtttccaa gttagtgaca gatcttacca aagtccacac ggaatgctgc     1080
   catggagatc tgcttgaatg tgctgatgac agggcggacc ttgccaagta tatctgtgaa     1140
   aatcaagatt cgatctccag taaactgaag gaatgctgtg aaaaacctct gttggaaaaa     1200
   tcccactgca ttgccgaagt ggaaaatgat gagatgcctg ctgacttgcc ttcattagct     1260
   gctgattttg ttgaaagtaa ggatgtttgc aaaaactatg ctgaggcaaa ggatgtcttc     1320
   ctgggcatgt ttttgtatga atatgcaaga aggcatcctg attactctgt cgtgctgctg     1380
   ctgagacttg ccaagacata tgaaaccact ctagagaagt gctgtgccgc tgcagatcct     1440
   catgaatgct atgccaaagt gttcgatgaa tttaaacctc ttgtggaaga gcctcagaat     1500
   ttaatcaaac aaaattgtga gctttttgag cagcttggag agtacaaatt ccagaatgcg     1560
   ctattagttc gttacaccaa gaaagtaccc caagtgtcaa ctccaactct tgtagaggtc     1620
   tcaagaaacc taggaaaagt gggcagcaaa tgttgtaaac atcctgaagc aaaaagaatg     1680
   ccctgtgcag aagactatct atccgtggtc ctgaaccagt tatgtgtgtt gcatgagaaa     1740
   acgccagtaa gtgacagagt caccaaatgc tgcacagaat ccttggtgaa caggcgacca     1800
   tgcttttcag ctctggaagt cgatgaaaca tacgttccca aagagtttaa tgctgaaacg     1860
   ttcaccttcc atgcagatat atgcacactt tctgagaagg agagacaaat caagaaacaa     1920
   actgcacttg ttgagcttgt gaaacacaag cccaaggcaa caaaagagca actgaaagct     1980
   gttatggatg atttcgcagc ttttgtagag aagtgctgca aggctgacga taaggagacc     2040
   tgctttgccg aggagggtaa aaaacttgtt gctgcaagtc aagctgcctt aggcttacac     2100
   caccaccacc accactga                                                   2118
<210> 22
   <211> 705
   <212> PRT
   <213> amino acid sequence of ss-TMP-L3-TMP-L5-H-fip-HSA-His
<400> 22
   Met Glu Thr Asp Thr Leu Leu Leu Trp Val Leu Leu Leu Trp Val Pro
   1               5                   10                  15
   Gly Ser Thr Gly Asp Gly Ile Glu Gly Pro Thr Leu Arg Gln Trp Leu
               20                  25                  30
   Ala Ala Arg Ala Gly Gly Pro Ser Gly Ile Glu Gly Pro Thr Leu Arg
           35                  40                  45
   Gln Trp Leu Ala Ala Arg Ala Gly Gly Gly Gly Ser Arg Ser Glu Pro
       50                  55                  60
   Lys Ser Ser Asp Lys Thr His Thr Cys Pro Pro Cys Val Ser Arg Asp
   65                  70                  75                  80
   Glu Leu Met Glu Ala Ile Gln Lys Gln Glu Glu Ile Asn Phe Arg Leu
                   85                  90                  95
   Gln Asp Tyr Ile Asp Arg Ile Ile Val Ala Ile Met Glu Thr Asn Pro
               100                 105                 110
   Ser Ile Asp Ala His Lys Ser Glu Val Ala His Arg Phe Lys Asp Leu
           115                 120                 125
   Gly Glu Glu Asn Phe Lys Ala Leu Val Leu Ile Ala Phe Ala Gln Tyr
       130                 135                 140
   Leu Gln Gln Cys Pro Phe Glu Asp His Val Lys Leu Val Asn Glu Val
   145                 150                 155                 160
   Thr Glu Phe Ala Lys Thr Cys Val Ala Asp Glu Ser Ala Glu Asn Cys
                   165                 170                 175
   Asp Lys Ser Leu His Thr Leu Phe Gly Asp Lys Leu Cys Thr Val Ala
               180                 185                 190
   Thr Leu Arg Glu Thr Tyr Gly Glu Met Ala Asp Cys Cys Ala Lys Gln
           195                 200                 205
   Glu Pro Glu Arg Asn Glu Cys Phe Leu Gln His Lys Asp Asp Asn Pro
       210                 215                 220
   Asn Leu Pro Arg Leu Val Arg Pro Glu Val Asp Val Met Cys Thr Ala
   225                 230                 235                 240
   Phe His Asp Asn Glu Glu Thr Phe Leu Lys Lys Tyr Leu Tyr Glu Ile
                   245                 250                 255
   Ala Arg Arg His Pro Tyr Phe Tyr Ala Pro Glu Leu Leu Phe Phe Ala
               260                 265                 270
   Lys Arg Tyr Lys Ala Ala Phe Thr Glu Cys Cys Gln Ala Ala Asp Lys
           275                 280                 285
   Ala Ala Cys Leu Leu Pro Lys Leu Asp Glu Leu Arg Asp Glu Gly Lys
       290                 295                 300
   Ala Ser Ser Ala Lys Gln Arg Leu Lys Cys Ala Ser Leu Gln Lys Phe
   305                 310                 315                 320
   Gly Glu Arg Ala Phe Lys Ala Trp Ala Val Ala Arg Leu Ser Gln Arg
                   325                 330                 335
   Phe Pro Lys Ala Glu Phe Ala Glu Val Ser Lys Leu Val Thr Asp Leu
               340                 345                 350
   Thr Lys Val His Thr Glu Cys Cys His Gly Asp Leu Leu Glu Cys Ala
           355                 360                 365
   Asp Asp Arg Ala Asp Leu Ala Lys Tyr Ile Cys Glu Asn Gln Asp Ser
       370                 375                 380
   Ile Ser Ser Lys Leu Lys Glu Cys Cys Glu Lys Pro Leu Leu Glu Lys
   385                 390                 395                 400
   Ser His Cys Ile Ala Glu Val Glu Asn Asp Glu Met Pro Ala Asp Leu
                   405                 410                 415
   Pro Ser Leu Ala Ala Asp Phe Val Glu Ser Lys Asp Val Cys Lys Asn
               420                 425                 430
   Tyr Ala Glu Ala Lys Asp Val Phe Leu Gly Met Phe Leu Tyr Glu Tyr
           435                 440                 445
   Ala Arg Arg His Pro Asp Tyr Ser Val Val Leu Leu Leu Arg Leu Ala
       450                 455                 460
   Lys Thr Tyr Glu Thr Thr Leu Glu Lys Cys Cys Ala Ala Ala Asp Pro
   465                 470                 475                 480
   His Glu Cys Tyr Ala Lys Val Phe Asp Glu Phe Lys Pro Leu Val Glu
                   485                 490                 495
   Glu Pro Gln Asn Leu Ile Lys Gln Asn Cys Glu Leu Phe Glu Gln Leu
                   500                 505                 510
   Gly Glu Tyr Lys Phe Gln Asn Ala Leu Leu Val Arg Tyr Thr Lys Lys
           515                 520                 525
   Val Pro Gln Val Ser Thr Pro Thr Leu Val Glu Val Ser Arg Asn Leu
       530                 535                 540
   Gly Lys Val Gly Ser Lys Cys Cys Lys His Pro Glu Ala Lys Arg Met
   545                 550                 555                 560
   Pro Cys Ala Glu Asp Tyr Leu Ser Val Val Leu Asn Gln Leu Cys Val
                   565                 570                 575
   Leu His Glu Lys Thr Pro Val Ser Asp Arg Val Thr Lys Cys Cys Thr
               580                 585                 590
   Glu Ser Leu Val Asn Arg Arg Pro Cys Phe Ser Ala Leu Glu Val Asp
           595                 600                 605
   Glu Thr Tyr Val Pro Lys Glu Phe Asn Ala Glu Thr Phe Thr Phe His
       610                 615                 620
   Ala Asp Ile Cys Thr Leu Ser Glu Lys Glu Arg Gln Ile Lys Lys Gln
   625                 630                 635                 640
   Thr Ala Leu Val Glu Leu Val Lys His Lys Pro Lys Ala Thr Lys Glu
                   645                 650                 655
   Gln Leu Lys Ala Val Met Asp Asp Phe Ala Ala Phe Val Glu Lys Cys
               660                 665                 670
   Cys Lys Ala Asp Asp Lys Glu Thr Cys Phe Ala Glu Glu Gly Lys Lys
           675                 680                 685
   Leu Val Ala Ala Ser Gln Ala Ala Leu Gly Leu His His His His His
       690                 695                 700
   His
   705
<210> 23
   <211> 2133
   <212> DNA
   <213> gene sequence of ss-TMP-L4-TMP-L5-H-fip-HSA-His
<400> 23
   atggagacag acacactcct gctatgggta ctgctgctct gggttccagg ttccactggt 60
   gacggtatcg aaggccccac actgcggcag tggctggctg ctagggccgg cggtggcggc 120
   agcggtggcg gctccggaat cgagggaccc accctgaggc agtggctggc cgccagagcc 180
   ggcggcggcg gaagcagatc tgagcccaag agcagcgaca aaactcacac atgcccaccg 240
   tgcgtgagca gggacgagct gatggaggcc atccagaagc aggaggagat caacttcagg 300
   ctgcaggact acatcgacag gatcatcgtg gccatcatgg agaccaaccc cagcatcgat 360
   gcacacaaga gtgaggttgc tcatcggttt aaagatttgg gagaagaaaa tttcaaagcc 420
   ttggtgttga ttgcctttgc tcagtatctt cagcagtgtc catttgaaga tcatgtaaaa 480
   ttagtgaatg aagtaactga atttgcaaaa acatgtgttg ctgatgagtc agctgaaaat 540
   tgtgacaaat cacttcatac cctttttgga gacaaattat gcacagttgc aactcttcgt 600
   gaaacctatg gtgaaatggc tgactgttgt gcaaaacaag aacctgagag aaatgaatgc 660
   ttcttgcaac acaaagatga caacccaaac ctcccccgat tggtgagacc agaggttgat 720
   gtgatgtgca ctgcttttca tgacaatgaa gagacatttt tgaaaaaata cttatatgaa 780
   attgccagaa gacatcctta cttttatgcc ccggaactcc ttttctttgc taaaaggtat 840
   aaagctgctt ttacagaatg ttgccaagct gctgataaag ctgcctgcct gttgccaaag 900
   ctcgatgaac ttcgggatga agggaaggct tcgtctgcca aacagagact caagtgtgcc 960
   agtctccaaa aatttggaga aagagctttc aaagcatggg cagtagctcg cctgagccag 1020
   agatttccca aagctgagtt tgcagaagtt tccaagttag tgacagatct taccaaagtc 1080
   cacacggaat gctgccatgg agatctgctt gaatgtgctg atgacagggc ggaccttgcc 1140
   aagtatatct gtgaaaatca agattcgatc tccagtaaac tgaaggaatg ctgtgaaaaa 1200
   cctctgttgg aaaaatccca ctgcattgcc gaagtggaaa atgatgagat gcctgctgac 1260
   ttgccttcat tagctgctga ttttgttgaa agtaaggatg tttgcaaaaa ctatgctgag 1320
   gcaaaggatg tcttcctggg catgtttttg tatgaatatg caagaaggca tcctgattac 1380
   tctgtcgtgc tgctgctgag acttgccaag acatatgaaa ccactctaga gaagtgctgt 1440
   gccgctgcag atcctcatga atgctatgcc aaagtgttcg atgaatttaa acctcttgtg 1500
   gaagagcctc agaatttaat caaacaaaat tgtgagcttt ttgagcagct tggagagtac 1560
   aaattccaga atgcgctatt agttcgttac accaagaaag taccccaagt gtcaactcca 1620
   actcttgtag aggtctcaag aaacctagga aaagtgggca gcaaatgttg taaacatcct 1680
   gaagcaaaaa gaatgccctg tgcagaagac tatctatccg tggtcctgaa ccagttatgt 1740
   gtgttgcatg agaaaacgcc agtaagtgac agagtcacca aatgctgcac agaatccttg 1800
   gtgaacaggc gaccatgctt ttcagctctg gaagtcgatg aaacatacgt tcccaaagag 1860
   tttaatgctg aaacgttcac cttccatgca gatatatgca cactttctga gaaggagaga 1920
   caaatcaaga aacaaactgc acttgttgag cttgtgaaac acaagcccaa ggcaacaaaa 1980
   gagcaactga aagctgttat ggatgatttc gcagcttttg tagagaagtg ctgcaaggct 2040
   gacgataagg agacctgctt tgccgaggag ggtaaaaaac ttgttgctgc aagtcaagct 2100
   gccttaggct tacaccacca ccaccaccac tga 2133
<210> 24
   <211> 710
   <212> PRT
   <213> amino acid sequence of ss-TMP-L4-TMP-L5-H-fip-HSA-His
<400> 24
   Met Glu Thr Asp Thr Leu Leu Leu Trp Val Leu Leu Leu Trp Val Pro
   1               5                   10                  15
   Gly Ser Thr Gly Asp Gly Ile Glu Gly Pro Thr Leu Arg Gln Trp Leu
               20                  25                  30
   Ala Ala Arg Ala Gly Gly Gly Gly Ser Gly Gly Gly Ser Gly Ile Glu
           35                  40                  45
   Gly Pro Thr Leu Arg Gln Trp Leu Ala Ala Arg Ala Gly Gly Gly Gly
       50                  55                  60
   Ser Arg Ser Glu Pro Lys Ser Ser Asp Lys Thr His Thr Cys Pro Pro
   65                  70                  75                  80
   Cys Val Ser Arg Asp Glu Leu Met Glu Ala Ile Gln Lys Gln Glu Glu
                   85                  90                  95
   Ile Asn Phe Arg Leu Gln Asp Tyr Ile Asp Arg Ile Ile Val Ala Ile
               100                 105                 110
   Met Glu Thr Asn Pro Ser Ile Asp Ala His Lys Ser Glu Val Ala His
           115                 120                 125
   Arg Phe Lys Asp Leu Gly Glu Glu Asn Phe Lys Ala Leu Val Leu Ile
       130                 135                 140
   Ala Phe Ala Gln Tyr Leu Gln Gln Cys Pro Phe Glu Asp His Val Lys
   145                 150                 155                 160
   Leu Val Asn Glu Val Thr Glu Phe Ala Lys Thr Cys Val Ala Asp Glu
                   165                 170                 175
   Ser Ala Glu Asn Cys Asp Lys Ser Leu His Thr Leu Phe Gly Asp Lys
               180                 185                 190
   Leu Cys Thr Val Ala Thr Leu Arg Glu Thr Tyr Gly Glu Met Ala Asp
           195                 200                 205
   Cys Cys Ala Lys Gln Glu Pro Glu Arg Asn Glu Cys Phe Leu Gln His
       210                 215                 220
   Lys Asp Asp Asn Pro Asn Leu Pro Arg Leu Val Arg Pro Glu Val Asp
   225                 230                 235                 240
   Val Met Cys Thr Ala Phe His Asp Asn Glu Glu Thr Phe Leu Lys Lys
                   245                 250                 255
   Tyr Leu Tyr Glu Ile Ala Arg Arg His Pro Tyr Phe Tyr Ala Pro Glu
               260                 265                 270
   Leu Leu Phe Phe Ala Lys Arg Tyr Lys Ala Ala Phe Thr Glu Cys Cys
           275                 280                 285
   Gln Ala Ala Asp Lys Ala Ala Cys Leu Leu Pro Lys Leu Asp Glu Leu
       290                 295                 300
   Arg Asp Glu Gly Lys Ala Ser Ser Ala Lys Gln Arg Leu Lys Cys Ala
   305                 310                 315                 320
   Ser Leu Gln Lys Phe Gly Glu Arg Ala Phe Lys Ala Trp Ala Val Ala
                   325                 330                 335
   Arg Leu Ser Gln Arg Phe Pro Lys Ala Glu Phe Ala Glu Val Ser Lys
               340                 345                 350
   Leu Val Thr Asp Leu Thr Lys Val His Thr Glu Cys Cys His Gly Asp
           355                 360                 365
   Leu Leu Glu Cys Ala Asp Asp Arg Ala Asp Leu Ala Lys Tyr Ile Cys
       370                 375                 380
   Glu Asn Gln Asp Ser Ile Ser Ser Lys Leu Lys Glu Cys Cys Glu Lys
   385                 390                 395                 400
   Pro Leu Leu Glu Lys Ser His Cys Ile Ala Glu Val Glu Asn Asp Glu
                   405                 410                 415
   Met Pro Ala Asp Leu Pro Ser Leu Ala Ala Asp Phe Val Glu Ser Lys
               420                 425                 430
   Asp Val Cys Lys Asn Tyr Ala Glu Ala Lys Asp Val Phe Leu Gly Met
           435                 440                 445
   Phe Leu Tyr Glu Tyr Ala Arg Arg His Pro Asp Tyr Ser Val Val Leu
       450                 455                 460
   Leu Leu Arg Leu Ala Lys Thr Tyr Glu Thr Thr Leu Glu Lys Cys Cys
   465                 470                 475                 480
   Ala Ala Ala Asp Pro His Glu Cys Tyr Ala Lys Val Phe Asp Glu Phe
                   485                 490                 495
   Lys Pro Leu Val Glu Glu Pro Gln Asn Leu Ile Lys Gln Asn Cys Glu
               500                 505                 510
   Leu Phe Glu Gln Leu Gly Glu Tyr Lys Phe Gln Asn Ala Leu Leu Val
           515                 520                 525
   Arg Tyr Thr Lys Lys Val Pro Gln Val Ser Thr Pro Thr Leu Val Glu
       530                 535                 540
   Val Ser Arg Asn Leu Gly Lys Val Gly Ser Lys Cys Cys Lys His Pro
   545                 550                 555                 560
   Glu Ala Lys Arg Met Pro Cys Ala Glu Asp Tyr Leu Ser Val Val Leu
                   565                 570                 575
   Asn Gln Leu Cys Val Leu His Glu Lys Thr Pro Val Ser Asp Arg Val
               580                 585                 590
   Thr Lys Cys Cys Thr Glu Ser Leu Val Asn Arg Arg Pro Cys Phe Ser
           595                 600                 605
   Ala Leu Glu Val Asp Glu Thr Tyr Val Pro Lys Glu Phe Asn Ala Glu
       610                 615                 620
   Thr Phe Thr Phe His Ala Asp Ile Cys Thr Leu Ser Glu Lys Glu Arg
   625                 630                 635                 640
   Gln Ile Lys Lys Gln Thr Ala Leu Val Glu Leu Val Lys His Lys Pro
                   645                 650                 655
   Lys Ala Thr Lys Glu Gln Leu Lys Ala Val Met Asp Asp Phe Ala Ala
               660                 665                 670
   Phe Val Glu Lys Cys Cys Lys Ala Asp Asp Lys Glu Thr Cys Phe Ala
           675                 680                 685
   Glu Glu Gly Lys Lys Leu Val Ala Ala Ser Gln Ala Ala Leu Gly Leu
       690                 695                 700
   His His His His His His
   705                 710
<210> 25
   <211> 709
   <212> DNA
   <213> gene sequence of HSA(Post2)-His
<400> 25
   tctagagaag tgctgtgccg ctgcagatcc tcatgaatgc tatgccaaag tgttcgatga      60
   atttaaacct cttgtggaag agcctcagaa tttaatcaaa caaaattgtg agctttttga     120
   gcagcttgga gagtacaaat tccagaatgc gctattagtt cgttacacca agaaagtacc     180
   ccaagtgtca actccaactc ttgtagaggt ctcaagaaac ctaggaaaag tgggcagcaa     240
   atgttgtaaa catcctgaag caaaaagaat gccctgtgca gaagactatc tatccgtggt     300
   cctgaaccag ttatgtgtgt tgcatgagaa aacgccagta agtgacagag tcaccaaatg     360
   ctgcacagaa tccttggtga acaggcgacc atgcttttca gctctggaag tcgatgaaac     420
   atacgttccc aaagagttta atgctgaaac gttcaccttc catgcagata tatgcacact     480
   ttctgagaag gagagacaaa tcaagaaaca aactgcactt gttgagcttg tgaaacacaa     540
   gcccaaggca acaaaagagc aactgaaagc tgttatggat gatttcgcag cttttgtaga     600
   gaagtgctgc aaggctgacg ataaggagac ctgctttgcc gaggagggta aaaaacttgt     660
   tgctgcaagt caagctgcct taggcttaca ccaccaccac caccactga                 709
<210> 26
   <211> 235
   <212> PRT
   <213> amino acid sequence of HSA(Post2)-His
<400> 26
   Leu Glu Lys Cys Cys Ala Ala Ala Asp Pro His Glu Cys Tyr Ala Lys
   1               5                   10                  15
   Val Phe Asp Glu Phe Lys Pro Leu Val Glu Glu Pro Gln Asn Leu Ile
               20                  25                  30
   Lys Gln Asn Cys Glu Leu Phe Glu Gln Leu Gly Glu Tyr Lys Phe Gln
           35                  40                  45
   Asn Ala Leu Leu Val Arg Tyr Thr Lys Lys Val Pro Gln Val Ser Thr
       50                  55                  60
   Pro Thr Leu Val Glu Val Ser Arg Asn Leu Gly Lys Val Gly Ser Lys
   65                  70                  75                  80
   Cys Cys Lys His Pro Glu Ala Lys Arg Met Pro Cys Ala Glu Asp Tyr
                   85                  90                  95
   Leu Ser Val Val Leu Asn Gln Leu Cys Val Leu His Glu Lys Thr Pro
               100                 105                 110
   Val Ser Asp Arg Val Thr Lys Cys Cys Thr Glu Ser Leu Val Asn Arg
           115                 120                 125
   Arg Pro Cys Phe Ser Ala Leu Glu Val Asp Glu Thr Tyr Val Pro Lys
       130                 135                 140
   Glu Phe Asn Ala Glu Thr Phe Thr Phe His Ala Asp Ile Cys Thr Leu
   145                 150                 155                 160
   Ser Glu Lys Glu Arg Gln Ile Lys Lys Gln Thr Ala Leu Val Glu Leu
                   165                 170                 175
   Val Lys His Lys Pro Lys Ala Thr Lys Glu Gln Leu Lys Ala Val Met
               180                 185                 190
   Asp Asp Phe Ala Ala Phe Val Glu Lys Cys Cys Lys Ala Asp Asp Lys
           195                 200                 205
   Glu Thr Cys Phe Ala Glu Glu Gly Lys Lys Leu Val Ala Ala Ser Gln
       210                 215                 220
   Ala Ala Leu Gly Leu His His His His His His
   225                 230                 235
<210> 27
   <211> 1071
   <212> DNA
   <213> gene sequence of HSA(Mid)
<400> 27
   tgcacacaag agtgaggttg ctcatcggtt taaagatttg ggagaagaaa atttcaaagc      60
   cttggtgttg attgcctttg ctcagtatct tcagcagtgt ccatttgaag atcatgtaaa     120
   attagtgaat gaagtaactg aatttgcaaa aacatgtgtt gctgatgagt cagctgaaaa     180
   ttgtgacaaa tcacttcata ccctttttgg agacaaatta tgcacagttg caactcttcg     240
   tgaaacctat ggtgaaatgg ctgactgttg tgcaaaacaa gaacctgaga gaaatgaatg     300
   cttcttgcaa cacaaagatg acaacccaaa cctcccccga ttggtgagac cagaggttga     360
   tgtgatgtgc actgcttttc atgacaatga agagacattt ttgaaaaaat acttatatga     420
   aattgccaga agacatcctt acttttatgc cccggaactc cttttctttg ctaaaaggta     480
   taaagctgct tttacagaat gttgccaagc tgctgataaa gctgcctgcc tgttgccaaa     540
   gctcgatgaa cttcgggatg aagggaaggc ttcgtctgcc aaacagagac tcaagtgtgc     600
   cagtctccaa aaatttggag aaagagcttt caaagcatgg gcagtagctc gcctgagcca     660
   gagatttccc aaagctgagt ttgcagaagt ttccaagtta gtgacagatc ttaccaaagt     720
   ccacacggaa tgctgccatg gagatctgct tgaatgtgct gatgacaggg cggaccttgc     780
   caagtatatc tgtgaaaatc aagattcgat ctccagtaaa ctgaaggaat gctgtgaaaa     840
   acctctgttg gaaaaatccc actgcattgc cgaagtggaa aatgatgaga tgcctgctga     900
   cttgccttca ttagctgctg attttgttga aagtaaggat gtttgcaaaa actatgctga     960
   ggcaaaggat gtcttcctgg gcatgttttt gtatgaatat gcaagaaggc atcctgatta    1020
   ctctgtcgtg ctgctgctga gacttgccaa gacatatgaa accactctag a             1071
<210> 28
   <211> 356
   <212> PRT
   <213> amino acid sequence of HSA(Mid)
<400> 28
   Ala His Lys Ser Glu Val Ala His Arg Phe Lys Asp Leu Gly Glu Glu
   1               5                   10                  15
   Asn Phe Lys Ala Leu Val Leu Ile Ala Phe Ala Gln Tyr Leu Gln Gln
               20                  25                  30
   Cys Pro Phe Glu Asp His Val Lys Leu Val Asn Glu Val Thr Glu Phe
           35                  40                  45
   Ala Lys Thr Cys Val Ala Asp Glu Ser Ala Glu Asn Cys Asp Lys Ser
       50                  55                  60
   Leu His Thr Leu Phe Gly Asp Lys Leu Cys Thr Val Ala Thr Leu Arg
   65                  70                  75                  80
   Glu Thr Tyr Gly Glu Met Ala Asp Cys Cys Ala Lys Gln Glu Pro Glu
                   85                  90                  95
   Arg Asn Glu Cys Phe Leu Gln His Lys Asp Asp Asn Pro Asn Leu Pro
               100                 105                 110
   Arg Leu Val Arg Pro Glu Val Asp Val Met Cys Thr Ala Phe His Asp
           115                 120                 125
   Asn Glu Glu Thr Phe Leu Lys Lys Tyr Leu Tyr Glu Ile Ala Arg Arg
       130                 135                 140
   His Pro Tyr Phe Tyr Ala Pro Glu Leu Leu Phe Phe Ala Lys Arg Tyr
   145                 150                 155                 160
   Lys Ala Ala Phe Thr Glu Cys Cys Gln Ala Ala Asp Lys Ala Ala Cys
                   165                 170                 175
   Leu Leu Pro Lys Leu Asp Glu Leu Arg Asp Glu Gly Lys Ala Ser Ser
               180                 185                 190
   Ala Lys Gln Arg Leu Lys Cys Ala Ser Leu Gln Lys Phe Gly Glu Arg
           195                 200                 205
   Ala Phe Lys Ala Trp Ala Val Ala Arg Leu Ser Gln Arg Phe Pro Lys
       210                 215                 220
   Ala Glu Phe Ala Glu Val Ser Lys Leu Val Thr Asp Leu Thr Lys Val
   225                 230                 235                 240
   His Thr Glu Cys Cys His Gly Asp Leu Leu Glu Cys Ala Asp Asp Arg
                   245                 250                 255
   Ala Asp Leu Ala Lys Tyr Ile Cys Glu Asn Gln Asp Ser Ile Ser Ser
               260                 265                 270
   Lys Leu Lys Glu Cys Cys Glu Lys Pro Leu Leu Glu Lys Ser His Cys
           275                 280                 285
   Ile Ala Glu Val Glu Asn Asp Glu Met Pro Ala Asp Leu Pro Ser Leu
       290                 295                 300
   Ala Ala Asp Phe Val Glu Ser Lys Asp Val Cys Lys Asn Tyr Ala Glu
   305                 310                 315                 320
   Ala Lys Asp Val Phe Leu Gly Met Phe Leu Tyr Glu Tyr Ala Arg Arg
                   325                 330                 335
   His Pro Asp Tyr Ser Val Val Leu Leu Leu Arg Leu Ala Lys Thr Tyr
               340                 345                 350
   Glu Thr Thr Leu
           355
<210> 29
   <211> 101
   <212> DNA
   <213> gene sequence of ss-HSA(Pre2)
<400> 29
   atggagacag acacactcct gctatgggta ctgctgctct gggttccagg ttccactggt       60
   gacggtgatg cacacaagag tgaggttgct catcggttta a                          101
<210> 30
   <211> 33
   <212> PRT
   <213> amino acid sequence of ss-HSA(Pre2)
<400> 30
   Met Glu Thr Asp Thr Leu Leu Leu Trp Val Leu Leu Leu Trp Val Pro
   1               5                   10                  15
   Gly Ser Thr Gly Asp Gly Asp Ala His Lys Ser Glu Val Ala His Arg
               20                  25                  30
   Phe
<210> 31
   <211> 1150
   <212> DNA
   <213> gene sequence of ss-HSA(pre3)
<400> 31
   atggagacag acacactcct gctatgggta ctgctgctct gggttccagg ttccactggt      60
   gacggtgatg cacacaagag tgaggttgct catcggttta aagatttggg agaagaaaat     120
   ttcaaagcct tggtgttgat tgcctttgct cagtatcttc agcagtgtcc atttgaagat     180
   catgtaaaat tagtgaatga agtaactgaa tttgcaaaaa catgtgttgc tgatgagtca     240
   gctgaaaatt gtgacaaatc acttcatacc ctttttggag acaaattatg cacagttgca     300
   actcttcgtg aaacctatgg tgaaatggct gactgttgtg caaaacaaga acctgagaga     360
   aatgaatgct tcttgcaaca caaagatgac aacccaaacc tcccccgatt ggtgagacca     420
   gaggttgatg tgatgtgcac tgcttttcat gacaatgaag agacattttt gaaaaaatac     480
   ttatatgaaa ttgccagaag acatccttac ttttatgccc cggaactcct tttctttgct     540
   aaaaggtata aagctgcttt tacagaatgt tgccaagctg ctgataaagc tgcctgcctg     600
   ttgccaaagc tcgatgaact tcgggatgaa gggaaggctt cgtctgccaa acagagactc     660
   aagtgtgcca gtctccaaaa atttggagaa agagctttca aagcatgggc agtagctcgc     720
   ctgagccaga gatttcccaa agctgagttt gcagaagttt ccaagttagt gacagatctt     780
   accaaagtcc acacggaatg ctgccatgga gatctgcttg aatgtgctga tgacagggcg     840
   gaccttgcca agtatatctg tgaaaatcaa gattcgatct ccagtaaact gaaggaatgc     900
   tgtgaaaaac ctctgttgga aaaatcccac tgcattgccg aagtggaaaa tgatgagatg     960
   cctgctgact tgccttcatt agctgctgat tttgttgaaa gtaaggatgt ttgcaaaaac    1020
   tatgctgagg caaaggatgt cttcctgggc atgtttttgt atgaatatgc aagaaggcat    1080
   cctgattact ctgtcgtgct gctgctgaga cttgccaaga catatgaaac cactctagag    1140
   aagtgctgtg                                                           1150
<210> 32
   <211> 383
   <212> PRT
   <213> amino acid sequence of ss-HSA(pre3)
<400> 32
   Met Glu Thr Asp Thr Leu Leu Leu Trp Val Leu Leu Leu Trp Val Pro
   1               5                   10                  15
   Gly Ser Thr Gly Asp Gly Asp Ala His Lys Ser Glu Val Ala His Arg
               20                  25                  30
   Phe Lys Asp Leu Gly Glu Glu Asn Phe Lys Ala Leu Val Leu Ile Ala
           35                  40                  45
   Phe Ala Gln Tyr Leu Gln Gln Cys Pro Phe Glu Asp His Val Lys Leu
       50                  55                  60
   Val Asn Glu Val Thr Glu Phe Ala Lys Thr Cys Val Ala Asp Glu Ser
   65                  70                  75                  80
   Ala Glu Asn Cys Asp Lys Ser Leu His Thr Leu Phe Gly Asp Lys Leu
                   85                  90                  95
   Cys Thr Val Ala Thr Leu Arg Glu Thr Tyr Gly Glu Met Ala Asp Cys
               100                 105                 110
   Cys Ala Lys Gln Glu Pro Glu Arg Asn Glu Cys Phe Leu Gln His Lys
           115                 120                 125
   Asp Asp Asn Pro Asn Leu Pro Arg Leu Val Arg Pro Glu Val Asp Val
       130                 135                 140
   Met Cys Thr Ala Phe His Asp Asn Glu Glu Thr Phe Leu Lys Lys Tyr
   145                 150                 155                 160
   Leu Tyr Glu Ile Ala Arg Arg His Pro Tyr Phe Tyr Ala Pro Glu Leu
                   165                 170                 175
   Leu Phe Phe Ala Lys Arg Tyr Lys Ala Ala Phe Thr Glu Cys Cys Gln
               180                 185                 190
   Ala Ala Asp Lys Ala Ala Cys Leu Leu Pro Lys Leu Asp Glu Leu Arg
           195                 200                 205
   Asp Glu Gly Lys Ala Ser Ser Ala Lys Gln Arg Leu Lys Cys Ala Ser
       210                 215                 220
   Leu Gln Lys Phe Gly Glu Arg Ala Phe Lys Ala Trp Ala Val Ala Arg
   225                 230                 235                 240
   Leu Ser Gln Arg Phe Pro Lys Ala Glu Phe Ala Glu Val Ser Lys Leu
                   245                 250                 255
   Val Thr Asp Leu Thr Lys Val His Thr Glu Cys Cys His Gly Asp Leu
               260                 265                 270
   Leu Glu Cys Ala Asp Asp Arg Ala Asp Leu Ala Lys Tyr Ile Cys Glu
           275                 280                 285
   Asn Gln Asp Ser Ile Ser Ser Lys Leu Lys Glu Cys Cys Glu Lys Pro
       290                 295                 300
   Leu Leu Glu Lys Ser His Cys Ile Ala Glu Val Glu Asn Asp Glu Met
   305                 310                 315                 320
   Pro Ala Asp Leu Pro Ser Leu Ala Ala Asp Phe Val Glu Ser Lys Asp
                   325                 330                 335
   Val Cys Lys Asn Tyr Ala Glu Ala Lys Asp Val Phe Leu Gly Met Phe
               340                 345                 350
   Leu Tyr Glu Tyr Ala Arg Arg His Pro Asp Tyr Ser Val Val Leu Leu
           355                 360                 365
   Leu Arg Leu Ala Lys Thr Tyr Glu Thr Thr Leu Glu Lys Cys Cys
       370                 375                 380
<210> 33
   <211> 780
   <212> DNA
   <213> gene sequence of ss-HSA(pre3)
<400> 33
   gaaaccactc tagagaagtg ctgtgccgct gcagatcctc atgaatgcta tgccaaagtg        60
   ttcgatgaat ttaaacctct tgtggaagag cctcagaatt taatcaaaca aaattgtgag       120
   ctttttgagc agcttggaga gtacaaattc cagaatgcgc tattagttcg ttacaccaag       180
   aaagtacccc aagtgtcaac tccaactctt gtagaggtct caagaaacct aggaaaagtg       240
   ggcagcaaat gttgtaaaca tcctgaagca aaaagaatgc cctgtgcaga agactatcta       300
   tccgtggtcc tgaaccagtt atgtgtgttg catgagaaaa cgccagtaag tgacagagtc       360
   accaaatgct gcacagaatc cttggtgaac aggcgaccat gcttttcagc tctggaagtc       420
   gatgaaacat acgttcccaa agagtttaat gctgaaacgt tcaccttcca tgcagatata       480
   tgcacacttt ctgagaagga gagacaaatc aagaaacaaa ctgcacttgt tgagcttgtg       540
   aaacacaagc ccaaggcaac aaaagagcaa ctgaaagctg ttatggatga tttcgcagct       600
   tttgtagaga agtgctgcaa ggctgacgat aaggagacct gctttgccga ggagggtaaa       660
   aaacttgttg ctgcaagtca agctgcctta ggcttaggcg gcggcggttc cggactggag       720
   cccaagagct gcgacaagac ccacacctgc cctccctgcg aattcggtgg tggcggcagc       780
<210> 34
   <211> 260
   <212> PRT
   <213> amino acid sequence of ss-HSA(pre3)
<400> 34
   Glu Thr Thr Leu Glu Lys Cys Cys Ala Ala Ala Asp Pro His Glu Cys
   1               5                   10                  15
   Tyr Ala Lys Val Phe Asp Glu Phe Lys Pro Leu Val Glu Glu Pro Gln
               20                  25                  30
   Asn Leu Ile Lys Gln Asn Cys Glu Leu Phe Glu Gln Leu Gly Glu Tyr
           35                  40                  45
   Lys Phe Gln Asn Ala Leu Leu Val Arg Tyr Thr Lys Lys Val Pro Gln
       50                  55                  60
   Val Ser Thr Pro Thr Leu Val Glu Val Ser Arg Asn Leu Gly Lys Val
   65                  70                  75                  80
   Gly Ser Lys Cys Cys Lys His Pro Glu Ala Lys Arg Met Pro Cys Ala
                   85                  90                  95
   Glu Asp Tyr Leu Ser Val Val Leu Asn Gln Leu Cys Val Leu His Glu
               100                 105                 110
   Lys Thr Pro Val Ser Asp Arg Val Thr Lys Cys Cys Thr Glu Ser Leu
           115                 120                 125
   Val Asn Arg Arg Pro Cys Phe Ser Ala Leu Glu Val Asp Glu Thr Tyr
       130                 135                 140
   Val Pro Lys Glu Phe Asn Ala Glu Thr Phe Thr Phe His Ala Asp Ile
   145                 150                 155                 160
   Cys Thr Leu Ser Glu Lys Glu Arg Gln Ile Lys Lys Gln Thr Ala Leu
                   165                 170                 175
   Val Glu Leu Val Lys His Lys Pro Lys Ala Thr Lys Glu Gln Leu Lys
               180                 185                 190
   Ala Val Met Asp Asp Phe Ala Ala Phe Val Glu Lys Cys Cys Lys Ala
           195                 200                 205
   Asp Asp Lys Glu Thr Cys Phe Ala Glu Glu Gly Lys Lys Leu Val Ala
       210                 215                 220
   Ala Ser Gln Ala Ala Leu Gly Leu Gly Gly Gly Gly Ser Gly Leu Glu
   225                 230                 235                 240
   Pro Lys Ser Cys Asp Lys Thr His Thr Cys Pro Pro Cys Glu Phe Gly
                   245                 250                 255
   Gly Gly Gly Ser
               260
<210> 35
   <211> 2004
   <212> DNA
   <213> gene sequence of ss-HSA-L6-H-L7-TMP-L1-TMP
<400> 35
   atggagacag acacactcct gctatgggta ctgctgctct gggttccagg ttccactggt      60
   gacggtgatg cacacaagag tgaggttgct catcggttta aagatttggg agaagaaaat     120
   ttcaaagcct tggtgttgat tgcctttgct cagtatcttc agcagtgtcc atttgaagat     180
   catgtaaaat tagtgaatga agtaactgaa tttgcaaaaa catgtgttgc tgatgagtca     240
   gctgaaaatt gtgacaaatc acttcatacc ctttttggag acaaattatg cacagttgca     300
   actcttcgtg aaacctatgg tgaaatggct gactgttgtg caaaacaaga acctgagaga     360
   aatgaatgct tcttgcaaca caaagatgac aacccaaacc tcccccgatt ggtgagacca     420
   gaggttgatg tgatgtgcac tgcttttcat gacaatgaag agacattttt gaaaaaatac     480
   ttatatgaaa ttgccagaag acatccttac ttttatgccc cggaactcct tttctttgct     540
   aaaaggtata aagctgcttt tacagaatgt tgccaagctg ctgataaagc tgcctgcctg     600
   ttgccaaagc tcgatgaact tcgggatgaa gggaaggctt cgtctgccaa acagagactc     660
   aagtgtgcca gtctccaaaa atttggagaa agagctttca aagcatgggc agtagctcgc     720
   ctgagccaga gatttcccaa agctgagttt gcagaagttt ccaagttagt gacagatctt     780
   accaaagtcc acacggaatg ctgccatgga gatctgcttg aatgtgctga tgacagggcg     840
   gaccttgcca agtatatctg tgaaaatcaa gattcgatct ccagtaaact gaaggaatgc 900
   tgtgaaaaac ctctgttgga aaaatcccac tgcattgccg aagtggaaaa tgatgagatg 960
   cctgctgact tgccttcatt agctgctgat tttgttgaaa gtaaggatgt ttgcaaaaac 1020
   tatgctgagg caaaggatgt cttcctgggc atgtttttgt atgaatatgc aagaaggcat 1080
   cctgattact ctgtcgtgct gctgctgaga cttgccaaga catatgaaac cactctagag 1140
   aagtgctgtg ccgctgcaga tcctcatgaa tgctatgcca aagtgttcga tgaatttaaa 1200
   cctcttgtgg aagagcctca gaatttaatc aaacaaaatt gtgagctttt tgagcagctt 1260
   ggagagtaca aattccagaa tgcgctatta gttcgttaca ccaagaaagt accccaagtg 1320
   tcaactccaa ctcttgtaga ggtctcaaga aacctaggaa aagtgggcag caaatgttgt 1380
   aaacatcctg aagcaaaaag aatgccctgt gcagaagact atctatccgt ggtcctgaac 1440
   cagttatgtg tgttgcatga gaaaacgcca gtaagtgaca gagtcaccaa atgctgcaca 1500
   gaatccttgg tgaacaggcg accatgcttt tcagctctgg aagtcgatga aacatacgtt 1560
   cccaaagagt ttaatgctga aacgttcacc ttccatgcag atatatgcac actttctgag 1620
   aaggagagac aaatcaagaa acaaactgca cttgttgagc ttgtgaaaca caagcccaag 1680
   gcaacaaaag agcaactgaa agctgttatg gatgatttcg cagcttttgt agagaagtgc 1740
   tgcaaggctg acgataagga gacctgcttt gccgaggagg gtaaaaaact tgttgctgca 1800
   agtcaagctg ccttaggctt aggcggcggc ggttccggac tggagcccaa gagctgcgac 1860
   aagacccaca cctgccctcc ctgcgaattc ggtggtggcg gcagcatcga aggccccaca 1920
   ctgcggcagt ggctggctgc tagggccggt ggcggctccg gaatcgaggg acccaccctg 1980
   aggcagtggc tggccgccag agcc 2004
<210> 36
   <211> 668
   <212> PRT
   <213> amino acid sequence of ss-HSA-L6-H-L7-TMP-L1-TMP
<400> 36
   Met Glu Thr Asp Thr Leu Leu Leu Trp Val Leu Leu Leu Trp Val Pro
   1               5                   10                  15
   Gly Ser Thr Gly Asp Gly Asp Ala His Lys Ser Glu Val Ala His Arg
               20                  25                  30
   Phe Lys Asp Leu Gly Glu Glu Asn Phe Lys Ala Leu Val Leu Ile Ala
           35                  40                  45
   Phe Ala Gln Tyr Leu Gln Gln Cys Pro Phe Glu Asp His Val Lys Leu
       50                  55                  60
   Val Asn Glu Val Thr Glu Phe Ala Lys Thr Cys Val Ala Asp Glu Ser
   65                  70                  75                  80
   Ala Glu Asn Cys Asp Lys Ser Leu His Thr Leu Phe Gly Asp Lys Leu
                   85                  90                  95
   Cys Thr Val Ala Thr Leu Arg Glu Thr Tyr Gly Glu Met Ala Asp Cys
               100                 105                 110
   Cys Ala Lys Gln Glu Pro Glu Arg Asn Glu Cys Phe Leu Gln His Lys
           115                 120                 125
   Asp Asp Asn Pro Asn Leu Pro Arg Leu Val Arg Pro Glu Val Asp Val
       130                 135                 140
   Met Cys Thr Ala Phe His Asp Asn Glu Glu Thr Phe Leu Lys Lys Tyr
   145                 150                 155                 160
   Leu Tyr Glu Ile Ala Arg Arg His Pro Tyr Phe Tyr Ala Pro Glu Leu
                   165                 170                 175
   Leu Phe Phe Ala Lys Arg Tyr Lys Ala Ala Phe Thr Glu Cys Cys Gln
               180                 185                 190
   Ala Ala Asp Lys Ala Ala Cys Leu Leu Pro Lys Leu Asp Glu Leu Arg
           195                 200                 205
   Asp Glu Gly Lys Ala Ser Ser Ala Lys Gln Arg Leu Lys Cys Ala Ser
       210                 215                 220
   Leu Gln Lys Phe Gly Glu Arg Ala Phe Lys Ala Trp Ala Val Ala Arg
   225                 230                 235                 240
   Leu Ser Gln Arg Phe Pro Lys Ala Glu Phe Ala Glu Val Ser Lys Leu
                   245                 250                 255
   Val Thr Asp Leu Thr Lys Val His Thr Glu Cys Cys His Gly Asp Leu
               260                 265                 270
   Leu Glu Cys Ala Asp Asp Arg Ala Asp Leu Ala Lys Tyr Ile Cys Glu
           275                 280                 285
   Asn Gln Asp Ser Ile Ser Ser Lys Leu Lys Glu Cys Cys Glu Lys Pro
       290                 295                 300
   Leu Leu Glu Lys Ser His Cys Ile Ala Glu Val Glu Asn Asp Glu Met
   305                 310                 315                 320
   Pro Ala Asp Leu Pro Ser Leu Ala Ala Asp Phe Val Glu Ser Lys Asp
                   325                 330                 335
   Val Cys Lys Asn Tyr Ala Glu Ala Lys Asp Val Phe Leu Gly Met Phe
               340                 345                 350
   Leu Tyr Glu Tyr Ala Arg Arg His Pro Asp Tyr Ser Val Val Leu Leu
           355                 360                 365
   Leu Arg Leu Ala Lys Thr Tyr Glu Thr Thr Leu Glu Lys Cys Cys Ala
       370                 375                 380
   Ala Ala Asp Pro His Glu Cys Tyr Ala Lys Val Phe Asp Glu Phe Lys
   385                 390                 395                 400
   Pro Leu Val Glu Glu Pro Gln Asn Leu Ile Lys Gln Asn Cys Glu Leu
                   405                 410                 415
   Phe Glu Gln Leu Gly Glu Tyr Lys Phe Gln Asn Ala Leu Leu Val Arg
               420                 425                 430
   Tyr Thr Lys Lys Val Pro Gln Val Ser Thr Pro Thr Leu Val Glu Val
           435                 440                 445
   Ser Arg Asn Leu Gly Lys Val Gly Ser Lys Cys Cys Lys His Pro Glu
       450                 455                 460
   Ala Lys Arg Met Pro Cys Ala Glu Asp Tyr Leu Ser Val Val Leu Asn
   465                 470                 475                 480
   Gln Leu Cys Val Leu His Glu Lys Thr Pro Val Ser Asp Arg Val Thr
                   485                 490                 495
   Lys Cys Cys Thr Glu Ser Leu Val Asn Arg Arg Pro Cys Phe Ser Ala
               500                 505                 510
   Leu Glu Val Asp Glu Thr Tyr Val Pro Lys Glu Phe Asn Ala Glu Thr
           515                 520                 525
   Phe Thr Phe His Ala Asp Ile Cys Thr Leu Ser Glu Lys Glu Arg Gln
       530                 535                 540
   Ile Lys Lys Gln Thr Ala Leu Val Glu Leu Val Lys His Lys Pro Lys
   545                 550                 555                 560
   Ala Thr Lys Glu Gln Leu Lys Ala Val Met Asp Asp Phe Ala Ala Phe
                   565                 570                 575
   Val Glu Lys Cys Cys Lys Ala Asp Asp Lys Glu Thr Cys Phe Ala Glu
               580                 585                 590
   Glu Gly Lys Lys Leu Val Ala Ala Ser Gln Ala Ala Leu Gly Leu Gly
           595                 600                 605
   Gly Gly Gly Ser Gly Leu Glu Pro Lys Ser Cys Asp Lys Thr His Thr
       610                 615                 620
   Cys Pro Pro Cys Glu Phe Gly Gly Gly Gly Ser Ile Glu Gly Pro Thr
   625                 630                 635                 640
   Leu Arg Gln Trp Leu Ala Ala Arg Ala Gly Gly Gly Ser Gly Ile Glu
                   645                 650                 655
   Gly Pro Thr Leu Arg Gln Trp Leu Ala Ala Arg Ala
               660                 665
<210> 37
   <211> 1995
   <212> DNA
   <213> gene sequence of ss-HSA-L6-H-L7-TMP-L2-TMP
<400> 37
   atggagacag acacactcct gctatgggta ctgctgctct gggttccagg ttccactggt      60
   gacggtgatg cacacaagag tgaggttgct catcggttta aagatttggg agaagaaaat     120
   ttcaaagcct tggtgttgat tgcctttgct cagtatcttc agcagtgtcc atttgaagat     180
   catgtaaaat tagtgaatga agtaactgaa tttgcaaaaa catgtgttgc tgatgagtca     240
   gctgaaaatt gtgacaaatc acttcatacc ctttttggag acaaattatg cacagttgca     300
   actcttcgtg aaacctatgg tgaaatggct gactgttgtg caaaacaaga acctgagaga     360
   aatgaatgct tcttgcaaca caaagatgac aacccaaacc tcccccgatt ggtgagacca     420
   gaggttgatg tgatgtgcac tgcttttcat gacaatgaag agacattttt gaaaaaatac     480
   ttatatgaaa ttgccagaag acatccttac ttttatgccc cggaactcct tttctttgct     540
   aaaaggtata aagctgcttt tacagaatgt tgccaagctg ctgataaagc tgcctgcctg     600
   ttgccaaagc tcgatgaact tcgggatgaa gggaaggctt cgtctgccaa acagagactc     660
   aagtgtgcca gtctccaaaa atttggagaa agagctttca aagcatgggc agtagctcgc     720
   ctgagccaga gatttcccaa agctgagttt gcagaagttt ccaagttagt gacagatctt     780
   accaaagtcc acacggaatg ctgccatgga gatctgcttg aatgtgctga tgacagggcg     840
   gaccttgcca agtatatctg tgaaaatcaa gattcgatct ccagtaaact gaaggaatgc     900
   tgtgaaaaac ctctgttgga aaaatcccac tgcattgccg aagtggaaaa tgatgagatg 960
   cctgctgact tgccttcatt agctgctgat tttgttgaaa gtaaggatgt ttgcaaaaac 1020
   tatgctgagg caaaggatgt cttcctgggc atgtttttgt atgaatatgc aagaaggcat 1080
   cctgattact ctgtcgtgct gctgctgaga cttgccaaga catatgaaac cactctagag 1140
   aagtgctgtg ccgctgcaga tcctcatgaa tgctatgcca aagtgttcga tgaatttaaa 1200
   cctcttgtgg aagagcctca gaatttaatc aaacaaaatt gtgagctttt tgagcagctt 1260
   ggagagtaca aattccagaa tgcgctatta gttcgttaca ccaagaaagt accccaagtg 1320
   tcaactccaa ctcttgtaga ggtctcaaga aacctaggaa aagtgggcag caaatgttgt 1380
   aaacatcctg aagcaaaaag aatgccctgt gcagaagact atctatccgt ggtcctgaac 1440
   cagttatgtg tgttgcatga gaaaacgcca gtaagtgaca gagtcaccaa atgctgcaca 1500
   gaatccttgg tgaacaggcg accatgcttt tcagctctgg aagtcgatga aacatacgtt 1560
   cccaaagagt ttaatgctga aacgttcacc ttccatgcag atatatgcac actttctgag 1620
   aaggagagac aaatcaagaa acaaactgca cttgttgagc ttgtgaaaca caagcccaag 1680
   gcaacaaaag agcaactgaa agctgttatg gatgatttcg cagcttttgt agagaagtgc 1740
   tgcaaggctg acgataagga gacctgcttt gccgaggagg gtaaaaaact tgttgctgca 1800
   agtcaagctg ccttaggctt aggcggcggc ggttccggac tggagcccaa gagctgcgac 1860
   aagacccaca cctgccctcc ctgcgaattc ggtggtggcg gcagcatcga aggccccaca 1920
   ctgcggcagt ggctggctgc tagggcctcc ggaatcgagg gacccaccct gaggcagtgg 1980
   ctggccgcca gagcc 1995
<210> 38
   <211> 665
   <212> PRT
   <213> amino acid sequence of ss-HSA-L6-H-L7-TMP-L2-TMP
<400> 38
   Met Glu Thr Asp Thr Leu Leu Leu Trp Val Leu Leu Leu Trp Val Pro
   1 5 10 15
   Gly Ser Thr Gly Asp Gly Asp Ala His Lys Ser Glu Val Ala His Arg
   20 25 30
   Phe Lys Asp Leu Gly Glu Glu Asn Phe Lys Ala Leu Val Leu Ile Ala
   35 40 45
   Phe Ala Gln Tyr Leu Gln Gln Cys Pro Phe Glu Asp His Val Lys Leu
   50 55 60
   Val Asn Glu Val Thr Glu Phe Ala Lys Thr Cys Val Ala Asp Glu Ser
   65                  70                  75                  80
   Ala Glu Asn Cys Asp Lys Ser Leu His Thr Leu Phe Gly Asp Lys Leu
                   85                  90                  95
   Cys Thr Val Ala Thr Leu Arg Glu Thr Tyr Gly Glu Met Ala Asp Cys
               100                 105                 110
   Cys Ala Lys Gln Glu Pro Glu Arg Asn Glu Cys Phe Leu Gln His Lys
           115                 120                 125
   Asp Asp Asn Pro Asn Leu Pro Arg Leu Val Arg Pro Glu Val Asp Val
       130                 135                 140
   Met Cys Thr Ala Phe His Asp Asn Glu Glu Thr Phe Leu Lys Lys Tyr
   145                 150                 155                 160
   Leu Tyr Glu Ile Ala Arg Arg His Pro Tyr Phe Tyr Ala Pro Glu Leu
                   165                 170                 175
   Leu Phe Phe Ala Lys Arg Tyr Lys Ala Ala Phe Thr Glu Cys Cys Gln
               180                 185                 190
   Ala Ala Asp Lys Ala Ala Cys Leu Leu Pro Lys Leu Asp Glu Leu Arg
           195                 200                 205
   Asp Glu Gly Lys Ala Ser Ser Ala Lys Gln Arg Leu Lys Cys Ala Ser
       210                 215                 220
   Leu Gln Lys Phe Gly Glu Arg Ala Phe Lys Ala Trp Ala Val Ala Arg
   225                 230                 235                 240
   Leu Ser Gln Arg Phe Pro Lys Ala Glu Phe Ala Glu Val Ser Lys Leu
                   245                 250                 255
   Val Thr Asp Leu Thr Lys Val His Thr Glu Cys Cys His Gly Asp Leu
               260                 265                 270
   Leu Glu Cys Ala Asp Asp Arg Ala Asp Leu Ala Lys Tyr Ile Cys Glu
           275                 280                 285
   Asn Gln Asp Ser Ile Ser Ser Lys Leu Lys Glu Cys Cys Glu Lys Pro
       290                 295                 300
   Leu Leu Glu Lys Ser His Cys Ile Ala Glu Val Glu Asn Asp Glu Met
   305                 310                 315                 320
   Pro Ala Asp Leu Pro Ser Leu Ala Ala Asp Phe Val Glu Ser Lys Asp
                   325                 330                 335
   Val Cys Lys Asn Tyr Ala Glu Ala Lys Asp Val Phe Leu Gly Met Phe
               340                 345                 350
   Leu Tyr Glu Tyr Ala Arg Arg His Pro Asp Tyr Ser Val Val Leu Leu
           355                 360                 365
   Leu Arg Leu Ala Lys Thr Tyr Glu Thr Thr Leu Glu Lys Cys Cys Ala
       370                 375                 380
   Ala Ala Asp Pro His Glu Cys Tyr Ala Lys Val Phe Asp Glu Phe Lys
   385                 390                 395                 400
   Pro Leu Val Glu Glu Pro Gln Asn Leu Ile Lys Gln Asn Cys Glu Leu
                   405                 410                 415
   Phe Glu Gln Leu Gly Glu Tyr Lys Phe Gln Asn Ala Leu Leu Val Arg
               420                 425                 430
   Tyr Thr Lys Lys Val Pro Gln Val Ser Thr Pro Thr Leu Val Glu Val
           435                 440                 445
   Ser Arg Asn Leu Gly Lys Val Gly Ser Lys Cys Cys Lys His Pro Glu
       450                 455                 460
   Ala Lys Arg Met Pro Cys Ala Glu Asp Tyr Leu Ser Val Val Leu Asn
   465                 470                 475                 480
   Gln Leu Cys Val Leu His Glu Lys Thr Pro Val Ser Asp Arg Val Thr
                   485                 490                 495
   Lys Cys Cys Thr Glu Ser Leu Val Asn Arg Arg Pro Cys Phe Ser Ala
               500                 505                 510
   Leu Glu Val Asp Glu Thr Tyr Val Pro Lys Glu Phe Asn Ala Glu Thr
           515                 520                 525
   Phe Thr Phe His Ala Asp Ile Cys Thr Leu Ser Glu Lys Glu Arg Gln
       530                 535                 540
   Ile Lys Lys Gln Thr Ala Leu Val Glu Leu Val Lys His Lys Pro Lys
   545                 550                 555                 560
   Ala Thr Lys Glu Gln Leu Lys Ala Val Met Asp Asp Phe Ala Ala Phe
                   565                 570                 575
   Val Glu Lys Cys Cys Lys Ala Asp Asp Lys Glu Thr Cys Phe Ala Glu
               580                 585                 590
   Glu Gly Lys Lys Leu Val Ala Ala Ser Gln Ala Ala Leu Gly Leu Gly
           595                 600                 605
   Gly Gly Gly Ser Gly Leu Glu Pro Lys Ser Cys Asp Lys Thr His Thr
       610                 615                 620
   Cys Pro Pro Cys Glu Phe Gly Gly Gly Gly Ser Ile Glu Gly Pro Thr
   625                 630                 635                 640
   Leu Arg Gln Trp Leu Ala Ala Arg Ala Ser Gly Ile Glu Gly Pro Thr
                   645                 650                 655
   Leu Arg Gln Trp Leu Ala Ala Arg Ala
               660                 665
<210> 39
   <211> 2004
   <212> DNA
   <213> gene sequence of ss-HSA-L6-H-L7-TMP-L3-TMP
<400> 39
   atggagacag acacactcct gctatgggta ctgctgctct gggttccagg ttccactggt      60
   gacggtgatg cacacaagag tgaggttgct catcggttta aagatttggg agaagaaaat     120
   ttcaaagcct tggtgttgat tgcctttgct cagtatcttc agcagtgtcc atttgaagat     180
   catgtaaaat tagtgaatga agtaactgaa tttgcaaaaa catgtgttgc tgatgagtca     240
   gctgaaaatt gtgacaaatc acttcatacc ctttttggag acaaattatg cacagttgca     300
   actcttcgtg aaacctatgg tgaaatggct gactgttgtg caaaacaaga acctgagaga     360
   aatgaatgct tcttgcaaca caaagatgac aacccaaacc tcccccgatt ggtgagacca     420
   gaggttgatg tgatgtgcac tgcttttcat gacaatgaag agacattttt gaaaaaatac     480
   ttatatgaaa ttgccagaag acatccttac ttttatgccc cggaactcct tttctttgct     540
   aaaaggtata aagctgcttt tacagaatgt tgccaagctg ctgataaagc tgcctgcctg     600
   ttgccaaagc tcgatgaact tcgggatgaa gggaaggctt cgtctgccaa acagagactc     660
   aagtgtgcca gtctccaaaa atttggagaa agagctttca aagcatgggc agtagctcgc     720
   ctgagccaga gatttcccaa agctgagttt gcagaagttt ccaagttagt gacagatctt     780
   accaaagtcc acacggaatg ctgccatgga gatctgcttg aatgtgctga tgacagggcg     840
   gaccttgcca agtatatctg tgaaaatcaa gattcgatct ccagtaaact gaaggaatgc     900
   tgtgaaaaac ctctgttgga aaaatcccac tgcattgccg aagtggaaaa tgatgagatg     960
   cctgctgact tgccttcatt agctgctgat tttgttgaaa gtaaggatgt ttgcaaaaac     1020
   tatgctgagg caaaggatgt cttcctgggc atgtttttgt atgaatatgc aagaaggcat     1080
   cctgattact ctgtcgtgct gctgctgaga cttgccaaga catatgaaac cactctagag     1140
   aagtgctgtg ccgctgcaga tcctcatgaa tgctatgcca aagtgttcga tgaatttaaa     1200
   cctcttgtgg aagagcctca gaatttaatc aaacaaaatt gtgagctttt tgagcagctt     1260
   ggagagtaca aattccagaa tgcgctatta gttcgttaca ccaagaaagt accccaagtg     1320
   tcaactccaa ctcttgtaga ggtctcaaga aacctaggaa aagtgggcag caaatgttgt     1380
   aaacatcctg aagcaaaaag aatgccctgt gcagaagact atctatccgt ggtcctgaac     1440
   cagttatgtg tgttgcatga gaaaacgcca gtaagtgaca gagtcaccaa atgctgcaca     1500
   gaatccttgg tgaacaggcg accatgcttt tcagctctgg aagtcgatga aacatacgtt     1560
   cccaaagagt ttaatgctga aacgttcacc ttccatgcag atatatgcac actttctgag     1620
   aaggagagac aaatcaagaa acaaactgca cttgttgagc ttgtgaaaca caagcccaag     1680
   gcaacaaaag agcaactgaa agctgttatg gatgatttcg cagcttttgt agagaagtgc     1740
   tgcaaggctg acgataagga gacctgcttt gccgaggagg gtaaaaaact tgttgctgca     1800
   agtcaagctg ccttaggctt aggcggcggc ggttccggac tggagcccaa gagctgcgac     1860
   aagacccaca cctgccctcc ctgcgaattc ggtggtggcg gcagcatcga aggccccaca     1920
   ctgcggcagt ggctggctgc tagggccggt ggcccctccg gaatcgaggg acccaccctg     1980
   aggcagtggc tggccgccag agcc                                            2004
<210> 40
   <211> 668
   <212> PRT
   <213> amino acid sequence of ss-HSA-L6-H-L7-TMP-L3-TMP
<400> 40
   Met Glu Thr Asp Thr Leu Leu Leu Trp Val Leu Leu Leu Trp Val Pro
   1               5                   10                  15
   Gly Ser Thr Gly Asp Gly Asp Ala His Lys Ser Glu Val Ala His Arg
               20                  25                  30
   Phe Lys Asp Leu Gly Glu Glu Asn Phe Lys Ala Leu Val Leu Ile Ala
           35                  40                  45
   Phe Ala Gln Tyr Leu Gln Gln Cys Pro Phe Glu Asp His Val Lys Leu
       50                  55                  60
   Val Asn Glu Val Thr Glu Phe Ala Lys Thr Cys Val Ala Asp Glu Ser
   65                  70                  75                  80
   Ala Glu Asn Cys Asp Lys Ser Leu His Thr Leu Phe Gly Asp Lys Leu
                   85                  90                  95
   Cys Thr Val Ala Thr Leu Arg Glu Thr Tyr Gly Glu Met Ala Asp Cys
               100                 105                 110
   Cys Ala Lys Gln Glu Pro Glu Arg Asn Glu Cys Phe Leu Gln His Lys
           115                 120                 125
   Asp Asp Asn Pro Asn Leu Pro Arg Leu Val Arg Pro Glu Val Asp Val
       130                 135                 140
   Met Cys Thr Ala Phe His Asp Asn Glu Glu Thr Phe Leu Lys Lys Tyr
   145                 150                 155                 160
   Leu Tyr Glu Ile Ala Arg Arg His Pro Tyr Phe Tyr Ala Pro Glu Leu
                   165                 170                 175
   Leu Phe Phe Ala Lys Arg Tyr Lys Ala Ala Phe Thr Glu Cys Cys Gln
               180                 185                 190
   Ala Ala Asp Lys Ala Ala Cys Leu Leu Pro Lys Leu Asp Glu Leu Arg
           195                 200                 205
   Asp Glu Gly Lys Ala Ser Ser Ala Lys Gln Arg Leu Lys Cys Ala Ser
       210                 215                 220
   Leu Gln Lys Phe Gly Glu Arg Ala Phe Lys Ala Trp Ala Val Ala Arg
   225                 230                 235                 240
   Leu Ser Gln Arg Phe Pro Lys Ala Glu Phe Ala Glu Val Ser Lys Leu
                   245                 250                 255
   Val Thr Asp Leu Thr Lys Val His Thr Glu Cys Cys His Gly Asp Leu
               260                 265                 270
   Leu Glu Cys Ala Asp Asp Arg Ala Asp Leu Ala Lys Tyr Ile Cys Glu
           275                 280                 285
   Asn Gln Asp Ser Ile Ser Ser Lys Leu Lys Glu Cys Cys Glu Lys Pro
       290                 295                 300
   Leu Leu Glu Lys Ser His Cys Ile Ala Glu Val Glu Asn Asp Glu Met
   305                 310                 315                 320
   Pro Ala Asp Leu Pro Ser Leu Ala Ala Asp Phe Val Glu Ser Lys Asp
                   325                 330                 335
   Val Cys Lys Asn Tyr Ala Glu Ala Lys Asp Val Phe Leu Gly Met Phe
               340                 345                 350
   Leu Tyr Glu Tyr Ala Arg Arg His Pro Asp Tyr Ser Val Val Leu Leu
           355                 360                 365
   Leu Arg Leu Ala Lys Thr Tyr Glu Thr Thr Leu Glu Lys Cys Cys Ala
       370                 375                 380
   Ala Ala Asp Pro His Glu Cys Tyr Ala Lys Val Phe Asp Glu Phe Lys
   385                 390                 395                 400
   Pro Leu Val Glu Glu Pro Gln Asn Leu Ile Lys Gln Asn Cys Glu Leu
                   405                 410                 415
   Phe Glu Gln Leu Gly Glu Tyr Lys Phe Gln Asn Ala Leu Leu Val Arg
               420                 425                 430
   Tyr Thr Lys Lys Val Pro Gln Val Ser Thr Pro Thr Leu Val Glu Val
           435                 440                 445
   Ser Arg Asn Leu Gly Lys Val Gly Ser Lys Cys Cys Lys His Pro Glu
       450                 455                 460
   Ala Lys Arg Met Pro Cys Ala Glu Asp Tyr Leu Ser Val Val Leu Asn
   465                 470                 475                 480
   Gln Leu Cys Val Leu His Glu Lys Thr Pro Val Ser Asp Arg Val Thr
                   485                 490                 495
   Lys Cys Cys Thr Glu Ser Leu Val Asn Arg Arg Pro Cys Phe Ser Ala
               500                 505                 510
   Leu Glu Val Asp Glu Thr Tyr Val Pro Lys Glu Phe Asn Ala Glu Thr
           515                 520                 525
   Phe Thr Phe His Ala Asp Ile Cys Thr Leu Ser Glu Lys Glu Arg Gln
       530                 535                 540
   Ile Lys Lys Gln Thr Ala Leu Val Glu Leu Val Lys His Lys Pro Lys
   545                 550                 555                 560
   Ala Thr Lys Glu Gln Leu Lys Ala Val Met Asp Asp Phe Ala Ala Phe
                   565                 570                 575
   Val Glu Lys Cys Cys Lys Ala Asp Asp Lys Glu Thr Cys Phe Ala Glu
               580                 585                 590
   Glu Gly Lys Lys Leu Val Ala Ala Ser Gln Ala Ala Leu Gly Leu Gly
           595                 600                 605
   Gly Gly Gly Ser Gly Leu Glu Pro Lys Ser Cys Asp Lys Thr His Thr
       610                 615                 620
   Cys Pro Pro Cys Glu Phe Gly Gly Gly Gly Ser Ile Glu Gly Pro Thr
   625                 630                 635                 640
   Leu Arg Gln Trp Leu Ala Ala Arg Ala Gly Gly Pro Ser Gly Ile Glu
                   645                 650                 655
   Gly Pro Thr Leu Arg Gln Trp Leu Ala Ala Arg Ala
               660                 665
<210> 41
   <211> 2019
   <212> DNA
   <213> gene sequence of ss-HSA-L6-H-L7-TMP-L4-TMP
<400> 41
   atggagacag acacactcct gctatgggta ctgctgctct gggttccagg ttccactggt      60
   gacggtgatg cacacaagag tgaggttgct catcggttta aagatttggg agaagaaaat     120
   ttcaaagcct tggtgttgat tgcctttgct cagtatcttc agcagtgtcc atttgaagat     180
   catgtaaaat tagtgaatga agtaactgaa tttgcaaaaa catgtgttgc tgatgagtca     240
   gctgaaaatt gtgacaaatc acttcatacc ctttttggag acaaattatg cacagttgca     300
   actcttcgtg aaacctatgg tgaaatggct gactgttgtg caaaacaaga acctgagaga     360
   aatgaatgct tcttgcaaca caaagatgac aacccaaacc tcccccgatt ggtgagacca     420
   gaggttgatg tgatgtgcac tgcttttcat gacaatgaag agacattttt gaaaaaatac     480
   ttatatgaaa ttgccagaag acatccttac ttttatgccc cggaactcct tttctttgct     540
   aaaaggtata aagctgcttt tacagaatgt tgccaagctg ctgataaagc tgcctgcctg     600
   ttgccaaagc tcgatgaact tcgggatgaa gggaaggctt cgtctgccaa acagagactc     660
   aagtgtgcca gtctccaaaa atttggagaa agagctttca aagcatgggc agtagctcgc     720
   ctgagccaga gatttcccaa agctgagttt gcagaagttt ccaagttagt gacagatctt     780
   accaaagtcc acacggaatg ctgccatgga gatctgcttg aatgtgctga tgacagggcg     840
   gaccttgcca agtatatctg tgaaaatcaa gattcgatct ccagtaaact gaaggaatgc     900
   tgtgaaaaac ctctgttgga aaaatcccac tgcattgccg aagtggaaaa tgatgagatg     960
   cctgctgact tgccttcatt agctgctgat tttgttgaaa gtaaggatgt ttgcaaaaac    1020
   tatgctgagg caaaggatgt cttcctgggc atgtttttgt atgaatatgc aagaaggcat     1080
   cctgattact ctgtcgtgct gctgctgaga cttgccaaga catatgaaac cactctagag     1140
   aagtgctgtg ccgctgcaga tcctcatgaa tgctatgcca aagtgttcga tgaatttaaa     1200
   cctcttgtgg aagagcctca gaatttaatc aaacaaaatt gtgagctttt tgagcagctt     1260
   ggagagtaca aattccagaa tgcgctatta gttcgttaca ccaagaaagt accccaagtg     1320
   tcaactccaa ctcttgtaga ggtctcaaga aacctaggaa aagtgggcag caaatgttgt     1380
   aaacatcctg aagcaaaaag aatgccctgt gcagaagact atctatccgt ggtcctgaac     1440
   cagttatgtg tgttgcatga gaaaacgcca gtaagtgaca gagtcaccaa atgctgcaca     1500
   gaatccttgg tgaacaggcg accatgcttt tcagctctgg aagtcgatga aacatacgtt     1560
   cccaaagagt ttaatgctga aacgttcacc ttccatgcag atatatgcac actttctgag     1620
   aaggagagac aaatcaagaa acaaactgca cttgttgagc ttgtgaaaca caagcccaag     1680
   gcaacaaaag agcaactgaa agctgttatg gatgatttcg cagcttttgt agagaagtgc     1740
   tgcaaggctg acgataagga gacctgcttt gccgaggagg gtaaaaaact tgttgctgca     1800
   agtcaagctg ccttaggctt aggcggcggc ggttccggac tggagcccaa gagctgcgac     1860
   aagacccaca cctgccctcc ctgcgaattc ggtggtggcg gcagcatcga aggccccaca     1920
   ctgcggcagt ggctggctgc tagggccggc ggtggcggca gcggtggcgg ctccggaatc     1980
   gagggaccca ccctgaggca gtggctggcc gccagagcc                            2019
<210> 42
   <211> 673
   <212> PRT
   <213> amino acid sequence of ss-HSA-L6-H-L7-TMP-L4-TMP
<400> 42
   Met Glu Thr Asp Thr Leu Leu Leu Trp Val Leu Leu Leu Trp Val Pro
   1               5                   10                  15
   Gly Ser Thr Gly Asp Gly Asp Ala His Lys Ser Glu Val Ala His Arg
               20                  25                  30
   Phe Lys Asp Leu Gly Glu Glu Asn Phe Lys Ala Leu Val Leu Ile Ala
           35                  40                  45
   Phe Ala Gln Tyr Leu Gln Gln Cys Pro Phe Glu Asp His Val Lys Leu
       50                  55                  60
   Val Asn Glu Val Thr Glu Phe Ala Lys Thr Cys Val Ala Asp Glu Ser
   65                  70                  75                  80
   Ala Glu Asn Cys Asp Lys Ser Leu His Thr Leu Phe Gly Asp Lys Leu
                   85                  90                  95
   Cys Thr Val Ala Thr Leu Arg Glu Thr Tyr Gly Glu Met Ala Asp Cys
               100                 105                 110
   Cys Ala Lys Gln Glu Pro Glu Arg Asn Glu Cys Phe Leu Gln His Lys
           115                 120                 125
   Asp Asp Asn Pro Asn Leu Pro Arg Leu Val Arg Pro Glu Val Asp Val
       130                 135                 140
   Met Cys Thr Ala Phe His Asp Asn Glu Glu Thr Phe Leu Lys Lys Tyr
   145                 150                 155                 160
   Leu Tyr Glu Ile Ala Arg Arg His Pro Tyr Phe Tyr Ala Pro Glu Leu
                   165                 170                 175
   Leu Phe Phe Ala Lys Arg Tyr Lys Ala Ala Phe Thr Glu Cys Cys Gln
               180                 185                 190
   Ala Ala Asp Lys Ala Ala Cys Leu Leu Pro Lys Leu Asp Glu Leu Arg
           195                 200                 205
   Asp Glu Gly Lys Ala Ser Ser Ala Lys Gln Arg Leu Lys Cys Ala Ser
       210                 215                 220
   Leu Gln Lys Phe Gly Glu Arg Ala Phe Lys Ala Trp Ala Val Ala Arg
   225                 230                 235                 240
   Leu Ser Gln Arg Phe Pro Lys Ala Glu Phe Ala Glu Val Ser Lys Leu
                   245                 250                 255
   Val Thr Asp Leu Thr Lys Val His Thr Glu Cys Cys His Gly Asp Leu
               260                 265                 270
   Leu Glu Cys Ala Asp Asp Arg Ala Asp Leu Ala Lys Tyr Ile Cys Glu
           275                 280                 285
   Asn Gln Asp Ser Ile Ser Ser Lys Leu Lys Glu Cys Cys Glu Lys Pro
       290                 295                 300
   Leu Leu Glu Lys Ser His Cys Ile Ala Glu Val Glu Asn Asp Glu Met
   305                 310                 315                 320
   Pro Ala Asp Leu Pro Ser Leu Ala Ala Asp Phe Val Glu Ser Lys Asp
                   325                 330                 335
   Val Cys Lys Asn Tyr Ala Glu Ala Lys Asp Val Phe Leu Gly Met Phe
               340                 345                 350
   Leu Tyr Glu Tyr Ala Arg Arg His Pro Asp Tyr Ser Val Val Leu Leu
           355                 360                 365
   Leu Arg Leu Ala Lys Thr Tyr Glu Thr Thr Leu Glu Lys Cys Cys Ala
       370                 375                 380
   Ala Ala Asp Pro His Glu Cys Tyr Ala Lys Val Phe Asp Glu Phe Lys
   385                 390                 395                 400
   Pro Leu Val Glu Glu Pro Gln Asn Leu Ile Lys Gln Asn Cys Glu Leu
                   405                 410                 415
   Phe Glu Gln Leu Gly Glu Tyr Lys Phe Gln Asn Ala Leu Leu Val Arg
               420                 425                 430
   Tyr Thr Lys Lys Val Pro Gln Val Ser Thr Pro Thr Leu Val Glu Val
           435                 440                 445
   Ser Arg Asn Leu Gly Lys Val Gly Ser Lys Cys Cys Lys His Pro Glu
       450                 455                 460
   Ala Lys Arg Met Pro Cys Ala Glu Asp Tyr Leu Ser Val Val Leu Asn
   465                 470                 475                 480
   Gln Leu Cys Val Leu His Glu Lys Thr Pro Val Ser Asp Arg Val Thr
                   485                 490                 495
   Lys Cys Cys Thr Glu Ser Leu Val Asn Arg Arg Pro Cys Phe Ser Ala
               500                 505                 510
   Leu Glu Val Asp Glu Thr Tyr Val Pro Lys Glu Phe Asn Ala Glu Thr
           515                 520                 525
   Phe Thr Phe His Ala Asp Ile Cys Thr Leu Ser Glu Lys Glu Arg Gln
       530                 535                 540
   Ile Lys Lys Gln Thr Ala Leu Val Glu Leu Val Lys His Lys Pro Lys
   545                 550                 555                 560
   Ala Thr Lys Glu Gln Leu Lys Ala Val Met Asp Asp Phe Ala Ala Phe
                   565                 570                 575
   Val Glu Lys Cys Cys Lys Ala Asp Asp Lys Glu Thr Cys Phe Ala Glu
               580                 585                 590
   Glu Gly Lys Lys Leu Val Ala Ala Ser Gln Ala Ala Leu Gly Leu Gly
   595 600 605
   Gly Gly Gly Ser Gly Leu Glu Pro Lys Ser Cys Asp Lys Thr His Thr
   610 615 620
   Cys Pro Pro Cys Glu Phe Gly Gly Gly Gly Ser Ile Glu Gly Pro Thr
   625 630 635 640
   Leu Arg Gln Trp Leu Ala Ala Arg Ala Gly Gly Gly Gly Ser Gly Gly
   645 650 655
   Gly Ser Gly Ile Glu Gly Pro Thr Leu Arg Gln Trp Leu Ala Ala Arg
   660 665 670
   Ala
<210> 43
   <211> 312
   <212> DNA
   <213> gene sequence of a-factor-TMP-L1
<400> 43
   atgagatttc cttcaatttt tactgctgtt ttattcgcag catcctccgc attagctgct 60
   ccagtcaaca ctacaacaga agatgaaacg gcacaaattc cggctgaagc tgtcatcggt 120
   tactcagatt tagaagggga tttcgatgtt gctgttttgc cattttccaa cagcacaaat 180
   aacgggttat tgtttataaa tactactatt gccagcattg ctgctaaaga agaaggggta 240
   tctctcgaga aaaggatcga aggccccaca ctgcggcagt ggctggctgc tagggccggt 300
   ggcggctccg ga 312
<210> 44
   <211> 104
   <212> PRT
   <213> amino acid sequence of a-factor-TMP-L1
<400> 44
   Met Arg Phe Pro Ser Ile Phe Thr Ala Val Leu Phe Ala Ala Ser Ser
   1 5 10 15
   Ala Leu Ala Ala Pro Val Asn Thr Thr Thr Glu Asp Glu Thr Ala Gln
   20 25 30
   Ile Pro Ala Glu Ala Val Ile Gly Tyr Ser Asp Leu Glu Gly Asp Phe
   35 40 45
   Asp Val Ala Val Leu Pro Phe Ser Asn Ser Thr Asn Asn Gly Leu Leu
       50                  55                  60
   Phe Ile Asn Thr Thr Ile Ala Ser Ile Ala Ala Lys Glu Glu Gly Val
   65                  70                  75                  80
   Ser Leu Glu Lys Arg Ile Glu Gly Pro Thr Leu Arg Gln Trp Leu Ala
                   85                  90                  95
   Ala Arg Ala Gly Gly Gly Ser Gly
               100
<210> 45
   <211> 703
   <212> DNA
   <213> gene sequence of HSA(post4)-His
<400> 45
   gaagtgctgt gccgctgcag atcctcatga atgctatgcc aaagtgttcg atgaatttaa       60
   acctcttgtg gaagagcctc agaatttaat caaacaaaat tgtgagcttt ttgagcagct      120
   tggagagtac aaattccaga atgcgctatt agttcgttac accaagaaag taccccaagt      180
   gtcaactcca actcttgtag aggtctcaag aaacctagga aaagtgggca gcaaatgttg      240
   taaacatcct gaagcaaaaa gaatgccctg tgcagaagac tatctatccg tggtcctgaa      300
   ccagttatgt gtgttgcatg agaaaacgcc agtaagtgac agagtcacca aatgctgcac      360
   agaatccttg gtgaacaggc gaccatgctt ttcagctctg gaagtcgatg aaacatacgt      420
   tcccaaagag tttaatgctg aaacgttcac cttccatgca gatatatgca cactttctga      480
   gaaggagaga caaatcaaga aacaaactgc acttgttgag cttgtgaaac acaagcccaa      540
   ggcaacaaaa gagcaactga aagctgttat ggatgatttc gcagcttttg tagagaagtg      600
   ctgcaaggct gacgataagg agacctgctt tgccgaggag ggtaaaaaac ttgttgctgc      660
   aagtcaagct gccttaggct tacaccacca ccaccaccac tga                        703
<210> 46
   <211> 233
   <212> PRT
   <213> amino acid sequence of HSA(post4)-His
<400> 46
   Lys Cys Cys Ala Ala Ala Asp Pro His Glu Cys Tyr Ala Lys Val Phe
   1               5                   10                  15
   Asp Glu Phe Lys Pro Leu Val Glu Glu Pro Gln Asn Leu Ile Lys Gln
               20                  25                  30
   Asn Cys Glu Leu Phe Glu Gln Leu Gly Glu Tyr Lys Phe Gln Asn Ala
           35                  40                  45
   Leu Leu Val Arg Tyr Thr Lys Lys Val Pro Gln Val Ser Thr Pro Thr
       50                  55                  60
   Leu Val Glu Val Ser Arg Asn Leu Gly Lys Val Gly Ser Lys Cys Cys
   65                  70                  75                  80
   Lys His Pro Glu Ala Lys Arg Met Pro Cys Ala Glu Asp Tyr Leu Ser
                   85                  90                  95
   Val Val Leu Asn Gln Leu Cys Val Leu His Glu Lys Thr Pro Val Ser
               100                 105                 110
   Asp Arg Val Thr Lys Cys Cys Thr Glu Ser Leu Val Asn Arg Arg Pro
           115                 120                 125
   Cys Phe Ser Ala Leu Glu Val Asp Glu Thr Tyr Val Pro Lys Glu Phe
       130                 135                 140
   Asn Ala Glu Thr Phe Thr Phe His Ala Asp Ile Cys Thr Leu Ser Glu
   145                 150                 155                 160
   Lys Glu Arg Gln Ile Lys Lys Gln Thr Ala Leu Val Glu Leu Val Lys
                   165                 170                 175
   His Lys Pro Lys Ala Thr Lys Glu Gln Leu Lys Ala Val Met Asp Asp
               180                 185                 190
   Phe Ala Ala Phe Val Glu Lys Cys Cys Lys Ala Asp Asp Lys Glu Thr
           195                 200                 205
   Cys Phe Ala Glu Glu Gly Lys Lys Leu Val Ala Ala Ser Gln Ala Ala
       210                 215                 220
   Leu Gly Leu His His His His His His
   225                 230
<210> 47
   <211> 1292
   <212> DNA
   <213> gene sequence of TMP-L5-H-fip-HSA(pre4)
<400> 47
   atcgagggac ccaccctgag gcagtggctg gccgccagag ccggcggcgg cggaagcaga      60
   tctgagccca agagcagcga caaaactcac acatgcccac cgtgcgtgag cagggacgag     120
   ctgatggagg ccatccagaa gcaggaggag atcaacttca ggctgcagga ctacatcgac     180
   aggatcatcg tggccatcat ggagaccaac cccagcatcg atgcacacaa gagtgaggtt     240
   gctcatcggt ttaaagattt gggagaagaa aatttcaaag ccttggtgtt gattgccttt     300
   gctcagtatc ttcagcagtg tccatttgaa gatcatgtaa aattagtgaa tgaagtaact     360
   gaatttgcaa aaacatgtgt tgctgatgag tcagctgaaa attgtgacaa atcacttcat     420
   accctttttg gagacaaatt atgcacagtt gcaactcttc gtgaaaccta tggtgaaatg     480
   gctgactgtt gtgcaaaaca agaacctgag agaaatgaat gcttcttgca acacaaagat     540
   gacaacccaa acctcccccg attggtgaga ccagaggttg atgtgatgtg cactgctttt     600
   catgacaatg aagagacatt tttgaaaaaa tacttatatg aaattgccag aagacatcct     660
   tacttttatg ccccggaact ccttttcttt gctaaaaggt ataaagctgc ttttacagaa     720
   tgttgccaag ctgctgataa agctgcctgc ctgttgccaa agctcgatga acttcgggat     780
   gaagggaagg cttcgtctgc caaacagaga ctcaagtgtg ccagtctcca aaaatttgga     840
   gaaagagctt tcaaagcatg ggcagtagct cgcctgagcc agagatttcc caaagctgag     900
   tttgcagaag tttccaagtt agtgacagat cttaccaaag tccacacgga atgctgccat     960
   ggagatctgc ttgaatgtgc tgatgacagg gcggaccttg ccaagtatat ctgtgaaaat    1020
   caagattcga tctccagtaa actgaaggaa tgctgtgaaa aacctctgtt ggaaaaatcc    1080
   cactgcattg ccgaagtgga aaatgatgag atgcctgctg acttgccttc attagctgct    1140
   gattttgttg aaagtaagga tgtttgcaaa aactatgctg aggcaaagga tgtcttcctg    1200
   ggcatgtttt tgtatgaata tgcaagaagg catcctgatt actctgtcgt gctgctgctg    1260
   agacttgcca agacatatga aaccactcta ga                                  1292
<210> 48
   <211> 430
   <212> PRT
   <213> amino acid sequence of TMP-L5-H-fip-HSA(pre4)
<400> 48
   Ile Glu Gly Pro Thr Leu Arg Gln Trp Leu Ala Ala Arg Ala Gly Gly
   1               5                   10                  15
   Gly Gly Ser Arg Ser Glu Pro Lys Ser Ser Asp Lys Thr His Thr Cys
               20                  25                  30
   Pro Pro Cys Val Ser Arg Asp Glu Leu Met Glu Ala Ile Gln Lys Gln
           35                  40                  45
   Glu Glu Ile Asn Phe Arg Leu Gln Asp Tyr Ile Asp Arg Ile Ile Val
       50                  55                  60
   Ala Ile Met Glu Thr Asn Pro Ser Ile Asp Ala His Lys Ser Glu Val
   65                  70                  75                  80
   Ala His Arg Phe Lys Asp Leu Gly Glu Glu Asn Phe Lys Ala Leu Val
                   85                  90                  95
   Leu Ile Ala Phe Ala Gln Tyr Leu Gln Gln Cys Pro Phe Glu Asp His
               100                 105                 110
   Val Lys Leu Val Asn Glu Val Thr Glu Phe Ala Lys Thr Cys Val Ala
           115                 120                 125
   Asp Glu Ser Ala Glu Asn Cys Asp Lys Ser Leu His Thr Leu Phe Gly
       130                 135                 140
   Asp Lys Leu Cys Thr Val Ala Thr Leu Arg Glu Thr Tyr Gly Glu Met
   145                 150                 155                 160
   Ala Asp Cys Cys Ala Lys Gln Glu Pro Glu Arg Asn Glu Cys Phe Leu
                   165                 170                 175
   Gln His Lys Asp Asp Asn Pro Asn Leu Pro Arg Leu Val Arg Pro Glu
               180                 185                 190
   Val Asp Val Met Cys Thr Ala Phe His Asp Asn Glu Glu Thr Phe Leu
           195                 200                 205
   Lys Lys Tyr Leu Tyr Glu Ile Ala Arg Arg His Pro Tyr Phe Tyr Ala
       210                 215                 220
   Pro Glu Leu Leu Phe Phe Ala Lys Arg Tyr Lys Ala Ala Phe Thr Glu
   225                 230                 235                 240
   Cys Cys Gln Ala Ala Asp Lys Ala Ala Cys Leu Leu Pro Lys Leu Asp
                   245                 250                 255
   Glu Leu Arg Asp Glu Gly Lys Ala Ser Ser Ala Lys Gln Arg Leu Lys
               260                 265                 270
   Cys Ala Ser Leu Gln Lys Phe Gly Glu Arg Ala Phe Lys Ala Trp Ala
           275                 280                 285
   Val Ala Arg Leu Ser Gln Arg Phe Pro Lys Ala Glu Phe Ala Glu Val
       290                 295                 300
   Ser Lys Leu Val Thr Asp Leu Thr Lys Val His Thr Glu Cys Cys His
   305                 310                 315                 320
   Gly Asp Leu Leu Glu Cys Ala Asp Asp Arg Ala Asp Leu Ala Lys Tyr
                   325                 330                 335
   Ile Cys Glu Asn Gln Asp Ser Ile Ser Ser Lys Leu Lys Glu Cys Cys
               340                 345                 350
   Glu Lys Pro Leu Leu Glu Lys Ser His Cys Ile Ala Glu Val Glu Asn
           355                 360                 365
   Asp Glu Met Pro Ala Asp Leu Pro Ser Leu Ala Ala Asp Phe Val Glu
       370                 375                 380
   Ser Lys Asp Val Cys Lys Asn Tyr Ala Glu Ala Lys Asp Val Phe Leu
   385                 390                 395                 400
   Gly Met Phe Leu Tyr Glu Tyr Ala Arg Arg His Pro Asp Tyr Ser Val
                   405                 410                 415
   Val Leu Leu Leu Arg Leu Ala Lys Thr Tyr Glu Thr Thr Leu
               420                 425                 430

## Description

### Technical Field

The invention relates to the field of genetic engineering pharmacy, and particularly to a dimerized thrombopoietin mimetic peptide - thrombopoietin mimetic peptide - Human Serum Albumin (TMP-TMP-HSA) fusion protein and its preparation and application (use).

### Background Art

Generally, the half-lives of many therapeutic proteins and peptides are short (Jin et al, 2010), and frequent injections are often required to maintain adequate levels in plasma. What is more, drugs of therapeutic proteins and peptides such like are very expensive. It usually brings heavy physical, mental, and economic burden to the patients and their family. Therefore, research of protein engineering has focused to prolong the half-life of these therapeutic proteins and peptides. To exploit a long life recombinant protein and active peptide has become an important trend on the development of the second generation genetic engineering products based on their first generation counterparts (Zhao et al, 2005).

One of the general methods to prolong the half-lives of therapeutic proteins and peptides is to introduce mutations. For example, long-acting EPO (Erythropoietin, produced by Aranesp, Amgen Inc.) and long-acting insulin (produced by Lantus, Aventis Inc.) are thereby obtained (Qi and Ma, 2006). However, actually, few drugs of active peptide and protein can turn to be prolonged their half-lives in vivo by this way. In fact, most of active peptides and proteins have strengthened antigenicity after mutation, thus leading to the production of neutralizing antibody in the body due to frequent injections. The second method to prolong the half-lives of therapeutic proteins and peptides is chemical modification. In this respect, long acting drugs, such as PEG modified interferon, have been approved and appeared on the markets, but the heterogeneity of desired products modified using such macromolecular compounds has never been solved at present (Jiang and Gong, 2012).

In recent years, peptides and proteins with the short half-life have been coupled to carrier proteins and then the half-lives the desired product were significantly prolonged. It is proven from the current result that the Fc domain of antibody and HSA can act as these kinds of carrier proteins. However, the Fc domain, which is not an inert protein, can mediate many immune responses. Additionally, Fc variants have been patented to Amgen Inc. Taken together, the application of the Fc domain as carrier protein is limited.

HSA is the major protein component of human blood plasma. It is comprised of 585 amino acid residues, and its molecular weight is about 66.5 KDa. Spherical shape HSA is single chain molecule. It is the main and fundamental carrier protein in blood plasma without enzymatic and immunological activity, which can carry and transport polypeptide, fatty acids, steroids and hormones (Fuyan et al., 2011). Because HSA is an inert protein, it can defense against the enzymatic degradation with a half-life of 14 - 20 days (Chuang and Otagiri, 2002). At present, HSA usually serves as carrier for many endogenous substances or exogenous drugs. After binding with these endogenous substances or exogenous drugs, HSA can increase their molecular weight and radius of hydration, thus reducing the ratio removed by renal glomerular filtration, reducing their bioavailability, improving their plasma half-lives. Yeh et al. (1992) firstly reported that HSA acts as a carrier to couple with CD4 by expression of fusion protein in yeast, then long-acting fusion protein drug for treating human diseases is obtained. In contrast to IFNα, the plasma half-life of HSA-IFNα (Albuferonα) developed by HGSI can reach to 144 hours, and it has already finished its Phase III clinical trial. The plasma half-life of albutropin, which is growth hormone coupled with HSA, another drug also developed by HGSI, increased by six-fold longer than that of growth hormone itself after subcutaneous injection in cynomolgus monkeys (Osborn et al., 2002). There are other HSA fusion proteins, such as HSA-granulocyte growth factor (Albugranin) (Halpern et al., 2002), HSA-B-type brain natriuretic peptide (Wang et al., 2004), HSA-insulin (Duttaroy et al., 2005), HSA- IL-2 (Melder et al., 2005), which all entered into Phase I/II trial. Recently, lots of works have reported that HSA was used as a carrier protein for developing long-acting drugs (Chang et al., 2006; Fu et al., 2011; Guo et al., 2008; Huang et al., 2011; Jin et al., 2010; Ding et al., 2009; Li et al., 2009; Liu et al., 2011; Wang et al., 2007; Long et al., 2008; Zhang et al., 2009; Zhang et al., 2008; Zhou et al., 2008). It gives us a notion that the construction of HSA fusion protein has already become a general technology to extend plasma half-life of active peptides or protein drugs.

Thrombopoietin (TPO) is composed of 332 amino acid residues with two structural domains: the N terminal, amino domain of 153 amino acids and the C terminal, carbohydrate domain of 179 amino acids. It stimulates the proliferation, differentiation, maturation of megakaryocyte and the production of blood platelets. TPO is the most important regulator for platelet production. TPO also can accelerate the entry of primitive hematopoietic stem cells into cell cycles and promote their proliferation, while it has no obvious effect on granulocyte clony and red blood cells (Chen and Song, 1998; Sui and Xiao, 2002). As a result, TPO is important for physiologically blood coagulation. At present, recombinant human TPO (rhTPO) has already developed by Sansheng Co., Shengyang and marketed under a trade name Tebiao, for treating thrombocytopenia induced by radiotherapy and chemotherapy. However, its sequential clinical trail was terminated after its Phase I/II trial in USA due to its inducing the production of neutralizing antibody against TPO, then leading to invalid rhTPO at further use, even extremely thrombocytopenia. In fact, rhTPO has been only approved in China throughout the world (Guo and Liu, 2011).

Many chemotherapeutic drugs for cancer therapy can induce the suppression of bone marrow, and the reduction of blood platelets, and subsequently it increases the risk of spontaneous bleeding, even threats to lives of the patients. Although a variety of human recombinant cytokines, such as IL - 1, IL - 3, IL - 6, IL - 11, have some certain effects of promoting platelet counts, most of them cause side effects of headache, fever, and hypertension (Li et al., 2002).

TPO physiologically binds to its receptor of c-mpl at target cell surface, inducing c-mpl dimerized in association with a number of target proteins phosphorylation, and leading to signal cascade amplification response (Wang and Yang, 1998). The research on TPO crystal structure showed that it has two receptor recognition sites. Therefore, one TPO can bind with two receptors and lead to the receptors dimerized, further activate signaling molecules its downstream (Feese et al., 2004). The researchers screened a non-homologous TPO with high-activity of promoting production by phage display library technology. This kind of thromobopoietin mimetic peptide (TMP) has 14 amino acid residues. It can selectively bind to TPO receptor. Especially when it forms as dimers, its affinity to TPO receptor will be equivalent to the native TPO in vitro (Cwirl et al., 1997). However, because the molecular weight of TMP is small, it can easy to be filtered through renal glomerular filtration. Similar to other peptides and proteins with small molecular weight, its plasma half-life is also short, only a few minutes. TMP has been fused with antibody segments of Fc to form a long-acting fusion protein by Amgen, and it has entered into market under a trade name Nplate for treating idiopathic thrombocytopenia in August 2008. Nplate has the activity of TPO, but because it is non-homogenous to TPO, the production of neutralizing antibodies is avoided. The reverse effects of Nplate mainly come from unwanted immune responses due to Fc fragment being not an inert protein.

To overcome shortcoming of Nplate, TMP has been coupled with HSA to prolong its plasma half-life (CN201110420870.0) and the fusion protein can promote the proliferation of Mo7e cells. However, the activity of promoting platelet production can hardly be detected after subcutaneous injection in normal mice. The therapeutic efficacy on idiopathic thrombocytopenia mice can not be observed. Above all, a new generation of drug for promoting platelet production is urgent to be developed.

The invention herein provided the preparation of dimerized thrombopoietin mimetic peptide TMP-TMP-HSA fusion protein and its application. In the present invention a dimerized structural domain H was introduced between HSA and tandem TMP-TMP, and at the same time the fip structural domain is selectively introduced. The tandem TMP-TMP-HSA fusion protein will form a dimerized fusion protein during its expression. In present invention, the gene of TMP-TMP-HSA fusion protein has been cloned, and inserted into efficient expression plasmid for obtaining desired fusion protein in yeast or mammal cells. The purified expression product was subcutaneously injected to normal mice and the platelet counts significantly increased. Idiopathic and secondary thrombocytopenia mice received subcutaneous injection of desired fusion protein, and their symptoms were significantly ameliorated.

D1 (WO 03/031589) describes peptide compounds having thrombopoietic activity, where the TMP tandem dimer is linked to a Fc dimer.

D2 (CN 102408485) describes a fusion protein of a thrombopoietin mimetic peptide (TMP) diad and a human serum albumin (HSA) without dimerization domains.

### References

Chang Shao-Hong, Gong Xin, Yang Zhi-Yu, Wang Tong-Ying, Ma Guo-Chang, Ma Qing-Jun and Wu Jun. 2006. Expression in Pichia pastoris and Properties of Human Serum Albumin-Interferonα2b Chimera. Chinese Journal of Biotechnology 22: 173-179.
Chen Xue-Ling, Song Li.1998. Progress in the study of thrombopoietin. Foreign Medical Sciences Section of Pathophysiology and Clinical Medical. 18: 99-101.
Fu Yan, Han Guo,Yang Xiao-nan, Fu Yu-song, Xing Jing, Hu Jun-xin,Chen Ying,Yun Wen-qi,Yu Zai-lin. 2011. Study about construction and expression of two expression strain with long-acting recombinant human serum album interleukin-α fusion protein and physicochemical property of fusion protein. Chin Med Biotechnol. 6: 84-95.
Guo Ze-feng, Duan Zuo-ying, Zhang Lian-fen, Jin Jian, Li Hua-zhong. Expression of interleukin-2/human serum album fusion protein in Pichia pastria. Journal of China Pharmaceutical University 2008, 39(1): 82- 86
Huang Ying-chun, Gou Xing-hua, Han Lei, Li De-hua, Zhao Lan-ying, Wu Qia-qing. Construction and Expression of Recombinant Human Serum Albumin-EPO Fusion Protein.J Sichuan Univ (Med Sci Edi) 2011;42(3):317 - 321
Jin Guang-ze, Duan Zu-ying, Zhang Lian-fen, Jin Jian, Li Hua-zhong. Expression of the Fusion Protein Human Serum Album/Mutant Human Interleukin 2C125A in Pichia pastoris. Journal of Food Science and Biotechnology29: 595-601.
Ding Yue-di, Lei Jian-yong, Chen Yun, et al. Secretory Expression of (BNP) 2-HSA Fusion Protein in Pichia pastoris and Activity of Expressed Product. Chin J Biologicals 22: 226-229.
Li Dao-yuan, Zhou Wen-yi, Fan Qing-lin, Song Li-hua.2009. Clone expression of human serum album and granulocyte colony stimulating factor fusion protein. Journal of Biology 26: 34-36.
Liu Jun-Li, Liu Bo, Song Shu-Jun, Si Shao-Yan,Tan Xiao-Qing, Wu Jun, Jing Qing-Ping, Zhang Jian-Zhong. Expression of the rhOPG179-HSA Fusion Protein in Pichia pastoris. Letters in Biotechnology 22: 15-18.
Wang Nan, Zhao Hong- liang, Liu Zhi- min, Liu Dang- sheng, Xue Chong, Xiong Xiang- hua. Cloning and Expressing of HSA- GHRH Fusion Protein. Letters in Biotechnology 18: 574-577.
Long Juan, Xue Chong, Zhao Hong-Liang, Yang Xiao-Hong, Liu Zhi-Min. Expression of 2Gly-hGLP-1-HSA Fusion Protein in Pichia pastoris. Letters in Biotechnology 19: 488-492.
Zhang Mei, Wu Minchen, Jin Jian. Construction and Expression of Recombinant Fusion Protein hPTH (1-34) a'a-HSA. Biotechnology Bulletin: 117-121.
Zhang Xin-guo, Wang Jing,Yan Lu-ying,Tang Li, Chen Jian-hua,Wang Min. Molecular design and determination activity of recombinant human serum album thymopentin fusion protein. Pharmaceutical Biotechnology15: 444-448.
Zhou Liping, Zhang Lianfen, Lei Jianyong, Zhang Wenting, Cai Yanfei, Jin Jian. Secretory Expression of the Fusion Protein HSA-CP in Pichia Pastoris. Biotechnology Bulletin 3: 71-75.
Jinag He, Gong Meng-jia. Development of the Chemical Modification of Long-acting Peptide Drugs. Journal of Chongqing University of Technology (Natural Science) 26: 33-39.
Qi Nan, Ma Qing-jun .Progress in the study of long-acting recombinant protein drug. China Biotechnology 26: 79-82.
Zhao Hong-Liang, Xue Chong, Xiong Xiang-Hua, Zhang Wei, Yang Bing_Fen and Liu Zhi-Min. Purification and Activity Assay of HSA-AX15(R13K)
Fusion Protein Expressed in Pichia pastoris. Chinese Journal ofBiotechnolog21 : 224-258.
Sui Jie, Xiao Can-peng. 2002. Progress in the study of human thrombopoietin. Letters in Biotechnology 13: 76-82.
Wang Zhi-Qing,Yang Xin-Ke.1998.Molecular biology progress in the study of thrombopoietin. Chinese J Exp Clin Virol 12: 394-397.
Li Yue-Xi, Li Chao ,Tao Kai-Hua , Jia Xiang-Hong, Cheng Du-Sheng , Huang Pei-Tang. 2002. Fusion expression and bionomics of TPO simulated peptide and human IgG1Fc. Chinese Journal of Biotechnology18: 424-430.
Guo Tao, Liu Dan. 2011. The present of thrombopoietin receptor agonist for curing immune thrombocytopenia. Journal of clinical hematology24: 387-388.
Wang Jun-Pin, Shen Ming-Qiang, Xu Yang, Chen Fang,Chen Mo, Wang Song, Wang Ai-Ping, Li Yong-Ping. CN201110420870.0 The preparation of a dimerized thrombopoietin mimetic peptide TMP-TMP Human Serum Albumin (HSA) fusion protein and its application.
Chuang K.U., Otagiri M., 2002, Pharmaceutical strategies utilizing recombinant human serum albumin, Pharm Res 19(5): 569-577.
Yeh P., Landais D.M., Maury I., Crenne J.Y., Becquart J., Murry-Brelier A., Boucher F., Montay G., Fleer R., 1992. Design of yeast-secreted albumin derivatives for human therapy: biological and antiviral properties of a serum albumin CD4genetic conjugate. PNAS 89(5): 1904-1908.
Osborn B.L., Sekut L., Corcoran M., Poortman C., Sturm B., Chen G., Mather D., Lin H.L., Parry T.J., 2002. Albutropin: a growth hormone-albumin fusion with improved pharmacokinetics and pharmacodynamics in rats and monkeys. Eur J Pharm 456: 149-158.
Duttaroy A., Kanakaraj P., Osborn B.L., Schneider H., Pickeral O.K.; Chen C.; Zhang G., Kaithamana S., Singh M., Schulingkamp R., Crossan D., Bock J., Kaufman T.E., Reavey P., Carey-Barber M., Krishnan S. R., Garcia A., Murphy K., Siskind J.K., McLean M.A., Cheng S., Ruben S., Birse C.E., Blondel O., 2005. Development of a long-acting insulin analog using albumin fusion technology. Diabetes 54: 251-258.
Melder R.J., Osborn B.L., Riccobene T., Kanakaraj P., Wei P., Chen G., Stolow D., Halpern W.G., Migone T.S., Wang Q., Grzegorzewski K.J., Gallant G., 2005. Pharmacokinetics and in vitro and in vivo anti-tumor response of an interleukin-2-human serum albumin fusion protein in mice. Cancer Immunol. Immunother. 54: 535-547.
Wang W., Ou Y., Shi AlbuY., 2004. BNP, a recombinant B-type natriuretic peptide and human serum albumin fusion hormone, as a long-term therapy of congestive heart failure. Pharm. Res. 21: 2105-2111.
Halpem W, Riccobene TA, Agostini H, Baker K, Stolow D, Gu ML, Hirsch J, Mahoney A, Carrell J, Boyd E, Grzegorzewski KJ, 2002. AlbugraninTM, a Recombinant Human Granulocyte Colony Stimulating Factor (G-CSF) Genetically Fused to Recombinant Human Albumin Induces Prolonged Myelopoietic Effects in Mice and Monkey. Pharm Res.19:1720-1729.
Feese MD, Tamada T, Kato Y, et al. 2004. Structure of the receptor-binding domain of human thrombopoietin determined by complexation with a neutralizing antibody fragment. PNAS 101: 1816-21.
Cwirla S.E., Balasubramanian P., Duffin D.J., Wagstrom C.R., Gates C.M., Singer S.C., Davis Ann M., Tansik R.L., Mattheakis L.C., Boytos C.M., Schatz P.J., Baccanari D.P., Wrighton N.C., Barrett R.W., Dower W.J., 1997. Peptide agonist of the thrombopoietin receptor as potent as the nature cytokine. Science276: 1696-1699.

### Disclosure of the Invention

The present disclosure describes a method for preparation of dimerized TMP-TMP-HSA fusion protein and its application.

The present invention provides a dimerized thrombopoietin mimetic peptide-thrombopoietin mimetic peptide-Human Serum Albumin (TMP-TMP-HSA) fusion protein, comprising tandem dimer thrombopoietin (TMP), peptide linker and human serum albumin (HSA), characterized in that the fusion protein further contains a dimerization domain, wherein disulfide bonds in said dimerization domain contribute to dimerization of the TMP-TMP-HSA and wherein the dimerization domain is from H or fip, in which the dimerization domain from H has a gene sequence shown as Seq ID No.1 and an amino acid sequence shown as Seq ID No.2, the dimerization domain from fip has a gene sequence shown as Seq ID No.3 and an amino acid sequence shown as Seq ID No.4, and the dimerization domain is comprised between the tandem dimer thrombopoietin and the human serum albumin.

In the present invention, said dimerization domain leads to dimerization of two TMP-TMP-HSA fusion protein monomers through covalent bonds formed disulfide bonds, and to produce dimerized TMP-TMP-HSA fusion protein.

In the present invention, said dimerization domain may be selected from H and fip.

In the present invention, HSA is conjugated to the N-terminal of the fusion protein on a structural formula of TMP-L-L5-H-fip-HSA; L and L5 are peptide linkers, H is dimerized domain peptide, fip is selected from fip peptide.

In the present invention, HSA is conjugated to the C-terminal of the fusion protein on a structural formula of HSA-L6-H-L7-TMP-L-TMP; L, L6 and L7 are peptide linkers, H is dimerization domain peptide.

In the present invention, the gene sequence of L is GGTGGCGGCTCCGGA, the amino acid sequence is GGGSG.

In the present invention, the gene sequence of L is TCCGGA, the amino acid sequence is SG.

In the present invention, the gene sequence of L is GGTGGCCCCTCCGGA, the amino acid sequence is GGPSG.

In the present invention, the gene sequence of L is GGCGGTGGCGGCAGCGGTGGCGGCTCCGGA, the amino acid sequence is GGGGSGGGSG.

In the present invention, the gene sequence of L5 is GGCGGCGGCGGAAGCAGATCT, the amino acid sequence is GGGGSRS.

In the present invention, the gene sequence of L6 is GGCGGCGGCGGTTCCGGACTG, the amino acid sequence is GGGGSGL.

In the present invention, the gene sequence of L7 is GGTGGCGGCTCCGGA, the amino acid sequence is EFGGGGS.

In the present invention, said gene sequence of TMP is shown as Seq ID No:5, the amino acid sequence is shown as Seq ID No:6, but said amino acid sequence with mutations still has thrombopoietic activity.

In the present invention, said gene sequence of HSA is shown as Seq ID No:7, the amino acid sequence is shown as Seq ID No:8, but the amino acid sequence with mutations still has thrombopoietic activity.

The present invention also provides a mammalian cell comprising the dimerized TMP-TMP-HSA fusion protein of the invention.

In the present invention, said mammalian cell is CHO cell.

The present invention also provides a yeast cell comprising the dimerized TMP-TMP-HSA fusion protein of the invention.

In the present invention, said yeast cell is *Pichia pastoris.*

The present invention also provides a composition comprising a dimerized TMP-TMP-HSA fusion protein of the invention, for use in a method of treating primary or secondary thrombocytopenia diseases.

### Brief Description of Drawings

Figure 1 The construction map of dimerized TMP-TMP-HSA with the tandem TMP-TMP at N-terminal of HSA.
Figure 2 The construction map of dimerized TMP-TMP-HSA with the tandem TMP-TMP at C-terminal of HSA.
Figure 3 The construction of pPinkα-TMP-L-TMP-L5-H-fip-HSA vector for the expression of dimerized TMP-TMP-HSA fusion protein in yeast.
Figure 4 The construction map of dimerized TMP-TMP-HSA with the tandem TMP-TMP at N-terminal of HSA.
Figure 5 The construction of pPinkα-HSA-L6-H-L7-TMP-L-TMP vector for the expression of dimerized TMP-TMP-HSA fusion protein in yeast.
Figure 6 The construction map of dimerized TMP-TMP-HSA with the tandem TMP-TMP at C-terminal of HSA.

### Detailed Description of Embodiments

### Example 1 Construction and screening of CHO expression vector of dimerized thrombopoietin mimetic peptide TMP-TMP Human Serum Albumin (HSA) fusion protein

### 1. Construction of vector containing TMP-L1-TMP-L5-H-fip-HSA

Clone of HSA cDNA fragment: HSA cDNA was obtained from plasmid pcDNA3.1-fip-HSA (complete HSA gene is fully synthesized by TaKaRa Biotechnology (Dalian) Co., Ltd., and plasmid pcDNA3.1 was purchased from Invitrogen, USA) by using two-step PCR amplification, which is fip (Post1)-HSA (Pre1) (fip is the peptide from fip domain, "Pre" means the first half part of HSA, "Post" denotes the latter part of HSA, its gene sequence as indicated in Seq ID No: 9, its amino acid sequence as shown in Seq ID No: 10) and HSA (Post1)-His-tag (Post fragment of HSA, His-tag, gene sequence of HSA (Post1)-His-tag is shown in Seq ID No:11, and the amino acid sequence is shown in Seq ID No: 12) respectively. HSA (Pre1) was amplified by primer P1 (cgtggccatcatggagaccaaccccagcatcgatgcacacaagagtgaggttgctc) and primer P2 (cacagcacttctctagagtggtttc). Then the desired fragment was purified by PCR purification Kit (Sangon Biotech (Shanghai) Co., Ltd.). Fip (Post1)-HSA (Pre1) was amplified with HSA (Pre1) as a template, with P2 (cacagcacttctctagagtggtttc) and P3 (gaagctgcaggactacatcgacaggatcatcgtggccatcatggagaccaacccc) as the primer. The desired fragment was digested with restriction enzyme PstI and XbaI (Sangon) and recoveried by Gel Extraction Kit (Sangon). HAS (Post1)-His-tag was amplified by primer P4 (gaaaccactctagagaagtgctgtg) and primer P5 (gaaactcgagtcagtggtggtggtggtggtgtaagcctaaggcagcttgacttgcagc). The desired fragment was digested with restriction enzyme XbaI and XhoI (Sangon) and recovered by Gel Extraction Kit. All primers mentioned above are synthesized by TaKaRa.

Clone of ss-TMP-L1-TMP-L5-H-fip(Pre1): Utilizing the method of gene synthesis to prepare TMP gene ss-TMP-L1-TMP (ss is signal peptide, TMP is thrombopoietin mimetic peptide, L1 is peptide linker, and its gene sequence is GGTGGCGGCTCCGGA, the amino acid sequence is GGGSG, the gene sequence of ss-TMP-L1-TMP is shown in Seq ID No: 13, amino acid sequence is shown in Seq ID No: 14) which contains signal sequence and linker, this sequence is synthesized by TaKaRa, and inserted the gene fragment mentioned above into pMD19-T Simple vector. The fragment of TMP cDNA was amplified from plasmid pMD19-T-ss-TMP-L1-TMP by PCR. ss-TMP-L1-TMP was amplified by primer P6 (ctggatggcctccatcagctcgtccctgctcacgcacggtgggcatgtgtgagttttg) and primer P7 (gaagaagctttgctatggagacagacacactcct). Then the product was purified the by Cyclo-Prep PCR Clean-Up Kit (Sangon). Then using the ss-TMP-L1-TMP as a template and by primer P7 (gaagaagctttgctatggagacagacacactcct) and primer P8 (gaagctgcagcctgaagttgatctcctcctgcttctggatggcctccatcagctcgtc) further obtain the amplified production of ss-TMP-L1-TMP-L5-H-fip (Pre1). Its gene sequence is shown in Seq ID No: 15, and the amino acid sequence is shown in Seq ID No: 16. And the sequence was then digested with Hindlll and Pstl and the desired fragment of ss-TMP-L1-TMP-L5-H-fip (Pre1) was recovered by Gel Extraction Kit.

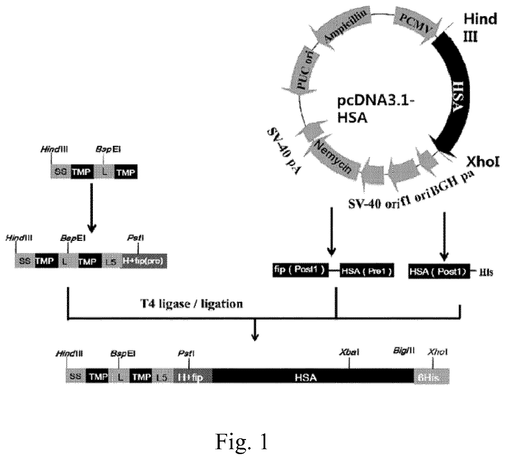

Construction of plasmid containing ss-TMP-L1-TMP-L5-H-fip-HSA-His: Plasmid pcDNA3.1-fip-HSA was digested with restriction enzyme HindIII and XhoI and the desired fragment of vector pcDNA3.1 was recovered by Gel Extraction Kit. Mixed the three prepared fragments of fip (Post1)-HSA (Pre1), HSA (Post1)-His and ss-TMP-L1-TMP-L5-H-fip (Pre1), with the recovered vector above by a Mole ratio of 5:5:5:1. 5U T4 ligase was then added in this reaction system, 16 °C for 4 hrs. The ligation product was transformed into E. coil DH5α (Tiangen) by the method of CaCl₂ hot shock. Transformed bacteria was spread on the LB plate (tryptone 10 g, yeast extract 5 g, NaCl 10 g, pH 7.0, added deionized water to 1 L) which contains ampicillin. Clones were picked from the plate and inoculated in 1 mL LB fluid medium, cultured at 220 rpm, 37 °C for 6-8 hrs. PCR was used to screen out positive colonies. Plasmid DNA was further isolated, and following cut by restriction enzymes. The correct clone will be identified by sequence to detect whether there is any mutant in the interest gene. The desired clone 1 which contains the interest gene of ss-TMP-L1-TMP-L5-H-fip-HSA-His-tag was finally obtained. The sequence of desired clone 1 is shown in Seq ID No: 17, and amino acid sequence is shown in Seq ID No:18. The map of the recombinant gene fragment (N-terminal sequence) of dimerized HSA protein vector and TPO mimetic peptide fusion protein is shown in Figure 1.

### 2. Construction of vector containing TMP-L2-TMP-L5-H-fip-HSA

Clone of HSA cDNA fragment: HSA cDNA was obtained from plasmid pcDNA3.1-fip-HSA (complete HSA gene is fully synthesized by TaKaRa, and plasmid pcDNA3.1 is from Invitrogen). HSA cDNA was amplified by using two-step PCR to obtain fip (Post1)-HSA (Pre1) (fip is the peptide from fip domain, Pre means the first half part of HSA, Post denotes the latter part of HSA, its gene sequence as indicated in Seq ID No:9, its amino acid sequence as shown in Seq ID No:10) and HSA (Post1)-His-tag (Post fragment of HSA, His-tag, gene sequence of HSA (Post1)-His-tag is shown in Seq ID No:11, and the amino acid sequence is shown in Seq ID No:12) respectively. HSA(Pre1) was amplified by primer P1 (cgtggccatcatggagaccaaccccagcatcgatgcacacaagagtgaggttgctc) and primer P2 (cacagcacttctctagagtggtttc). Then the resultant production was purified by PCR purification Kit (Sangon). Fip (Post1)-HSA (Pre1) was amplified with HSA (Pre1) as a template, with P2 (cacagcacttctctagagtggtttc) and P3 (gaagctgcaggactacatcgacaggatcatcgtggccatcatggagaccaacccc) as the primer. The resultant interest gene was cut by restriction enzyme PstI and XbaI (Sangon) and then recovered by Gel Extraction Kit (Sangon). HSA (Post1)-His-tag is amplified by primer P4 (gaaaccactctagagaagtgctgtg) and primer P5 (gaaactcgagtcagtggtggtggtggtggtgtaagcctaaggcagcttgacttgcagc). The desired fragment was digested by restriction enzyme XbaI and XhoI (Sangon) and recovered by Gel Extraction Kit. All primers mentioned above is synthesized by TaKaRa.

Clone of ss-TMP-L2-TMP-L5-H-fip(Pre1): Utilizing the method of gene synthesis to prepare TMP gene ss-TMP-L2-TMP (ss is signal peptide, TMP is thrombopoietin mimetic peptide, L2 is peptide linker, and its gene sequence is TCCGGA, amino acid sequence is SG), which contains signal sequence and the linker, was fully synthesized by TaKaRa, and inserted the gene fragment mentioned above into pMD19-T Simple vector. TMP cDNA was amplified from plasmid pMD19-T-ss-TMP-L2-TMP by PCR. ss-TMP-L2-TMP was amplified by primer P6 (ctggatggcctccatcagctcgtccctgctcacgcacggtgggcatgtgtgagttttg) and primer P7 (gaagaagctttgctatggagacagacacactcct). Then the PCR production of ss-TMP-L2-TMP was purified by Cyclo-Prep PCR Clean-Up Kit (Sangon). Following the ss-TMP-L2-TMP acted as a template and P7 (gaagaagctttgctatggagacagacacactcct) and P8 (gaagctgcagcctgaagttgatctcctcctgcttctggatggcctccatcagctcgtc) served as primers to further obtained ss-TMP-L2-TMP-L5-H-fip (Pre1) also by PCR. The resultant gene fragment was digested by HindIII and PstI and recovered by Gel Extraction Kit to obtain purified fragment of ss-TMP-L2-TMP-L5-H-fip (Pre1).

Construction of plasmid containing ss-TMP-L2-TMP-L5-H-fip-HSA-His: Plasmid pcDNA3.1-fip-HSA was cut by HindIII and XhoI and recovered by Gel Extraction Kit to obtain vector pcDNA3.1. The three recovered fragment of fip(Post1)-HSA (Pre1), HSA (Post1)-His, ss-TMP-L2-TMP-L5-H-fip (Pre1), and the recovered vector was mixed by a Mole ratio of 5:5:5:1. 5U T4 ligase was added in this reaction system, 16 °C for 4 hrs. The ligation product was transformed into Escherichia coli DH5α (Tiangen) by the method of CaCl2 hot shock. Transformed bacteria was spread on the LB plate (tryptone 10 g, yeast extract 5 g, NaCl 10 g, pH 7.0, added deionized water to 1 L) which contains ampicillin. Clones were picked from the plate and inoculated in 1 mL LB fluid medium, cultured at 220 rpm, 37 °C for 6-8hrs. PCR was then used to screen out positive colonies. Plasmid DNA was further isolated and following cut by restriction enzymes. The correct clones will be identified by sequence to detect whether there is any mutant in the interest gene. The correct clone 2 which contains the interest gene of ss-TMP-L2-TMP-L5-H-fip-HSA-His-tag was finally obtained. The sequence of desired clone 2 is shown in Seq ID No: 19, and amino acid sequence is shown in Seq ID No: 20.

### 3. Construction of vector containing TMP-L3-TMP-L5-H-fip-HSA

Clone of HSA cDNA fragment: HSA cDNA was obtained from plasmid pcDNA3.1-fip-HSA(complete HSA gene is fully synthesized by TaKaRa, and plasmid pcDNA3.1 is from Invitrogen) by using two-step PCR amplification, that is fip (Post1)-HSA (Pre1) (fip is the peptide from fip domain, Pre means the first half part of HSA, Post denotes the latter part of HSA, its gene sequence as indicated in Seq ID No: 9, its amino acid sequence as shown in Seq ID No: 10) and HSA (Post1)-His-tag (Post fragment of HSA, His-tag, gene sequence of HSA (Post1)-His-tag is shown in Seq ID No: 11, and the amino acid sequence is shown in Seq ID No: 12) respectively. HSA (Pre1) was amplified by primer P1 (cgtggccatcatggagaccaaccccagcatcgatgcacacaagagtgaggttgctc) and primer P2 (cacagcacttctctagagtggtttc). Then the resultant production was purified by PCR purification Kit (Sangon). Fip (Post1)-HSA (Pre1) was amplified with HSA (Pre1) as a template, with P2 (cacagcacttctctagagtggtttc) and P3 (gaagctgcaggactacatcgacaggatcatcgtggccatcatggagaccaacccc) as the primer. The resultant interest gene was cut by restriction enzyme PstI and XbaI (Sangon) and recovered by Gel Extraction Kit (Sangon). HSA(Post1)-His-tag was amplified by primer P4 (gaaaccactctagagaagtgctgtg) and primer P5 (gaaactcgagtcagtggtggtggtggtggtgtaagcctaaggcagcttgacttgcagc). The resultant interest gene was cut by restriction enzyme XbaI and XhoI (Sangon) and recovered the desired fragment by Gel Extraction Kit. All primers mentioned above is synthesized by TaKaRa.

Clone of ss-TMP-L3-TMP-L5-H-fip(Pre1): Utilizing the method of gene synthesis to prepare TMP gene ss-TMP-L3-TMP (ss is signal peptide, TMP is thrombopoietin mimetic peptide, L3 is peptide linker, and its gene sequence is GGTGGCCCCTCCGGA, amino acid sequence is GGPSG) which contains signal sequence and linker, was synthesized by TaKaRa, and inserted the gene fragment mentioned above into pMD19-T Simple vector. The fragment of TMP cDNA was amplified from plasmid pMD19-T-ss-TMP-L3-TMP by PCR. ss-TMP-L3-TMP was amplified by primer P6 (ctggatggcctccatcagctcgtccctgctcacgcacggtgggcatgtgtgagttttg) and primer P7 (gaagaagctttgctatggagacagacacactcct). Then the product was purified by Cyclo-Prep PCR Clean-Up Kit (Sangon). Following the ss-TMP-L3-TMP acted as a template and P7 (gaagaagctttgctatggagacagacacactcct) and P8 (gaagctgcagcctgaagttgatctcctcctgcttctggatggcctccatcagctcgtc) served as primers to further obtained ss-TMP-L3-TMP-L5-H-fip (Pre1) also by PCR. The resultant gene fragment was digested by HindIII and PstI and recovered by Gel Extraction Kit to obtain purified fragment of ss-TMP-L3-TMP-L5-H-fip (Pre1).

Construction of plasmid containing ss-TMP-L3-TMP-L5-H-fip-HSA-His: Plasmid pcDNA3.1-fip-HSA was digested with restriction enzyme HindIII and XhoI and the desired fragment of vector pcDNA3.1 was recovered by Gel Extraction Kit. Mixed the three prepared fragments of fip (Post1)-HSA (Pre1), HSA (Post1)-His and ss-TMP-L3-TMP-L5-H-fip (Pre1) with the recovered vector above by a Mole ratio of 5:5:5:1. 5U T4 ligase was then added in this reaction system, 16 °C for 4 hrs. The ligation product was transformed into Escherichia coli DH5α (Tiangen) by the method of CaCl₂ hot shock. Transformed bacteria was spread on the LB plate (tryptone 10 g, yeast extract 5 g, NaCl 10 g, pH 7.0. Added deionized water to 1 L) which contains ampicillin. Clones were picked from the plate and inoculated in 1 mL LB fluid medium, cultured at 220 rpm, 37 °C for 6-8 hrs. PCR was then used to screen out positive colonies. Plasmid DNA was further isolated and following cut by restriction enzymes. The correct clones will be identified by sequence to detect whether there is any mutant in the interest gene. The correct clone 3 which contains the interest gene of ss-TMP-L3-TMP-L5-H-fip-HSA-His-tag was finally obtained. The sequence of desired clone 3 is shown in Seq ID No: 21, and amino acid sequence is shown in Seq ID No: 22.

### 4. Construction of vector containing TMP-L4-TMP-L5-H-fip-HSA

Clone of HSA cDNA fragment: HSA cDNA was obtained from plasmid pcDNA3.1-fip-HSA (complete HSA gene is fully synthesized by TaKaRa, and plasmid pcDNA3.1 is from Invitrogen) by using two-step PCR amplification, that is fip (Post1)-HSA (Pre1) (fip is the peptide from fip domain, Pre means the first half part of HSA, Post denotes the latter part of HSA, its gene sequence as indicated in Seq ID No:9, the amino acid sequence as shown in Seq ID No:10) and HSA (Post1)-His-tag (Post fragment of HSA, His-tag, gene sequence of HSA (Post1)-His-tag is shown in Seq ID No: 11, and the amino acid sequence is shown in Seq ID No:12) respectively. HSA (Pre1) was amplified by primer P1 (cgtggccatcatggagaccaaccccagcatcgatgcacacaagagtgaggttgctc) and primer P2 (cacagcacttctctagagtggtttc). Then the resultant production was purified by PCR purification Kit (Sangon). Fip (Post1)-HSA (Pre1) was amplified with HSA (Pre1) as a template, with P2 (cacagcacttctctagagtggtttc) and P3 (gaagctgcaggactacatcgacaggatcatcgtggccatcatggagaccaacccc) as the primer. The resultant interest gene was cut by restriction enzyme PstI and XbaI (Sangon) and recovered the fragment by Gel Extraction Kit (Sangon). HSA (Post1)-His-tag was amplified by primer P4 (gaaaccactctagagaagtgctgtg) and primer P5 (gaaactcgagtcagtggtggtggtggtggtgtaagcctaaggcagcttgacttgcagc). HSA (Post1)-His-tag was digested by restriction enzyme XbaI and XhoI (Sangon) and recovered fragment by Gel Extraction Kit. All primers mentioned above is synthesized by TaKaRa.

Clone of ss-TMP-L4-TMP-L5-H-fip(Pre1): Utilizing the method of gene synthesis to prepare TMP gene ss-TMP-L4-TMP (ss is signal peptide, TMP is thrombopoietin mimetic peptide, L4 is peptide linker, and its gene sequence is GGCGGTGGCGGCAGCGGTGGCGGCTCCGGA, amino acid sequence is GGGGSGGGSG) which contains signal sequence and linker, this sequence is synthesized by TaKaRa, and inserted the gene fragment mentioned above into pMD19-T Simple vector. The fragment of TMP cDNA was amplified from plasmid pMD19-T-ss-TMP-L4-TMP by PCR. The amplified production is ss-TMP-L4-TMP by primer P6 (ctggatggcctccatcagctcgtccctgctcacgcacggtgggcatgtgtgagttttg) and primer P7 (gaagaagctttgctatggagacagacacactcct). Then purified by Cyclo-Prep PCR Clean-Up Kit (Sangon). Following the ss-TMP-L4-TMP acted as a template and P7 (gaagaagctttgctatggagacagacacactcct) and P8 (gaagctgcagcctgaagttgatctcctcctgcttctggatggcctccatcagctcgtc) served as primers to further obtained ss-TMP-L4-TMP-L5-H-fip (Pre1) also by PCR. The resultant gene fragment was digested by HindIII and PstI and recovered by Gel Extraction Kit to obtain purified fragment of ss-TMP-L4-TMP-L5-H-fip(Pre1).

Construction of plasmid containing ss-TMP-L4-TMP-L5-H-fip-HSA-His: Plasmid pcDNA3.1-fip-HSA was digested with restriction enzyme HindIII and XhoI and the desired fragment of vector pcDNA3.1 was recovered by Gel Extraction Kit. Mixed the three prepared fragments of fip (Post1)-HSA (Pre1), HSA (Post1)-His and ss-TMP-L4-TMP-L5-H-fip (Pre1) with the recovered vector above by a Mole ratio of 5:5:5:1. 5U T4 ligase was then added in this reaction system, 16 °C for 4 hrs. The ligation product was transformed into Escherichia coli DH5α (Tiangen) by the method of CaCl2 hot shock. Transformed bacteria was spread on the LB plate (tryptone 10g, yeast extract 5 g, NaCl 10 g, pH 7.0. Added deionized water to 1 L) which contains ampicillin. Clones were picked from the plate and inoculated in 1 mL LB fluid medium, cultured at 220 rpm, 37 °C for 6-8 hrs. PCR was then used to screen out positive colonies. Plasmid DNA was further isolated and following cut by restriction enzymes. The correct clone will be identified by sequence to detect whether there is any mutant in the interest gene. The desired clone 4 which contains the interest gene of ss-TMP-L4-TMP-L5-H-fip-HSA-His-tag was finally obtained. The sequence of desired clone 4 is shown in Seq ID No: 23, and amino acid sequence is shown in Seq ID No:24.

### 5. Construction of vector containing HSA-L6-H-L7-TMP-L1-TMP

Clone of ss-HSA-L6-H-L7: HSA(Post2)-His-tag was amplified by using P4 (gaaaccactctagagaagtgctgtg) and P5 (gaaactcgagtcagtggtggtggtggtggtgtaagcctaaggcagcttgacttgcagc) as primer. Its gene sequence is indicated in Seq ID No: 25, the amino acid sequence is shown in Seq ID No:26. The resultant production was digested by restriction enzyme XbaI and XhoI (Sangon) and recovered fragment by Gel Extraction Kit (Sangon). All primers mentioned above is synthesized by TaKaRa.

HSA (Mid) was amplified by primer P1 (gaagctgcaggactacatcgacaggatcatcgtggccatcatggagaccaacccc) and primer P2 (cgtggccatcatggagaccaaccccagcatcgatgcacacaagagtgaggttgctc). Its gene sequence is indicated in Seq ID No: 27, the amino acid sequence is shown in Seq ID No: 28. Then HSA (Mid) was purified by PCR purification Kit (Sangon).

Utilizing the method of gene synthesis to prepare the signal sequence expressed in CHO cell and the front end of HSA gene sequence ss-HSA (Pre2), this sequence is synthesized by TaKaRa, and inserted the gene fragment mentioned above into pMD19-T Simple vector. Plasmid pMD19-T-ss-HSA (Pre2) was digested with restriction enzyme HindIII and MseI (NEB) to obtain the fragment of ss-HSA (Pre2), the gene sequence is indicated in Seq ID No:29, the amino acid sequence is shown in Seq ID No: 30. And the desired fragment was recovered by Gel Extraction Kit.

Plasmid pcDNA3.1-fip-HSA was digested with restriction enzyme HindIII and XhoI and recovered fragment by Gel Extraction Kit to obtain vector pcDNA3.1. Mixed the three recovered fragment of ss-HSA (Pre2), HSA(Mid) and HSA (Post2)-His-tag with the recovered vector above by a Mole ratio of 5:5:5:1. 5U T4 ligase was then added in this reaction system, 16 °C for 4 hrs. The ligation product was transformed into Escherichia coli DH5α (Tiangen) by the method of CaCl₂ hot shock. Transformed bacteria was spread on the LB plate which contained ampicillin. Colonies with recombinant picked out was named pcDNA3.1-ss-HSA.

HSA cDNA was obtained from plasmid pcDNA3.1-ss-HSA by using two-step PCR. ss-HSA (Pre3) was amplified by primer P7 (gaagaagctttgctatggagacagacacactcct) and primer P2 (cgtggccatcatggagaccaaccccagcatcgatgcacacaagagtgaggttgctc). The gene sequence of ss-HSA (Pre3) is shown in Seq ID No: 31, and the amino acid sequence is shown in Seq ID No: 32. The fragment was digested with restriction enzyme HindIII and XbaI and recovered it by Gel Extraction Kit. HSA(Post3)-L6-H-L7 was amplified by primer P4 (gaaaccactctagagaagtgctgtg) and primer P9 (gaaggaattccttgccgggggacaggctca). The gene sequence is shown in Seq ID No: 33, and the amino acid sequence is shown in Seq ID No: 34. The desired fragment was digested with restriction enzyme XbaI and EcoRI and recovered fragment by Gel Extraction Kit.

The synthesis of TMP-L1-TMP sequence: Utilizing the method of gene synthesis to prepare TMP gene TMP-L1-TMP (the gene sequence of L1 is GGTGGCGGCTCCGGA, amino acid sequence is GGGSG, this sequence is fully synthesized by TaKaRa, and inserted the gene fragment mentioned above into pMD19-T Simple vector). pMD19-T-TMP-L1-TMP was digested with restriction enzyme EcoRI and XhoI to obtain the fragment of TMP-L1-TMP and recovered fragment by Gel Extraction Kit.

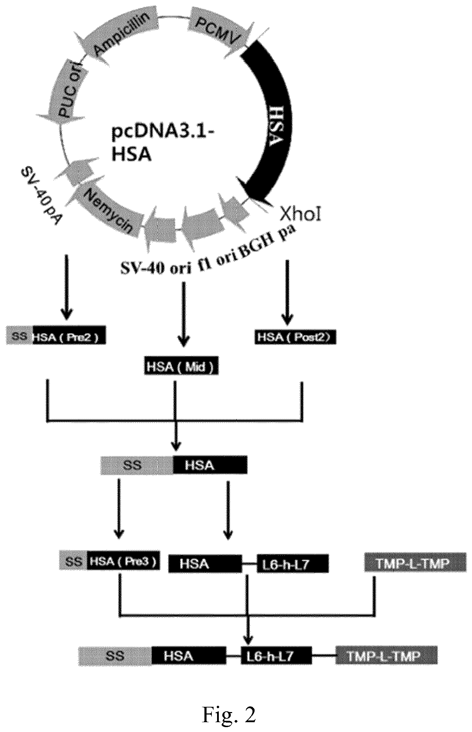

Construction of plasmid containing ss-HSA-L6-H-L7-TMP-L1-TMP: Plasmid pcDNA3.1-fip-HSA was digested with restriction enzyme HindIII and XhoI and recovered fragment by Gel Extraction Kit to obtain vector pcDNA3.1. Mixed the three recovered fragment of ss-HSA (pre3), HSA (Post3)-L6-H-L7 and TMP-L1-TMP, with the recovered vector above by a Mole ratio of 5:5:5:1. 5U T4 ligase was added in this reaction system, 16 °C for 4 hrs. The ligation product was transformed into Escherichia coli DH5α (Tiangen) by the method of CaCl₂ hot shock. Transformed bacteria was spread on the LB plate which contains ampicillin. Clones were picked from the plate and inoculated in 1 mL LB fluid medium, cultured at 220 rpm, 37 °C for 6-8 hrs. PCR was then used to screen out positive colonies. Plasmid DNA was further isolated and following cut by restriction enzymes. The correct clone will be identified by sequence to detect whether there is any mutant in the interest gene. The desired clone 5 which contains the interest gene of ss-HSA-L6-H-L7-TMP-L1 -TMP was finally obtained. The sequence of desired clone 5 is shown in Seq ID No: 35, and amino acid sequence is shown in Seq ID No: 36. The constructed map of the gene fragment (C-terminal sequence) of dimerized HSA protein vector and TPO mimetic peptide fusion protein is shown in Figure 2.

### 6. Construction of vector containing HSA-L6-H-L7-TMP-L2-TMP

Clone of ss-HSA-L6-H-L7: HSA (Post2)-His-tag was amplified by using P4 (gaaaccactctagagaagtgctgtg) and P5 (gaaactcgagtcagtggtggtggtggtggtgtaagcctaaggcagcttgacttgcagc) as primer. Its gene sequence is indicated in Seq ID No: 25, the amino acid sequence is shown in Seq ID No: 26. The resultant production was digested by restriction enzyme XbaI and XhoI (Sangon) and recovered fragment by Gel Extraction Kit (Sangon). All primers mentioned above is synthesized by TaKaRa.

HSA (Mid) was amplified by primer P1 (gaagctgcaggactacatcgacaggatcatcgtggccatcatggagaccaacccc) and primer P2 (cgtggccatcatggagaccaaccccagcatcgatgcacacaagagtgaggttgctc). Its gene sequence is indicated in Seq ID No: 27, the amino acid sequence is shown in Seq ID No: 28. Then HSA (Mid) was purified by PCR purification Kit (Sangon).

Utilizing the method of gene synthesis to prepare the signal sequence expressed in CHO cell and the front end of HSA gene sequence ss-HSA (Pre2), this sequence is synthesized by TaKaRa, and inserted the gene fragment mentioned above into pMD19-T Simple vector. Plasmid pMD19-T-ss-HSA (Pre2) was digested with restriction enzyme HindIII and MseI (NEB) to obtain the fragment of ss-HSA (Pre2), the gene sequence is indicated in Seq ID No: 29, the amino acid sequence is shown in Seq ID No: 30. And the desired fragment was recovered by Gel Extraction Kit.

Plasmid pcDNA3.1-fip-HSA was digested with restriction enzyme HindIII and XhoI and recovered fragment by Gel Extraction Kit to obtain vector pcDNA3.1. Mixed the three recovered fragment of ss-HSA (Pre2), HSA(Mid) and HSA (Post2)-His-tag with the recovered vector above by a Mole ratio of 5:5:5:1. 5U T4 ligase was then added in this reaction system, 16°C for 4 hrs. The ligation product was transformed into Escherichia coli DH5α (Tiangen) by the method of CaCl₂ hot shock. Transformed bacteria was spread on the LB plate which contains ampicillin. Colonies with recombinant picked out was named pcDNA3.1-ss-HSA.

HSA cDNA was obtained from plasmid pcDNA3.1-ss-HSA by using two-step PCR. ss-HSA (Pre3) was amplified by primer P7 (gaagaagctttgctatggagacagacacactcct) and primer P2 (cgtggccatcatggagaccaaccccagcatcgatgcacacaagagtgaggttgctc). The gene sequence of ss-HSA (Pre3) is shown in Seq ID No: 31, and the amino acid sequence is shown in Seq ID No: 32. The fragment was digested with restriction enzyme HindIII and XbaI and recovered it by Gel Extraction Kit. HSA (Post3)-L6-H-L7 was amplified by primer P4 (gaaaccactctagagaagtgctgtg) and primer P9 (gaaggaattccttgccgggggacaggctca). The gene sequence is shown in Seq ID No: 33, and the amino acid sequence is shown in Seq ID No: 34. The desired fragment was digested with restriction enzyme XbaI and EcoRI and recovered fragment by Gel Extraction Kit.

The synthesis of TMP-L2-TMP sequence: Utilizing the method of gene synthesis to prepare TMP gene TMP-L2-TMP (the gene sequence of L2 is TCCGGA, amino acid sequence is SG, this sequence is synthesized by TaKaRa, and inserted the gene fragment mentioned above into pMD19-T Simple vector.). pMD19-T-TMP-L2-TMP was digested with restriction enzyme EcoRI and XhoI to obtain the fragment of TMP-L2-TMP and recovered fragment by Gel Extraction Kit.

Construction of plasmid containing ss-HSA-L6-H-L7-TMP-L2-TMP: Plasmid pcDNA3.1-fip-HSA was digested with restriction enzyme HindIII and XhoI and recovered fragment by Gel Extraction Kit to obtain vector pcDNA3.1. Mixed the three recovered fragment of ss-HSA (pre3), HSA(Post3)-L6-H-L7 and TMP-L2-TMP, with the recovered vector above by a Mole ratio of 5:5:5:1. 5U T4 ligase was added in this reaction system, 16 °C for 4 hrs. The ligation product was transformed into Escherichia coli DH5α (Tiangen) by the method of CaCl₂ hot shock. Transformed bacteria was spread on the LB plate which contains ampicillin. Clones were picked from the plate and inoculated in 1 mL LB fluid medium, cultured at 220 rpm, 37 °C for 6-8 hrs. PCR was then used to screen out positive colonies. Plasmid DNA was further isolated and following cut by restriction enzymes. The correct clone will be identified by sequence to detect whether there is any mutant in the interest gene. The desired clone 6 which contains the interest gene of ss-HSA-L6-H-L7-TMP-L2-TMP was finally obtained. The sequence of desired clone 6 is shown in Seq ID No: 37, and amino acid sequence is shown in Seq ID No: 38.

### 7. Construction of vector containing HSA-L6-H-L7-TMP-L3-TMP

Clone of ss-HSA-L6-H-L7: Clone of ss-HSA-L6-H-L7: HSA (Post2)-His-tag was amplified by using P4 (gaaaccactctagagaagtgctgtg) and P5 (gaaactcgagtcagtggtggtggtggtggtgtaagcctaaggcagcttgacttgcagc) as primer. Its gene sequence is indicated in Seq ID No: 25, the amino acid sequence is shown in Seq ID No: 26. The resultant production was digested by restriction enzyme XbaI and XhoI (Sangon) and recovered fragment by Gel Extraction Kit (Sangon). All primers mentioned above is synthesized by TaKaRa.

HSA (Mid) was amplified by primer P1 (gaagctgcaggactacatcgacaggatcatcgtggccatcatggagaccaacccc) and primer P2 (cgtggccatcatggagaccaaccccagcatcgatgcacacaagagtgaggttgctc). Its gene sequence is indicated in Seq ID No: 27, the amino acid sequence is shown in Seq ID No: 28. Then HSA (Mid) was purified by PCR purification Kit (Sangon).

Utilizing the method of gene synthesis to prepare the signal sequence expressed in CHO cell and the front end of HSA gene sequence ss-HSA (Pre2), this sequence is synthesized by TaKaRa, and inserted the gene fragment mentioned above into pMD19-T Simple vector. Plasmid pMD19-T-ss-HSA (Pre2) was digested with restriction enzyme HindIII and MseI (NEB) to obtain the fragment of ss-HSA (Pre2), the gene sequence is indicated in Seq ID No: 29, the amino acid sequence is shown in Seq ID No: 30. And the desired fragment was recovered by Gel Extraction Kit.

Plasmid pcDNA3.1-fip-HSA was digested with restriction enzyme HindIII and XhoI and recovered fragment by Gel Extraction Kit to obtain vector pcDNA3.1. Mixed the three recovered fragment of ss-HSA (Pre2), HSA (Mid) and HSA (Post2)-His-tag with the recovered vector above by a Mole ratio of 5:5:5:1. 5U T4 ligase was then added in this reaction system, 16 °C for 4 hrs. The ligation product was transformed into Escherichia coli DH5α (Tiangen) by the method of CaCl2 hot shock. Transformed bacteria was spread on the LB plate which contains ampicillin. Colonies with recombinant picked out was named pcDNA3.1-ss-HSA.

HSA cDNA was obtained from plasmid pcDNA3.1-ss-HSA by using two-step PCR. Amplification ss-HSA (Pre3) was amplified by primer P7 (gaagaagctttgctatggagacagacacactcct) and primer P2 (cgtggccatcatggagaccaaccccagcatcgatgcacacaagagtgaggttgctc). The gene sequence of ss-HSA (Pre3) is shown in Seq ID No: 31, and the amino acid sequence is shown in Seq ID No: 32. The fragment was digested with restriction enzyme HindIII and XbaI and recovered it by Gel Extraction Kit. HSA (Post3)-L6-H-L7 was amplified by primer P4 (gaaaccactctagagaagtgctgtg) and primer P9 (gaaggaattccttgccgggggacaggctca). The gene sequence is shown in Seq ID No: 33, and the amino acid sequence is shown in Seq ID No: 34. The desired fragment was digested with restriction enzyme XbaI and EcoRI and recovered fragment by Gel Extraction Kit.

The synthesis of TMP-L3-TMP sequence: Utilizing the method of gene synthesis to prepare TMP gene TMP-L3-TMP (the gene sequence of L3 is GGTGGCCCCTCCGGA, amino acid sequence is GGPSG, this sequence is fully synthesized by TaKaRa, and inserted the gene fragment mentioned above into pMD19-T Simple vector). pMD19-T-TMP-L3-TMP was digested with restriction enzyme EcoRI and XhoI to obtain TMP-L3-TMP and recovered fragment by Gel Extraction Kit.

Construction of plasmid containing ss-HSA-L6-H-L7-TMP-L3-TMP: Plasmid pcDNA3.1-fip-HSA was digested with restriction enzyme HindIII and XhoI and recovered fragment by Gel Extraction Kit to obtain vector pcDNA3.1. Mixed the three recovered fragment of ss-HSA (pre3), HSA (Post3)-L6-H-L7 and TMP-L3-TMP, with the recovered vector above by a Mole ratio of 5:5:5:1. 5U T4 ligase was added in this reaction system, 16 °C for 4 hrs. The ligation product was transformed into Escherichia coli DH5α (Tiangen) by the method of CaCl₂ hot shock. Transformed bacteria was spread on the LB plate which contains ampicillin. Clones were picked from the plate and inoculated in 1 mL LB fluid medium, cultured at 220rpm, 37 °C for 6-8 hrs. PCR was then used to screen out positive colonies. Plasmid DNA was further isolated and following cut by restriction enzymes. The correct clone will be identified by sequence to detect whether there is any mutant in the interest gene. The desired clone 7 which contains the interest gene of ss-HSA-L6-H-L7-TMP-L3-TMP was finally obtained. The sequence of desired clone 8 is shown in Seq ID No: 39, and amino acid sequence is shown in Seq ID No: 40.

### 8. Construction of vector containing HSA-L6-H-L7-TMP-L4-TMP

Clone of ss-HSA-L6-H-L7: Clone of ss-HSA-L6-H-L7: HSA (Post2)-His-tag was amplified by using P4 (gaaaccactctagagaagtgctgtg) and P5 (gaaactcgagtcagtggtggtggtggtggtgtaagcctaaggcagcttgacttgcagc) as primer. Its gene sequence is indicated in Seq ID No: 25, the amino acid sequence is shown in Seq ID No: 26. The resultant production was digested by restriction enzyme XbaI and XhoI (Sangon) and recovered fragment by Gel Extraction Kit (Sangon). All primers mentioned above is synthesized by TaKaRa.

HSA (Mid) was amplified by primer P1 (gaagctgcaggactacatcgacaggatcatcgtggccatcatggagaccaacccc) and primer P2 (cgtggccatcatggagaccaaccccagcatcgatgcacacaagagtgaggttgctc). Its gene sequence is indicated in Seq ID No: 27, the amino acid sequence is shown in Seq ID No: 28. Then HSA (Mid) was purified by PCR purification Kit (Sangon).

Utilizing the method of gene synthesis to prepare the signal sequence expressed in CHO cell and the front end of HSA gene sequence ss-HSA(Pre2), this sequence is fully synthesized by TaKaRa, and inserted the gene fragment mentioned above into pMD19-T Simple vector. Plasmid pMD19-T-ss-HSA (Pre2) was digested with restriction enzyme HindIII and MseI (NEB) to obtain the fragment of ss-HSA (Pre2), the gene sequence is indicated in Seq ID No: 29, the amino acid sequence is shown in Seq ID No: 30. And the desired fragment was recovered by Gel Extraction Kit.

Plasmid pcDNA3.1-fip-HSA was digested with restriction enzyme HindIII and XhoI and recovered fragment by Gel Extraction Kit to obtain vector pcDNA3.1. Mixed the three recovered fragment of ss-HSA (Pre2), HSA (Mid) and HSA (Post2)-His-tag with the recovered vector above by a Mole ratio of 5:5:5:1. 5U T4 ligase was then added in this reaction system, 16 °C for 4 hrs. The ligation product was transformed into Escherichia coli DH5α (Tiangen) by the method of CaCl₂ hot shock. Transformed bacteria was spread on the LB plate which contains ampicillin. Colonies with recombinant picked out was named pcDNA3.1-ss-HSA.

HSA cDNA was obtained from plasmid pcDNA3.1-ss-HSA by using two-step PCR. ss-HSA (Pre3) was amplified by primer P7 (gaagaagctttgctatggagacagacacactcct) and primer P2 (cgtggccatcatggagaccaaccccagcatcgatgcacacaagagtgaggttgctc). The gene sequence of ss-HSA (Pre3) is shown in Seq ID No: 31, and the amino acid sequence is shown in Seq ID No: 32. The fragment was digested with restriction enzyme HindIII and XbaI and recovered it by Gel Extraction Kit. HSA (Post3)-L6-H-L7 was amplified by primer P4 (gaaaccactctagagaagtgctgtg) and primer P9(gaaggaattccttgccgggggacaggctca). The gene sequence is shown in Seq ID No: 33, and the amino acid sequence is shown in Seq ID No: 34. The desired fragment was digested with restriction enzyme XbaI and EcoRI and recovered fragment by Gel Extraction Kit.

The synthesis of TMP-L4-TMP sequence: Utilizing the method of gene synthesis to prepare TMP gene TMP-L4-TMP (the gene sequence of L4 is GGCGGTGGCGGCAGCGGTGGCGGCTCCGGA, amino acid sequence is GGGGSGGGSG, this sequence is fully synthesized by TaKaRa, and inserted the gene fragment mentioned above into pMD19-T Simple vector). pMD19-T-TMP-L4-TMP was digested with restriction enzyme EcoRI and XhoI to obtain TMP-L4-TMP and recovered fragment by Gel Extraction Kit.

Construction of plasmid containing ss-HSA-L6-H-L7-TMP-L4-TMP: Plasmid pcDNA3.1-fip-HSA was digested with restriction enzyme HindIII and XhoI and recovered fragment by Gel Extraction Kit to obtain vector pcDNA3.1. Mixed the three recovered fragment of ss-HSA (pre3), HSA (Post3)-L6-H-L7 and TMP-L4-TMP, with the recovered vector above by a Mole ratio of 5:5:5:1. 5U T4 ligase was added in this reaction system, 16 °C for 4 hrs. The ligation product was transformed into Escherichia coli DH5α (Tiangen) by the method of CaCl₂ hot shock. Transformed bacteria was spread on the LB plate which contains ampicillin. Clones were picked from the plate and inoculated in 1 mL LB fluid medium, cultured at 220 rpm, 37 °C for 6-8 hrs. PCR was then used to screen out positive colonies. Plasmid DNA was further isolated and following cut by restriction enzymes. The correct clone will be identified by sequence to detect whether there is any mutant in the interest gene. The desired clone 8 which contains the interest gene of ss-HSA-L6-H-L7-TMP-L4-TMP was finally obtained. The sequence of desired clone 8 is shown in Seq ID No: 41, and amino acid sequence is shown in Seq ID No: 42.

### Example 2 The expression of dimerized TMP-TMP-HSA fusion protein in mammalian cells

A kit for extracting a large number of plasmids;
L-glutamine, Hypoxanthine sodium and Thymine supplement reagent (HT) (Invitrogen)
T75 flask (NUNC, Denmark)
2-L WAVE cell-bag (GE Healthcare, America)
Chinese hamster ovary suspension cell (CHO-S) was from Invitrogen, America. The cell line is established from the ovary of an adult Chinese hamster and a subclone of (CHO-K1). Parental CHO-S was used for transfection. CHO-S cell is adapted to serum-free suspension growth, FreeStyleTM CHO serum-free expression medium (Invitrogen) supplemented with 8 mM L-glutamine and 2 mM Hypoxanthine sodium and Thymine supplement reagent (HT), which can meet the need of keeping CHO-S cell in a good growth status and the higher level of transient expression fusion protein products.

25 kDa linearized PEI transfection reagent (ploySciences, America): PEI transfection reagent was prepared by double distilled water into 1 mg/mL of stock solution, adjusted the pH value to 7. 0 with 1M HCl, filtered through a 0. 22 µm sterile filter, stored at 4 °C before use.

### 1. Transient transfection in CHO-S WAVE Bioreactor on large scale

Removed two cryovials of CHO cells (over 5×10⁶ viable cells/mL), supplemented each with 15 mL fresh FreeStyleTM CHO serum-free expression medium to recovery the cells in two T75 flasks, respectively. Static cultured at 37 °C, 5%CO₂ (v/v) in incubator. Till it reached a density of 2-3×10⁶ viable cells/mL, transferred them into WAVE cell-bag with 470 mL fresh FreeStyleTM CHO serum-free expression medium, used WAVE Bioreactor continued to culture these cells on WAVE Bioreactor. The rocking angle and speed of WAVE reactor were set to 7.5° and 13.5 rpm, temperature was set to 37 °C, CO₂ gas flow was set to 10 Lpm. When cell density reached at 1×10⁶ cells/mL and cell vitality over 95% in cell-bag, 500 mL fresh complete medium was supplemented for transfection on the next day. For clone No. 1 plasmid extracted by maxiprep kit (Qiagen company, Shanghai) was used for transfection. The ratio of plasmid DNA/PEI was kept at 1:3, 1mg plasmid DNA and 3 mg PEI per 1L cell culture medium. 5% v / v (50 mL) sterile PBS was mixed with plasmid DNA, PEI, for transfection, after incubated at room temperature for 10 min, pumped them into the WAVE cell-bag, and cells were continuously cultured in a final 1L, the cell viability was detected everyday, when the cell viability decreased to 50% or at 7th days after transfection, the cell cultures in WAVE bag were harvested, then centrifuged at 4 °C, 10000 rpm for 10 minutes to obtain a cell transient expression supernatant which contained the desired fusion protein.

Clones of No. 2-8 clone was obtained that used the same method as Example 1 to transiently transfect CHO-S cell line, respectively. Cell transient expression supernatants that contained desired fusion protein of TMP-L2-TMP-L5-H-fip-HSA, TMP-L2-TMP-L5-H-fip-HSA, TMP-L2-TMP-L5-H-fip-HSA, HSA- L6-H-L7-TMP-L1-TMP, HSA-L6-H-L7-TMP-L2-TMP, HSA-L6-H-L7-TMP-L3-TMP, HSA-L6-H-L7 -TMP-L4-TMP then were harvested, respectively.

### Example 3 The purification of dimerized TMP-TMP-HSA fusion protein expressed in mammalian cells of CHO

For transient transfection CHO expression supernatants from clone No.1-4, the desired fusion proteins contained His-tag Ni Sepharose FF was used to perform affinity chromatography. After desired fusion proteins were captured, then Sephacryl S-200 molecular sieve technique was used to further isolate dimer fusion proteins and monomer fusion proteins from each other. For clone No. 5-8 without His-tag, Capto blue was used to perform affinity chromatography for capturing fusion protein containing HSA, Sephacryl S-200 molecular sieve technique was used to isolate dimerized fusion protein and fusion monomers from each other, similarly. The purified desired fusion proteins of clone No. 1-8 CHO were identified by non-reduced SDS-PAGE, The results showed that the molecular weight of dimerized fusion protein was about 150 kDa, the molecular weight of monomer fusion protein was about 75 kDa, the monomer ratio was less than 10% totally.

### Example 4 The construction and screening of dimerized TMP-TMP-HSA fusion protein in yeast expression vector

### 1. Construction of clones containing TMP-L1-TMP-L5-H-fip-HSA

Yeast expression vector pPinka-HC (Invitrogen) was used as a template, P12 (cccgctttttggatgatt), P13 (gacttccggagccgccaccggccctagcagccagccactgcc
gcagtgtggggccttcgatccttttctcgagagatacc) were used as primers to amplify α-factor-TMP-L1 by PCR. The interested gene sequence is shown as Seq ID No: 43, amino acid sequence is shown as Seq ID No: 44.

The plasmid pcDNA3. 1-TMP-L1-TMP-L5-H-fip-HSA from clone No.1 was isolated and then was used as a template, P14 (gaaaccactctagagaagtgctgtg), P15 (cggggtacctcagtggtggtggtggtggtgtaagcctaaggcagcttgacttgcagc) were used as primers to amplify HSA (post4)-His-tag by PCR. The interested gene sequence is shown as Seq ID No: 45, amino acid sequence is shown as Seq ID No: 46.

In this PCR , the template DNA is 250 ng, primers is 100 pM, polymerase is EasyA Polymerase (Stratagene, USA), reaction volume is 50 µL: (The program of PCR was set as 94 °C, 3 min; 94 °C, 30 sec, 55 °C, 30 sec, 72 °C, 2min for 30 cycles).

5 µg pcDNA3.1-TMP-L1-TMP-L5-H-fip-HSA plasmid vector was cut sequently by BspEI and KpnI (TaKaRa) overnight at 37 °C, TMP-L5-H-fip-HSA (pre4) was obtained. The interested gene sequence is shown as Seq ID No: 47, amino acid sequence is shown as Seq ID No: 48.

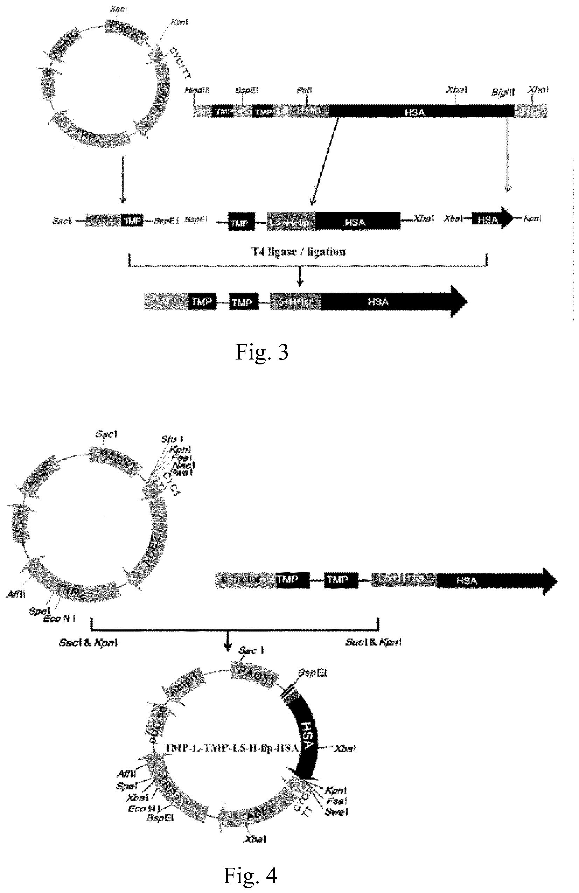

Similarly, 5 µg pPinkα-HC vector was cut by SacI and KpnI (Invitrogen) to prepare pPinkα-HC vector fragment (6902bp). 5µg α-factor-TMP-L1 was prepared after sequently digestion by BspEI, SacI, and gel extraction. 5 µg HSA (post4) fragment was prepared after sequently digestion by KpnI, XbaI, and gel extraction (Figure 3).

After ligation of the three fragments prepared above, human serum albumin-thrombopoietin fusion gene fragment was obtained, then it was inserted into the yeast expression vector pPinkα-HC to finally construct recombinant plasmid pPinkα-TMP-L1-TMP-L5-H-fip-HSA for further expression of desired fusion protein in Pichia pastoris (Figure 3, 4).

The ligation reaction system is shown below:
pPinkα-HCSacI/KpnI 1.5 µL
TMP-L5-H-fig-HSA (pre4) 6 µL
α-factor-TMP-L1 7 µL
HSA(Post4)-His 7 µL
10×T4 ligase buffer 2.5 µL
T4 DNA ligase 1 µL

Ligation reaction was kept at 16 °C overnight. 4 µL ligation product and 50 µL Escherichia coli TOP10 competent cell (Tiangen) were mixed then, transferred into 0.1 cm eletroporation cuvette, for electroporation by a shock at 1.8 KV, 3.4 ms, 1 mL LB medium was added rapidly then mixed, and transferred into a 1.5 mL centrifuge tube, maintained at 37 °C, in a shaker 150rpm for 1 hr, bacterium solution was sampled spread on LB (Amp+) plate, and kept in an incubator at 37 °C overnight.

Clones were picked up one by one from the above LB (Amp+) plate, and inoculated into LB liquid medium, respectively, cultured in a shaker at 37 °C, 220 rpm for 6-8 hrs. Following plasmid DNA was extracted from each these clones and then identified by PCR. Plasmid DNA was cut by double restriction enzymes for further screening to obtain positive clones, and was further extracted from positive clones. Finally, a complete correct clone has been validated by sequencing in Invitrogen. pPinkα-TMP-L1-TMP-L5-H-fip-HSA plasmid has been obtained an marked as clone No. 9.

### 2. Construction of clones containing TMP-L2-TMP-L5-H-fip-HSA:

Yeast expression vector pPinka-HC (Invitrogen) was used as a template, P12 (cccgctttttggatgatt), P16 (gacttccggaggccctagcagccagccactgccgcagtgtggggccttcgatccttttctcgagagatacc) were used as primers to amplify α-factor-TMP-L2 by PCR.

The plasmid pcDNA3. 1-TMP-L1-TMP-L5-H-fip-HSA from clone No.1 was isolated and then was used as a template, P14 (gaaaccactctagagaagtgctgtg), P15 (cggggtacctcagtggtggtggtggtggtgtaagcctaaggcagcttgacttgcagc) were used as primers to amplify HSA (post4)-His-tag by PCR. The interested gene sequence is shown as Seq ID No: 45, amino acid sequence is shown as Seq ID No: 46.

In this PCR, the template DNA is 250 ng, primers is 100 pM, polymerase is EasyA Polymerase (Stratagene), reaction volume is 50 µL: (The program of PCR was set as 94 °C, 3 min; 94 °C, 30 sec, 55 °C, 30 sec, 72 °C, 2 min for 30 cycles).

5 µg pcDNA3.1-TMP-L1-TMP-L5-H-fip-HSA plasmid vector was cut sequently by BspEI and KpnI (TaKaRa) overnight at 37 °C, TMP-L5-H-fip-HSA (pre4) was obtained. The interested gene sequence is shown as Seq ID No: 47, amino acid sequence is shown as Seq ID No: 48.

Similarly, 5 µg pPinka-HC vector was cut by SacI and KpnI (Invitrogen) to prepare pPinkα-HC vector fragment (6902bp). 5 µg α-factor-TMP-L2 was prepared after sequently digestion by BspEI, SacI, and gel extraction. 5 µg HSA (post4) fragment was prepared after sequently digestion by KpnI, XbaI, and gel extraction (Figure 3).

After ligation of the three fragments prepared above, human serum albumin-thrombopoietin fusion gene fragment was obtained, then it was inserted into the yeast expression vector pPinka-HC to finally construct recombinant plasmid pPinkα-TMP-L2-TMP-L5-H-fip-HSA for further expression of desired fusion protein in Pichia pastoris (Figure 3, 4).

The ligation reaction system is shown below:
pPinkα-HCSacI/KpnI 1.5 µL
TMP-L5-H-fig-HSA (pre4) 6 µL
α-factor-TMP-L2 7 µL
HSA(Post4)-His 7 µL 10×T4 ligase buffer 2.5µL
T4 DNA ligase 1 µL

Ligation reaction was kept at 16 °C overnight.4 µL ligation product and 50 µL Escherichia coli TOP10 competent cell (Tiangen) were mixed then, transferred into 0.1 cm eletroporation cuvette, for electroporation by one shock at 1.8 KV, 3.4 ms, 1 mL LB medium was added rapidly then mixed, and transferred into a 1.5 mL centrifuge tube, maintained at 37 °C, in a shaker 150 rpm for 1 hr, bacterium solution was sampled spread on LB (Amp+) plate, and kept in an incubator at 37 °C overnight.

Clones were picked up one by one from the above LB (Amp+) plate, and inoculated into LB liquid medium, respectively, cultured in a shaker at 37 °C, 220 rpm for 6-8 hrs. Following plasmid DNA was extracted from each these clones and then identified by PCR. Plasmid DNA was cut by double restriction enzymes for further screening to obtain positive clones, and was further extracted from positive clones. Finally, a complete correct clone has been validated by sequencing in Invitrogen. pPinkα-TMP-L2-TMP-L5-H-fip-HSA plasmid has been obtained an marked as clone No. 10.

### 3. Construction of clones containing TMP-L3-TMP-L5-H-fip-HSA

Yeast expression vector pPinkα-HC (Invitrogen) was used as a template, P12 (cccgctttttggatgatt), P17 (gacttccggaggggccaccggccctagcagccagccactg
ccgcagtgtggggccttcgatccttttctcgagagatacc) were used as primers to amplify α-factor-TMP-L3 by PCR.

The plasmid pcDNA3. 1-TMP-L1-TMP-L5-H-fip-HSA from clone No.1 was isolated and then was used as a template, P14 (gaaaccactctagagaagtgctgtg), P15 (cggggtacctcagtggtggtggtggtggtgtaagcctaaggcagcttgacttgcagc) were used as primers to amplify HSA (post4)-His-tag by PCR. The interested gene sequence is shown as Seq ID No: 45, amino acid sequence is shown as Seq ID No: 46.

In this PCR , the template DNA is 250 ng, primers is 100 pM, polymerase is EasyA Polymerase (Stratagene), reaction volume is 50 µL: (The program of PCR was set as 94 °C, 3 min; 94 °C, 30sec, 55 °C, 30 sec, 72 °C, 2 min for 30 cycles).

5µg pcDNA3. 1-TMP-L1-TMP-L5-H-fip-HSA plasmid vector was cut sequently by BspEI and KpnI (TaKaRa) overnight at 37°C, TMP-L5-H-fip-HSA (pre4) was obtained. The interests gene sequence is shown as Seq ID No: 47, amino acid sequence is shown as Seq ID No: 48.

Similarly, 5 µg pPinkα-HC vector was cut by SacI and KpnI (Invitrogen) to prepare pPinkα-HC vector fragment (6902bp). 5µg α-factor-TMP-L3 was prepared after sequently digestion by BspEI, SacI, and gel extraction. 5 µg HSA (post4) fragment was prepared after sequently digestion by KpnI, XbaI, and gel extraction (Figure 3).

After ligation of the three fragments prepared above, human serum albumin-thrombopoietin fusion gene fragment was obtained, then it was inserted into the yeast expression vector pPinkα-HC to finally construct recombinant plasmid pPinkα-TMP-L3-TMP-L5-H-fip-HSA for further expression of desired fusion protein in Pichia pastoris (Figure 3, 4).

The ligation reaction system is shown below:
pPinkα-HCSacI/KpnI 1.5 µL
TMP-L5-H-fig-HSA (pre4) 6 µL
α-factor-TMP-L3 7 µL
HSA(Post4)-His 7 µL
10×T4 ligase buffer 2.5µL
T4 DNA ligase 1 µL

Ligation reaction was kept at 16 °C overnight.4 µL ligation product and 50 µL Escherichia coli TOP10 competent cell (Tiangen) were mixed then, transferred into 0. 1 cm eletroporation cuvette, for electroporation by one shock at 1.8 KV, 3.4 ms, 1 mL LB medium was added rapidly then mixed, and transferred into a 1.5 mL centrifuge tube, maintained at 37 °C, in a shaker 150rpm for 1 hr, bacterium solution was sampled spread on LB (Amp+) plate, and kept in an incubator at 37 °C overnight.

Clones were picked up one by one from the above LB (Amp+) plate, and inoculated into LB liquid medium, respectively, cultured in a shaker at 37 °C, 220 rpm for 6-8 hrs. Following plasmid DNA was extracted from each these clones and then identified by PCR. Plasmid DNA was cut by double restriction enzymes for further screening to obtain positive clones, and was further extracted from positive clones. Finally, a complete correct clone has been validated by sequencing in invitrogen. pPinkα-TMP-L3-TMP-L5-H-fip-HSA plasmid has been obtained an marked as clone No. 11.

### 4. Construction of clones containing TMP-L4-TMP-L5-H-fip-HSA:

Yeast expression vector pPinkα-HC (Invitrogen) was used as a template, P12 (cccgctttttggatgatt), P18 (gacttccggagccgccaccgctgccgccaccgccggccctagcagccagccactgccgcagtgtggggccttcgat ccttttctcgagagatacc) were used as primers to amplify α-factor-TMP-L4 by PCR.

The plasmid pcDNA3. 1-TMP-L1-TMP-L5-H-fip-HSA from clone No.1 was isolated and then was used as a template, P14 (gaaaccactctagagaagtgctgtg), P15 (cggggtacctcagtggtggtggtggtggtgtaagcctaaggcagcttgacttgcagc)were used as primers to amplify HSA (post4)-His-tag by PCR. The intersted gene sequence is shown as Seq ID No: 45, amino acid sequence is shown as Seq ID No: 46.

In this PCR, the template DNA is 250 ng, primers is 100 pM, polymerase is EasyA Polymerase (Stratagene), reaction volume is 50µL: (The program of PCR was set as 94 °C, 3 min; 94 °C, 30 sec, 55 °C, 30 sec, 72 °C, 2 min for 30 cycles).

5 µg pcDNA3. 1-TMP-L1-TMP-L5-H-fip-HSA plasmid vector was cut sequently by BspEI and KpnI (TaKaRa) overnight at 37 °C, TMP-L5-H-fip-HSA (pre4) was obtained. The interests gene sequence is shown as Seq ID No: 47, amino acid sequence is shown as Seq ID No: 48.

Similarly, 5 µg pPinka-HC vector was cut by SacI and KpnI (Invitrogen) to prepare pPinkα-HC vector fragment (6902bp). 5 µg α-factor-TMP-L4 was prepared after sequently digestion by BspEI, Sacl, and gel extraction. 5µg HSA (post4) fragment was prepared after sequently digestion by KpnI, XbaI, and gel extraction (Figure 3).

After ligation of the three fragments prepared above, human serum albumin-thrombopoietin fusion gene fragment was obtained, then it was inserted into the yeast expression vector pPinkα-HC to finally construct recombinant plasmid pPinkα-TMP-L4-TMP-L5-H-fip-HSA for further expression of desired fusion protein in Pichia pastoris (Figure 3, 4).

The ligation reaction system is shown below:
pPinkα-HCSacI/KpnI 1.5 µL
TMP-L5-H-fig-HSA (pre4) 6 µL
α-factor-TMP-L4 7 µL
HSA(Post4)-His 7 µL
10×T4 ligase buffer 2.5 µL
T4 DNA ligase 1 µL

Ligation reaction was kept at 16 °C overnight.4 µL ligation product and 50 µL Escherichia coli TOP10 competent cell (Tiangen) were mixed then, transferred into 0. 1 cm eletroporation cuvette, for electroporation by one shock at 1. 8 KV, 3.4 ms, 1 mL LB medium was added rapidly then mixed, and transferred into a 1.5 mL centrifuge tube, maintained at 37 °C in a shaker 150 rpm for 1 hr, bacterium solution was sampled spread on LB (Amp+) plate .and kept in an incubator at 37 °C overnight.

Clones were picked up one by one from the above LB (Amp+) plate, and inoculated into LB liquid medium, respectively, cultured in a shaker at 37 °C, 220 rpm for 6-8 hrs. Following plasmid DNA was extracted from each these clones and then identified by PCR. Plasmid DNA was cut by double restriction enzymes for further screening to obtain positive clones, and was further extracted from positive clones. Finally, a complete correct clone has been validated by sequencing in Invitrogen. pPinkα-TMP-L4-TMP-L5-H-fip-HSA plasmid has been obtained an marked as clone No. 12.

### 5. Construction of vectors containing HSA-L6-H-L7-TMP-L1-TMP:

Fully synthesized the complete gene of complete gene TMP-L1-TMP fragment by TaKaRa, ligation of the prepared fragment to pMD19-T simple vector. The plasmid was inoculated into Escherichia coli TOP10 competent cell, and positive clones were picked up. Shaking cultured the bacteria, isolated plasmid to obtain pMD19-T simple-TMP-L1-TMP.

The prepared plasmid was cut by KpnI and EcoRI (TaKaRa) overnight at 37°C to obtain TMP-L1-TMP.

The plasmid vector pcDNA3. 1-HSA-L6-H-L7-TMP-L1-TMP was used as a template, P10 (GAAGGATATCGATGCACACAAGAGTGAGGT), P11 (GAAGGAATTCGTGGTGGTGGTGGTGGTGGCAGGGAGGGCAGGTGTGGGTCTT G) was used as primers to amplify HSA-L6-H-L7 by PCR.

In this PCR, the template DNA is 250 ng, primers is 100 pM, polymerase is EasyA Polymerase (Stratagene). Reaction volume is 50 µL: (The program of PCR was set as 94 °C, 3 min; 94 °C, 30 sec, 55 °C, 30 sec, 72 °C, 2 min for 30 cycles).

Prepared HSA-H by PCR, then plasmid was cut by EcoRI and EcoRV (TaKaRa) overnight at 37 °C to obtained HSA-L6-H-L7.

Similarly, 5 µg pPinkα-HC vector (Invitrogen) was cut by StuI and KpnI (TaKaRa) to prepare for pPinkα-HC fragment (7890bp).

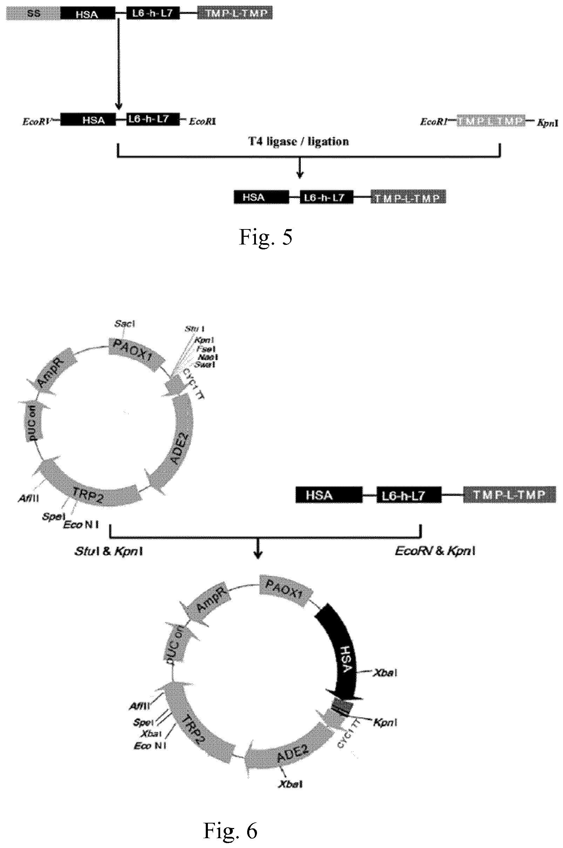

After ligation of the two fragments prepared above, a Human Serum albumin-thrombopoietin fusion gene fragment was obtained, then it was inserted into the yeast expression vector pPinkα-HC to finally construct recombinant plasmid pPinkα-HSA-L6-H-L7-TMP-L1-TMP for further expression of desired fusion protein in Pichia pastoris (Figure 5, 6).

The ligation reaction system is shown below:
pPinkα-HCSacI/KpnI 2 µL
TMP-L1-TMP (KpnI/EcoRI) 5 µL
HSA-L6-H-L7(EcoRI/EcoRV) 3 µL
10×T4 ligase buffer 1.5 µL
T4 DNA ligase 1 µL
ddH2O 2.5 µL

Ligation reaction was kept at 16 °C overnight. 4 µL ligation product and 50 µL Escherichia coli TOP10 competent cell (Tiangen) were mixed then transferred into 0. 1 cm eletroporation cuvette, for electroporation by one shock at 1.8 KV, 3.4 ms, 1 mL LB medium was added rapidly then mixed, and transferred into a 1.5 mL centrifuge tube, maintained at 37 °C in a shaker, 150 rpm for 1 hr, bacterium solution was sampled spread on LB (Amp+) plate, and kept in an incubator at 37 °C overnight.

Clones were picked up one by one from the above LB (Amp+) plate, and inoculated into LB liquid medium, respectively, cultured in a shaker at 37 °C, 220 rpm, for 6-8 hrs. Following plasmid DNA was extracted from these clones and then identified by PCR. Plasmid DNA was cut by double reaction enzymes for further screening to obtain positive clones, and was further extracted from positive clones. Finally, a complete correct clone has been validated by sequencing in Invitrogen pPinkα-HSA-L6-H-L7-TMP-L1-TMP plasmid has been clone No. 13.

### 6. Construction of vectors containing HSA-L6-H-L7-TMP-L2-TMP:

Fully synthesized complete gene of TMP-L2-TMP fragment by TaKaRa, ligation of the prepared fragment to pMD19-T simple vector. The plasmid was inoculated into Escherichia coli TOP10 competent cell, and positive clones were picked up. Shaking cultured the bacteria, isolated plasmid to obtain pMD19-T simple-TMP-L2-TMP.

The prepared plasmid was cut by KpnI and EcoRI (TaKaRa) overnight at 37 °C to obtain TMP-L2-TMP.

The plasmid vector pcDNA3.1-HSA-L6-H-L7-TMP-L1-TMP was used as a template, P10 (GAAGGATATCGATGCACACAAGAGTGAGGT), P11 (GAAGGAATTCGTGGTGGTGGTGGTGGTGGCAGGGAGGGCAGGTGTGGGTCTT G) was used as primers to amplify HSA-L6-H-L7 by PCR.

In this PCR, the template DNA is 250 ng, primers is 100 pM, polymerase is EasyA Polymerase (Stratagene). Reaction volume is 50 µL: (The program of PCR was set as 94 °C, 3 min; 94 °C, 30 sec, 55 °C, 30 sec, 72 °C, 2min for 30 cycles).

Amplified HSA-H by PCR, then plasmid was cut by EcoRI and EcoRV (TaKaRa) overnight at 37 °C to prepared HSA-L6-H-L7.

Similarly, 5 µg pPinkα-HC vector (Invitrogen) was cut by StuI and KpnI (TaKaRa) to prepare for pPinkα-HC fragment (7890bp).

After ligation of the two fragments prepared above, Human Serum albumin-thrombopoietin fusion gene fragment was obtained, then it was inserted into the yeast expression vector pPinka-HC to finally construct recombinant plasmid pPinkα-HSA-L6-H-L7-TMP-L2-TMP for further expression of desired fusion protein in Pichia pastoris (Figure 5, 6).

The ligation reaction system is shown below:
pPinkα-HCSacI/KpnI 2 µL
TMP-L2-TMP (Kpnl/ EcoRI) 5 µL
HSA-L6-H-L7(EcoRI/EcoRV) 3 µL
10×T4 ligase buffer 1.5 µL
T4 DNA ligase 1 µL
ddH2O 2.5 µL

Ligation reaction was kept at 16 °C overnight. 4 µL ligation product and 50 µL Escherichia coli TOP10 competent cell (Tiangen) were mixed then transferred into 0. 1 cm eletroporation cuvette, for electroporation by one shock at 1. 8 KV, 3.4 ms, 1 mL LB medium was added rapidly then mixed, and transferred into a 1.5 mL centrifuge tube, maintained at 37 °C in a shaker, 150 rpm for 1 hr, bacterium solution was sampled spread on LB (Amp+) plate, and kept in an incubator at 37 °C overnight.

Clones were picked up one by one from the above LB (Amp+) plate, and inoculated into LB liquid medium, respectively, cultured in a shaker at 37 °C, 220 rpm, for 6-8 hrs. Following plasmid DNA was extracted from these clones and then identified by PCR. Plasmid DNA was further extracted from a positive clone, and was cut by double reaction enzymes for further screening a positive clone. Finally, a complete correct clone has been validated by sequencing in Invitrogen pPinkα-HSA-L6-H-L7-TMP-L2-TMP plasmid has been clone No. 14.

### 7. Construction of vectors containing HSA-L6-H-L7-TMP-L3-TMP:

Fully synthesized the complete gene of TMP-L3-TMP fragment by TaKaRa, ligation of the prepared fragment to pMD19-T simple vector. The plasmid was inoculated into Escherichia coli TOP10 competent cell, and positive clones were picked up. Shaking cultured the bacteria, isolated plasmid to obtain pMD19-T simple-TMP-L3-TMP.

The prepared plasmid was cut by KpnI and EcoRI (TaKaRa) overnight at 37°C to obtain TMP-L3-TMP.

The plasmid vector pcDNA3. 1-HSA-L6-H-L7-TMP-L1-TMP was used as a template, P10 (GAAGGATATCGATGCACACAAGAGTGAGGT), P11 (GAAGGAATTCGTGGTGGTGGTGGTGGTGGCAGGGAGGGCAGGTGTGGGTCTT G) was used as primers to amplify HSA-L6-H-L7 by PCR.

In this PCR, the template DNA is 250 ng, primers is 100 pM, polymerase is EasyA Polymerase (Stratagene). Reaction volume is 50 µL: (The program of PCR was set as 94 °C, 3 min; 94 °C, 30 sec, 55 °C, 30 sec, 72 °C, 2min for 30 cycles).

Amplified HSA-H by PCR, then plasmid was cut by EcoRI and EcoRV (TaKaRa) overnight at 37 °C to prepared HSA-L6-H-L7.

Similarly, 5 µg pPinkα-HC vector (Invitrogen) was cut by StuI and KpnI (TaKaRa) to prepare for pPinkα-HC fragment (7890bp).

After ligation of the two fragments prepared above, Human Serum albumin-thrombopoietin fusion gene fragment was obtained, then it was inserted into the yeast expression vector pPinkα-HC to finally construct recombinant plasmid pPinkα-HSA-L6-H-L7-TMP-L3-TMP for further expression of desired fusion protein in Pichia pastoris (Figures 5 and 6).

The ligation reaction system is shown below:
pPinkα-HCSacI/KpnI 2 µL
TMP-L3-TMP (KpnI/ EcoRI) 5 µL
HSA-L6-H-L7(EcoRI/ EcoRV) 3 µL
10×T4 ligase buffer 1.5 µL
T4 DNA ligase 1 µL
ddH₂O 2.5 µL

Ligation reaction was kept at 16 °C overnight. 4 µL ligation product and 50 µL Escherichia coli TOP10 competent cell (Tiangen) were mixed then transferred into 0. 1 cm eletroporation cuvette, for electroporation by one shock at 1. 8KV, 3.4 ms, 1 mL LB medium was added rapidly then mixed, and transferred into a 1. 5 mL centrifuge tube, maintained at 37 °C in a shaker, 150 rpm for 1 hr, bacterium solution was sampled spread on LB (Amp+) plate, and kept in an incubator at 37 °C overnight.

Clones were picked up one by one from the above LB (Amp+) plate, and inoculated into LB liquid medium, respectively, cultured in a shaker at 37 °C, 220 rpm, for 6-8 hrs. Following plasmid DNA was extracted from these clones and then identified by PCR. Plasmid DNA was further extracted from a positive clone, and was cut by double reaction enzymes for further screening a positive clone. Finally, a complete correct clone has been validated by sequencing in Invitrogen pPinkα-HSA-L6-H-L7-TMP-L3-TMP plasmid has been clone No. 15.

### 8. Construction of vectors containing HSA-L6-H-L7-TMP-L4-TMP:

Fully synthesized the complete gene of complete gene TMP-L4-TMP fragment by TaKaRa, ligation of the prepared fragment to pMD19-T simple vector. The plasmid was inoculated into Escherichia coli TOP10 competent cell, and positive clones were picked up. Shaking cultured the bacteria, isolated plasmid to obtain pMD19-T simple-TMP-L4-TMP.

The prepared plasmid was cut by KpnI and EcoRI (TaKaRa) overnight at 37 °C to obtain TMP-L4-TMP.

The plasmid vector pcDNA3.1-HSA-L6-H-L7-TMP-L1-TMP was used as a template, P10 (GAAGGATATCGATGCACACAAGAGTGAGGT), P11 (GAAGGAATTCGTGGTGGTGGTGGTGGTGGCAGGGAGGGCAGGTGTGGGTCTT G) was used as primers, to amplify HSA-L6-H-L7 by PCR.

In this PCR, the template DNA is 250 ng, primers is 100 pM, polymerase is EasyA Polymerase (Stratagene). Reaction volume is 50 µL: (The program of PCR was set as 94 °C, 3 min; 94 °C, 30 sec, 55 °C, 30 sec, 72 °C, 2 min for 30 cycles).

Amplified HSA-H by PCR, then plasmid was cut by EcoRI and EcoRV (TaKaRa) overnight at 37 °C to prepared HSA-L6-H-L7.

Similarly, 5 µg pPinkα-HC vector (Invitrogen) was cut by StuI and KpnI (TaKaRa) to prepare for pPinkα-HC fragment (7890bp).

After ligation of the two fragments prepared above, Human Serum albumin-thrombopoietin fusion gene fragment was obtained, then it was inserted into the yeast expression vector pPinkα-HC to finally construct recombinant plasmid pPinkα-HSA-L6-H-L7-TMP-L4-TMP for further expression of desired fusion protein in Pichia pastoris (Figure 5, 6).

The ligation reaction system is shown below:
pPinkα-HC SacI/KpnI 2 µL
TMP-L4-TMP (KpnI/ EcoRI) 5 µL
HSA-L6-H-L7(EcoRI/ EcoRV) 3 µL
10×T4 ligase buffer 1.5µL
T4 DNA ligase 1 µL
ddH2O 2.5 µL

Ligation reaction was kept at 16 °C overnight. 4 µL ligation product and 50 µL Escherichia coli TOP10 competent cell (Tiangen) were mixed then transferred into 0. 1 cm eletroporation cuvette, for electroporation by one shock at 1.8 KV, 3.4 ms, 1 mL LB medium was added rapidly then mixed, and transferred into a 1.5 mL centrifuge tube, maintained at 37 °C in a shaker, 150 rpm for 1 hr, bacterium solution was sampled spread on LB (Amp+) plate, and kept in an incubator at 37 °C overnight.

Clones were picked up one by one from the above LB (Amp+) plate, and inoculated into LB liquid medium, respectively, cultured in a shaker at 37 °C, 220 rpm, for 6-8 hrs. Following plasmid DNA was extracted from these clones and then identified by PCR. Plasmid DNA was further extracted from a positive clone, and was cut by double reaction enzymes for further screening a positive clone. Finally, a complete correct clone has been validated by sequencing in Invitrogen pPinkα-HSA-L6-H-L7-TMP-L4-TMP plasmid has been clone No. 16.

### Example 5 The transformation and screening of the strain expressed dimerized TMP-TMP-HSA fusion protein in Pichia pastoris recombinant strain

### 1. The transformation of dimerized TMP-TMP-HSA fusion protein engineered yeast

Expression vectors has been routinely screened as following: firstly Escherichia coli TOP10, which contained the plasmid of pPinkα-TMP-L1-TMP-L5-H-fip-HSA derived from Example 4, was inoculated into LB liquid medium, cultured in a shaker at 37 °C, 220 rpm for16hrs, pPinkα-TMP-L1-TMP-L5-H-fip-HSA recombinant plasmid was isolated by a maxiprep plasmid DNA kit, then linearized vector by AflII and transferred routinely it into Pichia pastoris strain by electroprotion method, spread on the plate and cultured for 3-7 days. 3-8 white clones were picked up and inoculated each clone into liquid BMGY medium, respectively. BMMY liquid medium for continuously cultured in a flask for 3-4 days, desired fusion protein expression was detected by SDS-PAGE. All protocols and ingredients of culture medium can be found details in a manual of PichiaPinkTM Expressioin System (Invitrogen). The specific electroportion methods for preparing expression vector is shown below:
1) Recombinant plasmid of pPinkα-TMP-L1-TMP-L5-H-fip-HSA was isolated, linearized plasmid purified and quantified in preparation.
2) Prepared competent yeast: spread PichiaPinkTM Expression Strains (Invitrogen) on YPD plate, cultured at 28 °C for 3-5 days. Picked up a clone and inoculated into 10 mL YPD medium, cultured at 28 °C, 300 rpm for 1-2 days, removed 1 mL cultured and inoculated into 100 mL YPD medium, cultured at the same condition until OD600 reached 1.3-1.5. Transferred the cultures into a 250 mL centrifuge tube, centrifuged at 4 °C, 1500×g, for 5 min, discarded the supernatant, resuspended the pellet with ice cold water in 250 mL centrifuge tube. Repeated the previous centrifugation step, resuspended the pellet with ice cold water in a 50 mL centrifuge tube. Repeated the previous centrifugation step, resuspended the pellet with 1M ice cold sorbitol in a 10 mL centrifuge tube. Repeated the previous centrifugation step, resuspended the pellet in 300 µL of 1M ice cold sorbito. Kept the resultant competent yeast cell on ice, these cells should be used within the day they were prepared.
3) 10 µg linearized DNA pPinkα-TMP-L1-TMP-L5-H-fip-HSA plasmid DNA was mixed with competent yeast cells, and transferred into the 0.2 cm electroporation cuvette. Sit on ice for 5 min, then electroporatd by a shock. Immediately added 1 mL YPDS, kept in an incubator at 28 °C for 6hrs. The resultant yeast cells were spread on PAD plate, cultured at 28 °C for 3-10 days. When yeast clones appeared, picked up white colons for streaking inoculation on PAD plate.

Clones No. 2-8 are treated as above.

### 2. Shaking flask expression of desired fusion protein

1) The white clone of pPinkα yeast strain No. 1, which contains TMP-L1-TMP-L5-H-fip-HSA on streaking inoculation plate was picked up and transferred into 50 mL BMGY medium, cultured in a shaker at 28 °C, 300 rpm for 3 days.
2) Centrifuged the cultures and discarded supernatant, resuspended the pellets in10 mL BMMY medium, continuously cultured in a shaker at 28 °C, 300 rpm for 72-96 hrs.
3) 500 µL cultures were sampled every 12 hrs, and after that, added 500 µL 40 % methanol. Centrifuged the samples at 1500×g for 10 min, collected the supernatant and precipitation, saved at -80 °C for a later detection.
4) At the end of shaking flask, the cultures were harvested by centrifugation at 1500×g for 10 min, the supernatant and precipitation were collected and saved at -80 °C for a later detection.

Picked up the highly expression clones for desired fusion protein then obtained the No. 1 yeast recombinant strain containing TMP-L1-TMP-L5-H-fip-HSA. Use the same method treated No. 2-8 clones, respectively obtained the No. 2 pPinkα yeast recombinant strain that contains TMP-L2-TMP-L5-H-fip-HSA , the No. 3 pPinkα yeast recombinant strain that contains TMP-L3-TMP-L5-H-fip-HSA, the No. 4 pPinkα yeast recombinant strain that contains TMP-L4-TMP-L5-H-fip-HSA, the No. 5 pPinkα yeast recombinant strain that contains HSA-L6-H-L7-TMP-L1-TMP, the No. 6 pPinkα yeast recombinant strain that contains HSA-L6-H-L7-TMP-L2-TMP, the No. 7 pPinkα yeast recombinant strain that contains HSA-L6-H-L7-TMP-L3-TMP, the No. 8 pPinkα yeast recombinant strain that contains HSA-L6-H-L7-TMP-L4-TMP.

### Example 6 Purification of desired product which in the supernatant of Engineering Pichia cultures

The high-level expression engineering strains which have been screened from example 5 was used for further expressing desired recombinant fusion protein in 1000 mL shake flasks following above protocol. Samples was induced by methanol for 96 hrs, then centrifuged at 7500 rpm for 5 min and collected the supernatant. For resultant supernatants of No. 1-4 yeast strain, Ni Sepharose FF affinity chromatography was used to collect desired fusion protein with His-tag, then polished by Sephacryl S-200 molecular sieve chromatography to collect dimerized fusion proteins and monomer fusion proteins, respectively. For resultant supernatants of No. 5-8 yeast strain, there were no His-tag in the desired fusion protein, Capto blue column affinity chromatography was chosen to firstly capture the desired fusion protein, and then polished by Sephacryl S-200 molecular sieve chromatography to collect dimerized fusion proteins and monomer fusion proteins, respectively. The desired purified fractions of No. 1-8 yeast strains were identified by SDS-PAGE, the molecular weight of dimerized fusion proteins is shown to be 150 kDa, and the monomer fusion proteins is shown to be 75 kDa (the percentage of monomer was less than 10%).

### Example 7 The bioactivity assay of the dimerized HSA-TMP-TMP fusion protein based on the receptor of mpl dependent manner at a cell level

Lipofectamine2000 (Invitrogen, America)
Opti-MEM minus serum (Invitrogen, America)
Dual GloTM Luciferase assay system (Promega, America)
Passive lysis of cell (Promega, America)
pAdvantage recombinant vector (Promega, America)
c-mpl-pcDNA3. 1 recombinant vector (provided kindly by Professor Xu Peilin, Sun Yat-sen University, China)
c-fos-pGL3 recombinant vector (provided kindly by professor Toshiio Ishikawa, Tokyo University, Japan), Renilla recombinant vector (Promega, America)
DMEM (Invitrogen, America)
Trypsin (Sangon)
Fetal bovine serum (Hyclone, America)
Mouse embryo fibroblasts cell line NIH-3T3, purchased from Gansu Animal Cell Engineering Technology Research Center, Lanzhou, China.
1. Cell cultured
NIH-3T3 were cultured in high glucose DMEM with 10% (v/v) fetal bovine serum, CO₂ 5% (v/v), at 37 °C.

### 2. Preparation of the transfection complexes

Lipofectamine 2000 and four vectors: c-mpl-pcDNA3.1 (pcDNA3.1 as a negative control), c-fos-pGL3, pAdvantage and Renilla, were co-transfected into NIH-3T3 cells, the ratio between vectors is 5:2:2:0. 1. Totally added 0. 8 µg of the four vectors at the above ratio into 50 µL Opti-MEM minus serum, mixed gently, and incubated for 5 min at room temperature. Additionally, added 2 µL Lipofetamine 2000 into another 50 µL Opti-MEM minus serum, mixed gently, and incubated for 5 minutes at room temperature. Then mixed Lipofectamine 2000 with the vector, incubated for 20 min at room temperature, and obtained the transfection complexes.

### 3. The luciferase assay of dimerized TMP-TMP-HSA fusion protein

Seeded the cells in 24 well-plate at a density of 3×10⁴ cells/well, and incubated for 24 h before transfection. After the incubation, discarded the medium in the plate, and wash once time using serum-free then added 400 µL Opti-MEM minus serum. Added the transfection complexes by dropwise, mixed gently, and then maintained the plates in CO₂ incubator, at 37 °C, 5% CO₂ (v/v).

6 h after transfection, discarded the Opti-MEM, and added complete medium with 10% (v/v) serum instead.24 h after transfection, replaced the complete medium with serum-free medium to starve the cells for 24 h. After that, treated the cells with rhTPO (positive drug) or tested samples for 6h in a CO₂ incubator, at 37 °C, 5% (v/v) CO₂. Discarded the medium, washed twice with PBS, and then added 150 µL passive lysis buffer, shook gently at 30 °C for 15 min. Pipetted the cells and mixed thoroughly, transferred 30 µL of cell lyse per well into the 96 well plate. Firstly, determined the activity of firefly luciferase: added 40 µL Dual -Glo Luciferase Reagent into each well in above 96 well plate, mixed thoroughly and avoided bubbles, incubated 10 min at room temperature, then measured the activity of firefly luciferase using a fluorescence detector device. Following, measured the activity of Renilla luciferase: added 75 µL Dual-Glo Stop&Glo Reagent into each well in the same 96well plate, mixed thoroughly and avoided bubbles, incubated 10 min at room temperature, then measured the activity of Renilla luciferase also using a fluorescence detector device. At last, calculated the luminescence density ratio between firefly and Renilla: luminescence density ratio = the density of firefly luminescence / the density of the Renilla luminescence

Results indicates that the relative luminescence ratio of all groups that transfected with pcDNA3.1 empty vector control is 1.0. Data of groups transfected with mpl-pcDNA3.1 were shown in table1. Table1 revealed that the tested samples of dimerized fusion protein expressed in CHO cells and yeast cells had a significant mpl-dependent activity, but monomer fusion protein had little such bioactivity. In this experiment, rhTPO positive drug and dimerized fusion protein tested samples expressed in CHO and yeast functioned on c-mpl receptor dependent.

**Table1 c-mpl receptor dependent activity assays of dimerized TMP-TMP-HSA fusion protein**

| Groups | | The relative fluorescence ratio |
|---|---|---|
| Control | | 1.00 ± 0.04 |
| Positive group treated with rhTPO | | 1.65 ± 0.08 |
| No. 1 CHO cell line | Monomer fusion protein | 1.01 ± 0.07 |
| | Dimer fusion protein | 1.68 ± 0.06 |
| No. 2 CHO cell line | Monomer fusion protein | 1.05 ± 0.09 |
| | Dimer fusion protein | 1.65 ± 0.03 |
| No. 3 CHO cell line | Monomer fusion protein | 1.01 ± 0.05 |
| | Dimer fusion protein | 1.67 ± 0.05 |
| No. 4 CHO cell line | Monomer fusion protein | 1.12 ± 0.07 |
| | Dimer fusion protein | 1.69 ± 0.08 |
| No. 5 CHO cell line | Monomer fusion protein | 1.04 ± 0.09 |
| | Dimer fusion protein | 1.60 ± 0.11 |
| No. 6 CHO cell line | Monomer fusion protein | 1.03 ± 0.06 |
| | Dimer fusion protein | 1.66 ± 0.03 |
| No. 7 CHO cell line | Monomer fusion protein | 1.10 ± 0.17 |
| | Dimer fusion protein | 1.78 ± 0.12 |
| No. 8 CHO cell line | Monomer fusion protein | 1.04 ± 0.03 |
| | Dimer fusion protein | 1.72 ± 0.09 |
| No. 1 yeast strain | Monomer fusion protein | 1.05 ± 0.04 |
| | Dimer fusion protein | 1.66 ± 0.08 |
| No. 2 yeast strain | Monomer fusion protein | 1.05 ± 0.06 |
| | Dimer fusion protein | 1.71 ± 0.11 |
| No. 3 yeast strain | Monomer fusion protein | 1.02 ± 0.05 |
| | Dimer fusion protein | 1.62 ± 0.07 |
| No. 4 yeast strain | Monomer fusion protein | 1.10 ± 0.03 |
| | Dimer fusion protein | 1.65 ± 0.08 |
| No. 5 yeast strain | Monomer fusion protein | 1.07 ± 0.08 |
| | Dimer fusion protein | 1.62 ± 0.06 |
| No. 6 yeast strain | Monomer fusion protein | 1.08 ± 0.06 |
| | Dimer fusion protein | 1.59 ± 0.04 |
| No. 7 yeast strain | Monomer fusion protein | 1.04 ± 0.03 |
| | Dimer fusion protein | 1.61 ± 0.05 |
| | | |
| No. 8 yeast strain | Monomer fusion protein | 1.08 ± 0.04 |
| | Dimer fusion protein | 1.62 ± 0.09 |

### Example 8 The activity assays of dimerized TMP-TMP-HSA fusion protein promoted blood platelet production in normal mice

10 Kunming mice were randomly assigned in each group with 5 males and 5 females, weighting 20 ± 1 g. The mice were subcutaneously injected the tested sample at head-and-neck daily for 4 consecutive days. At the start of the third day after dosed, blood samples were collected from tail vein every other day. The number of blood platelets is routinely counted. Table 2 is the average number of blood platelets for 4 days. It showed that the tested dimerized fusion proteins expressed either in CHO or in yeast significantly improved the number of blood platelet, but the monomer fusion proteins have hardly any effect on the number of blood platelet.

**Table 2 The activity assays of dimerized TMP-TMP-HSA fusion protein promoting the number of blood platelet productions in normal mice.**

| Groups | | The amount of blood platelet (×10⁹/mL) |
|---|---|---|
| Control group treated with Normal saline | | 1.32 |
| Positive group treated with rhTPO | | 1.89 |
| No. 1 CHO cell line | Monomer fusion protein | 1.32 |
| | Dimer fusion protein | 1.80 |
| No. 2 CHO cell line | Monomer fusion protein | 1.31 |
| | Dimer fusion protein | 1.81 |
| No. 3 CHO cell line | Monomer fusion protein | 1.33 |
| | Dimer fusion protein | 2.21 |
| No. 4 CHO cell line | Monomer fusion protein | 1.32 |
| | Dimer fusion protein | 2.50 |
| No. 5 CHO cell line | Monomer fusion protein | 1.34 |
| | Dimer fusion protein | 1.94 |
| No. 6 CHO cell line | Monomer fusion protein | 1.32 |
| | Dimer fusion protein | 1.80 |
| No. 7 CHO cell line | Monomer fusion protein | 1.33 |
| | Dimer fusion protein | 2.10 |
| No. 8 CHO cell line | Monomer fusion protein | 1.37 |
| | Dimer fusion protein | 1.80 |
| No. 1 yeast strain | Monomer fusion protein | 1.31 |
| | Dimer fusion protein | 1.89 |
| No. 2 yeast strain | Monomer fusion protein | 1.35 |
| | Dimer fusion protein | 2.77 |
| No. 3 yeast strain | Monomer fusion protein | 1.32 |
| | Dimer fusion protein | 1.89 |
| | | |
| No. 4 yeast strain | Monomer fusion protein | 1.36 |
| | Dimer fusion protein | 1.85 |
| No. 5 yeast strain | Monomer fusion protein | 1.30 |
| | Dimer fusion protein | 2.45 |
| No. 6 yeast strain | Monomer fusion protein | 1.31 |
| | Dimer fusion protein | 2.20 |
| No. 7 yeast strain | Monomer fusion protein | 1.32 |
| | Dimer fusion protein | 2.56 |
| No. 8 yeast strain | Monomer fusion protein | 1.35 |
| | Dimer fusion protein | 2.38 |

### Example 9 The effect of dimerized TMP-TMP-HSA fusion protein on idiopathic thrombocytopenia mice model

Blood was collected by a removing eyeball method in Kunming mice, EDTA-Na₂ was used as anticoagulant agent, centrifuged at 1500 rpm for 10 min, supernatants were collected, then centrifuged at 4000 rpm for 15 min to precipitate blood platelets, pellets were collected to obtain blood platelets. The concentration of blood platelets was adjusted to 1-2×10⁹/L. These blood platelets were mixed with equivalent volume of complete Freund's adjuvant or incomplete Freund's adjuvant (Sigma) and then emulsified as antigen. Rabbits were subcutaneously injected such antigen with complete Freund's adjuvant on hind paw, back and inguen for the first week. Animals received antigen with incomplete Freund's adjuvant by subcutaneous injection for next 4 consecutive weeks. 200 µL emulsified antigen was used in each injected point, totally 8 points per injection for each rabbit. Whole blood without anticoagulant agent added was collected from jugular vein for the 6th week. Rabbit anti-mouse platelet anti-serum (APS) was obtained after the whole blood by centrifugation at 4000 rpm for 15 min, discarded the pellets, and stored at -20 °C before use.

APS was thawed completely and then sit in 56 °C water-bath for 30 min. Each mouse received intraperitoneal injection of APS diluted with normal saline by 1:4 at dose of 100 µL / 20 g. All mice were injected APS every other day to maintain the animals on continuous reduction in blood platelets till the end of the experiment. Mice intraperitoneally injected normal saline at a dose of 100 µL / 20 g were considered as negative control groups. 10 Kunming mice were randomly assigned in each group with 5 males and 5 females, weighting 20 ± 1g. From the second day of mice received APS, the mice began to received daily injection with the tested sample at head-and-neck subcutaneously for 4 consecutive days. At the start of the second day after dosed the tested fusion proteins, blood samples were collected from tail vein every other day. The number of blood platelets was routinely counted. Table 3 is the average number of blood platelets for 5 days. It showed that the tested dimerized fusion proteins expressed either in CHO or in yeast significantly inhibited the decrement of the number of blood platelets in idiopathic thrombocytopenia model mice, but the monomer fusion proteins did not.

**Table 3: The effect of dimerized TMP-TMP-HSA fusion protein on idiopathic thrombocytopenia (ITP) mice model.**

| Groups | | The amount of blood platelet (×10⁷ /mL) |
|---|---|---|
| Control group treated with Normal saline | | 134.0 |
| ITP Model group | | 27.8 |
| Positive group treated with rhTPO | | 45.7 |
| No. 1 CHO cell line | Monomer fusion protein | 23.2 |
| | Dimer fusion protein | 60.8 |
| | | |
| No. 2 CHO cell line | Monomer fusion protein | 31.1 |
| | Dimer fusion protein | 56.8 |
| No. 3 CHO cell line | Monomer fusion protein | 32.7 |
| | Dimer fusion protein | 61.5 |
| No. 4 CHO cell line | Monomer fusion protein | 34.1 |
| | Dimer fusion protein | 59.7 |
| No. 5 CHO cell line | Monomer fusion protein | 28.9 |
| | Dimer fusion protein | 61.8 |
| No. 6 CHO cell line | Monomer fusion protein | 30.5 |
| | Dimer fusion protein | 55.9 |
| No. 7 CHO cell line | Monomer fusion protein | 29.7 |
| | Dimer fusion protein | 59.6 |
| No. 8 CHO cell line | Monomer fusion protein | 31.8 |
| | Dimer fusion protein | 58.6 |
| No. 1 yeast strain | Monomer fusion protein | 25.8 |
| | Dimer fusion protein | 53.2 |
| No. 2 yeast strain | Monomer fusion protein | 27.3 |
| | Dimer fusion protein | 53.6 |
| No. 3 yeast strain | Monomer fusion protein | 25.3 |
| | Dimer fusion protein | 51.9 |
| No. 4 yeast strain | Monomer fusion protein | 28.7 |
| | Dimer fusion protein | 50.6 |
| No. 5 yeast strain | Monomer fusion protein | 30.1 |
| | Dimer fusion protein | 53.6 |
| No. 6 yeast strain | Monomer fusion protein | 27.4 |
| | Dimer fusion protein | 55.3 |
| No. 7 yeast strain | Monomer fusion protein | 29.1 |
| | Dimer fusion protein | 53.9 |
| No. 8 yeast strain | Monomer fusion protein | 28.2 |
| | Dimer fusion protein | 57.6 |

### Example 10 The therapeutic efficacy of dimerized TMP-TMP-HSA-fusion protein on Carboplatin induced thrombopenia model in mice

Kunming mouse was used to construct Carboplatin induced thrombopenia (CITP) pathological model. Chemotherapeutic drug of Carboplatin (Qilu Pharmacy Co., Ltd, batch number: 1110112ES) was single intraperitoneally injected to mice on a dosage of 80 mg/kg for all model groups. For control group, the normal saline was subcutaneous injected at head and neck region on a dosage of 100 µL / 20 g consecutively for the next 4 days. For treatment groups, the tested fusion protein was also subcutaneous injected at head and neck region consecutively for the next 4 days. There are ten mice in each group. Blood samples were collected at mouse tail vein every other day from the third injection. Platelets were manually counted by utilizing a hemocytometer, and the averages of platelets for five days are shown in table 4. The results showed that dimer fusion proteins in all tested samples expressed by CHO and yeast significantly inhibited the decrease of platelets in Carboplatin induced thrombopenia model mice, but monomer fusion protein did not.

**Table 4 The therapeutic efficacy of dimerized TMP-TMP-HSA-fusion protein on Carboplatin induced thrombopenia model in mice**

| Groups | | Platelet Counts(×10⁷ platelets/mL) |
|---|---|---|
| Control group treated with Normal saline | | 143.0 |
| CITP Model group | | 49.2 |
| Positive group treated with rhTPO | | 89.7 |
| No.1 Cell Strain of CHO | Monomer Fusion Protein | 50.1 |
| | Dimer Fusion Protein | 80.2 |
| No.2 Cell Strain of CHO | Monomer Fusion Protein | 48.9 |
| | Dimer Fusion Protein | 90.1 |
| No.3 Cell Strain of CHO | Monomer Fusion Protein | 50.3 |
| | Dimer Fusion Protein | 82.5 |
| No.4 Cell Strain of CHO | Monomer Fusion Protein | 51.5 |
| | Dimer Fusion Protein | 76.4 |
| No.5 Cell Strain of CHO | Monomer Fusion Protein | 50.9 |
| | Dimer Fusion Protein | 78.1 |
| No.6 Cell Strain of CHO | Monomer Fusion Protein | 47.2 |
| | Dimer Fusion Protein | 83.1 |
| No.7 Cell Strain of CHO | Monomer Fusion Protein | 48.0 |
| | Dimer Fusion Protein | 93.2 |
| No.8 Cell Strain of CHO | Monomer Fusion Protein | 48.6 |
| | Dimer Fusion Protein | 85.7 |
| No.1 Yeast Strain | Monomer Fusion Protein | 47.1 |
| | Dimer Fusion Protein | 88.6 |
| No.2 Yeast Strain | Monomer Fusion Protein | 48.8 |
| | Dimer Fusion Protein | 90.1 |
| No.3 Yeast Strain | Monomer Fusion Protein | 46.9 |
| | Dimer Fusion Protein | 89.4 |
| No.4 Yeast Strain | Monomer Fusion Protein | 48.4 |
| | Dimer Fusion Protein | 87.7 |
| No.5 Yeast Strain | Monomer Fusion Protein | 50.6 |
| | Dimer Fusion Protein | 85.8 |
| No.6 Yeast Strain | Monomer Fusion Protein | 50.2 |
| | Dimer Fusion Protein | 80.4 |
| No.7 Yeast Strain | Monomer Fusion Protein | 45.5 |
| | Dimer Fusion Protein | 86.9 |
| No.8 Yeast Strain | Monomer Fusion Protein | 48.2 |
| | Dimer Fusion Protein | 89.6 |

## Claims

1. A dimerized thrombopoietin mimetic peptide-thrombopoietin mimetic peptide-Human Serum Albumin (TMP-TMP-HSA) fusion protein, comprising tandem dimer thrombopoietin (TMP), peptide linker and human serum albumin (HSA), **characterized in that** the fusion protein further contains a dimerization domain, wherein disulfide bonds in said dimerization domain contribute to dimerization of the TMP-TMP-HSA and wherein the dimerization domain is from H or fip, in which the dimerization domain from H has a gene sequence shown as Seq ID No.1 and an amino acid sequence shown as Seq ID No.2, the dimerization domain from fip has a gene sequence shown as Seq ID No.3 and an amino acid sequence shown as Seq ID No.4, and the dimerization domain is comprised between the tandem dimer thrombopoietin and the human serum albumin.

2. The dimerized TMP-TMP-HSA fusion protein according to claim 1, **characterized in that** the dimerization domain is H and fip.

3. The dimerized TMP-TMP-HSA fusion protein according to claim 1, **characterized in that** the HSA can be conjugated to the N-terminal of the fusion protein on a structural formula of TMP-L-TMP-L5-H-fip-HSA; L and L5 are peptide linkers, the L has a gene sequence of GGTGGCGGCTCCGGA, TCCGGA, GGTGGCCCCTCCGGA or GGCGGTGGCGGCAGCGGTGGCGGCTCCGGA, and an amino acid sequence of GGGSGH, SG, GGPSG or GGGGSGGGSG; the H is dimerized domain peptide fragment, the L5 has a gene sequence of GGCGGCGGCGGAAGCAGATCT, and an amino acid sequence is GGGGSRS; the fip is a peptide fragment selected from fip domain.

4. The dimerized TMP-TMP-HSA fusion protein according to claim 1, **characterized in that** the HSA can be conjugated to the C-terminal of the fusion protein on a structural formula of HSA-L6-H-L7-TMP-L-TMP; L, L6 and L7 are peptide linkers, the L has a gene sequence of GGTGGCGGCTCCGGA, TCCGGA, GGTGGCCCCTCCGGA or GGCGGTGGCGGCAGCGGTGGCGGCTCCGGA, and an amino acid sequence of GGGSGH, SG, GGPSG or GGGGSGGGSG; the L6 has a gene sequence of GGCGGCGGCGGTTCCGGACTG and an amino acid sequence of GGGGSGL; the L7 has a gene sequence of GGTGGCGGCTCCGGA and an amino acid sequence of EFGGGGS; and the H is a dimerization domain peptide fragment.

5. The dimerized TMP-TMP-HSA fusion protein according to claim 1; **characterized in that** the TMP has a gene sequence shown as Seq ID No.5, and an amino acid sequence shown as Seq ID No.6, or said amino acid sequence with mutations still having thrombopoietic activity.

6. The dimerized TMP-TMP-HSA fusion protein according to claim 1, **characterized in that** the HSA has a gene sequence shown as Seq ID No.7, and an amino acid sequence shown as Seq ID No.8, or said amino acid sequence with mutations still having thrombopoietic activity.

7. A mammalian cell comprising the dimerized TMP-TMP-HSA fusion protein according to claim 1.

8. The mammalian cell according to claim 7, **characterized in that** said mammalian cell is a CHO cell.

9. A yeast cell comprising the dimerized TMP-TMP-HSA fusion protein according to claim 1.

10. The yeast cell according to claim 9, **characterized in that** said yeast cell is *Pichia pastoris.*

11. A composition comprising the dimerized TMP-TMP-HSA fusion protein according to any one of claims 1 to 10 for use in a method of treating primary or secondary thrombocytopenia diseases.

## Patentansprüche

1. Dimerisiertes Thrombopoetin-mimetisches Peptid-Thrombopoetin-mimetisches Peptid-Humanserumalbumin- (TMP-TMP-HSA) -Fusionsprotein, das Tandem-Dimer-Thrombopoetin (TMP), Peptidlinker und Humanserumalbumin (HSA) umfasst, **dadurch gekennzeichnet, dass** das Fusionsprotein ferner eine Dimerisierungsdomäne enthält, wobei Disulfidbindungen in der Dimerisierungsdomäne zur Dimerisierung des TMP-TMP-HSA beitragen, und wobei die Dimerisierungsdomäne von H oder fip ist, wobei die Dimerisierungsdomäne von H eine Gensequenz aufweist, die als Seq ID Nr. 1 gezeigt ist, und eine Aminosäuresequenz aufweist, die als Seq ID Nr. 2 gezeigt ist, die Dimerisierungsdomäne von fip eine Gensequenz aufweist, die als Seq ID Nr. 3 gezeigt ist, und eine Aminosäuresequenz aufweist, die als Seq ID Nr. 4 gezeigt ist, und die Dimerisierungsdomäne zwischen dem Tandem-Dimer-Thrombopoetin und dem Humanserumalbumin umfasst ist.

2. Dimerisiertes TMP-TMP-HSA-Fusionsprotein nach Anspruch 1, **dadurch gekennzeichnet, dass** die Dimerisierungsdomäne H und fip ist.

3. Dimerisiertes TMP-TMP-HSA-Fusionsprotein nach Anspruch 1, **dadurch gekennzeichnet, dass** das HSA an den N-Terminus des Fusionsproteins bei einer Strukturformel von TMP-L-TMP-L5-H-fip-HSA konjugiert werden kann; L und L5 Peptidlinker sind, das L eine Gensequenz von GGTGGCGGCTCCGGA, TCCGGA, GGTGGCCCCTCCGGA oder GGCGGTGGCGGCAGCGGTGGCGGCTCCGGA und eine Aminosäuresequenz von GGGSGH, SG, GGPSG oder GGGGSGGGSG aufweist; das H ein Dimerisierunagsdomänen-Peptidfragment ist, das L5 eine Gensequenz von GGCGGCGGCGGAAGCAGATCT aufweist und eine Aminosäuresequenz GGGGSRS ist; das fip ein Peptidfragment ist, das aus einer fip-Domäne ausgewählt ist.

4. Dimerisiertes TMP-TMP-HSA-Fusionsprotein nach Anspruch 1, **dadurch gekennzeichnet, dass** das HSA an den C-Terminus des Fusionsproteins bei einer Strukturformel von HSA-L6-H-L7-TMP-L-TMP konjugiert werden kann; L, L6 und L7 Peptidlinker sind, das L eine Gensequenz von GGTGGCGGCTCCGGA, TCCGGA, GGTGGCCCCTCCGGA oder GGCGGTGGCGGCAGCGGTGGCGGCTCCGGA und eine Aminosäuresequenz von GGGSGH, SG, GGPSG oder GGGGSGGGSG aufweist; das L6 eine Gensequenz von GGCGGCGGCGGTTCCGGACTG und eine Aminosäuresequenz von GGGGSGL aufweist; das L7 eine Gensequenz von GGTGGCGGCTCCGGA und eine Aminosäuresequenz von EFGGGGS aufweist; und H ein Dimerisierungsdomänen-Peptidfragment ist.

5. Dimerisiertes TMP-TMP-HSA-Fusionsprotein nach Anspruch 1; **dadurch gekennzeichnet, dass** das TMP eine Gensequenz aufweist, die als Seq ID Nr. 5 gezeigt ist, und eine Aminosäuresequenz, die als Seq ID Nr. 6 gezeigt ist, oder die Aminosäuresequenz mit Mutationen immer noch thrombopoietische Aktivität aufweist.

6. Dimerisiertes TMP-TMP-HSA Fusionsprotein nach Anspruch 1; **dadurch gekennzeichnet, dass** das HSA eine Gensequenz aufweist, die als Seq ID Nr. 7 gezeigt ist, und eine Aminosäuresequenz, die als Seq ID Nr. 8 gezeigt ist, oder die Aminosäuresequenz mit Mutationen immer noch thrombopoietische Aktivität aufweist.

7. Säugetierzelle, die das dimerisierte TMP-TMP-HSA-Fusionsprotein nach Anspruch 1 umfasst.

8. Säugetierzelle nach Anspruch 7, **dadurch gekennzeichnet, dass** die Säugetierzelle eine CHO-Zelle ist.

9. Hefezelle, welche das dimerisierte TMP-TMP-HSA-Fusionsprotein nach Anspruch 1 umfasst.

10. Hefezelle nach Anspruch 9, **dadurch gekennzeichnet, dass** die Hefezelle *Pichia pastoris* ist.

11. Zusammensetzung, welche das dimerisierte TMP-TMP-HSA-Fusionsprotein nach einem der Ansprüche 1 bis 10 zur Verwendung bei einem Verfahren zum Behandeln von primären oder sekundären Thrombozytopenie-Krankheiten umfasst.

## Revendications

1. Protéine de fusion de peptide mimétique de thrombopoïétine-peptide mimétique de thrombopoïétine-sérum albumine humaine (TMP-TMP-HSA) dimérisée, comprenant une thrombopoïétine de dimère en tandem (TMP), un lieur peptidique et une sérum albumine humaine (HSA), **caractérisée en ce que** la protéine de fusion contient en outre un domaine de dimérisation, des liaisons disulfure dans ledit domaine de dimérisation contribuant à la dimérisation TMP-TMP-HSA et le domaine de dimérisation étant à partir de H ou fip, le domaine de dimérisation à partir de H ayant une séquence génétique présentée comme SEQ ID NO: 1 et une séquence d'acides aminés présentée comme SEQ ID NO: 2, le domaine de dimérisation à partir de fip ayant une séquence génétique présentée comme SEQ ID NO : 3 et une séquence d'acides aminés présentée comme SEQ ID NO: 4, et le domaine de dimérisation étant compris entre la thrombopoïétine de dimère en tandem et la sérum albumine humaine.

2. Protéine de fusion de TMP-TMP-HSA dimérisée selon la revendication 1, **caractérisée en ce que** le domaine de dimérisation est H et fip.

3. Protéine de fusion de TMP-TMP-HSA dimérisée selon la revendication 1, **caractérisée en ce que** la HSA peut être conjuguée à l'extrémité N-terminale de la protéine de fusion sur une formule structurelle de TMP-L-TMP-L5-H-fip-HSA; L et L5 étant les lieurs peptidiques, L ayant une séquence génétique de GGTGGCGGCTCCGGA, TCCGGA, GGTGGCCCCTCCGGA ou GGCGGTGGCGGCAGCGGTGGCGGCTCCGGA et une séquence d'acides aminés de GGGSGH, SG, GGPSG ou GGGGSGGGSG ; le H étant un fragment de peptide de domaine dimérisé, le L5 ayant une séquence génétique de GGCGGCGGCGGAAGCAGATCT et une séquence d'acides aminés est GGGGSRS ; le fip étant un fragment de peptide choisi dans le domaine fip.

4. Protéine de fusion de TMP-TMP-HSA dimérisée selon la revendication 1, **caractérisée en ce que** la HSA peut être conjuguée à l'extrémité C-terminale de la protéine de fusion sur une formule structurelle de HSA-L6-H-L7-TMP-L-TMP ; L, L6 et L7 étant des lieurs peptidiques, le L ayant la séquence génétique de GGTGGCGGCTCCGGA, TCCGGA, GGTGGCCCCTCCGGA ou GGCGGTGGCGGCAGCGGTGGCGGCTCCGGA et une séquence d'acides aminés de GGGSGH, SG, GGPSG ou GGGGSGGGSG ; le L6 ayant une séquence génétique de GGCGGCGGCGGTTCCGGACTG et une séquence d'acides aminés de GGGGSGL ; le L7 ayant une séquence génétique de GGTGGCGGCTCCGGA et une séquence d'acides aminés de EFGGGGS ; et le H étant un fragment de peptide de domaine de dimérisation.

5. Protéine de fusion de TMP-TMP-HSA dimérisée selon la revendication 1 ; **caractérisée en ce que** la TMP a une séquence génétique présentée comme SEQ ID NO : 5 et une séquence d'acides aminés présentée comme SEQ ID NO : 6, ou ladite séquence d'acides aminés avec des mutations ayant toujours une activité thrombopoïétique.

6. Protéine de fusion de TMP-TMP-HSA dimérisée selon la revendication 1,
**caractérisée en ce que** la HSA a une séquence génétique présentée comme SEQ ID NO : 7, et une séquence d'acides aminés présentée comme SEQ ID NO: 8, ou ladite séquence d'acides aminés avec des mutations ayant toujours une activité thrombopoïétique.

7. Cellule de mammifère comprenant la protéine de fusion de TMP-TMP-HSA dimérisée selon la revendication 1.

8. Cellule de mammifère selon la revendication 7, **caractérisée en ce que** ladite cellule de mammifère est une cellule CHO.

9. Cellule de levure comprenant la protéine de fusion de TMP-TMP-HSA dimérisée selon la revendication 1.

10. Cellule de levure selon la revendication 9, **caractérisée en ce que** ladite cellule de levure est *Pichia pastoris.*

11. Composition comprenant la protéine de fusion de TMP-TMP-HSA dimérisée selon l'une quelconque des revendications 1 à 10, destinée à une utilisation dans un procédé de traitement de pathologie thrombocytopéniques primaires ou secondaires.
